(19) Europäisches Patentamt
European Patent Office
Office européen des brevets

(11) **EP 2 558 573 B1**

(12) **EUROPEAN PATENT SPECIFICATION**

(45) Date of publication and mention
of the grant of the patent:
**22.02.2017 Bulletin 2017/08**

(21) Application number: **11727564.4**

(22) Date of filing: **12.04.2011**

(51) Int Cl.:
***C12N 9/54*** (2006.01)

(86) International application number:
**PCT/US2011/032050**

(87) International publication number:
**WO 2011/130222 (20.10.2011 Gazette 2011/42)**

(54) **COMPOSITIONS AND METHODS COMPRISING VARIANT PROTEASES**

ZUSAMMENSETZUNGEN UND VERFAHREN MIT PROTEASEVARIANTEN

COMPOSITIONS ET PROCÉDÉS COMPRENANT DES PROTÉASES VARIANTES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.04.2010 US 324534 P**

(43) Date of publication of application:
**20.02.2013 Bulletin 2013/08**

(73) Proprietor: **Danisco US Inc.**
**Palo Alto, CA 94304 (US)**

(72) Inventors:
• **BOTT, Richard, R.**
**Palo Alto, CA 94304 (US)**
• **CASCAO-PEREIRA, Luis, G.**
**Palo Alto, CA 94304 (US)**
• **ESTELL, David, A.**
**Palo Alto, CA 94304 (US)**
• **GOEDEGEBUUR, Frits**
**Palo Alto, CA 94304 (US)**
• **POULOSE, Ayrookaran, Joseph**
**Palo Alto, CA 94304 (US)**

(74) Representative: **Casley, Christopher Stuart et al**
**Mewburn Ellis LLP**
**City Tower**
**40 Basinghall Street**
**London EC2V 5DE (GB)**

(56) References cited:
**WO-A1-2010/123754      WO-A2-02/077187**
**WO-A2-2008/112258      WO-A2-2010/056640**

**Description**

**FIELD OF THE INVENTION**

The present invention provides variant proteases that are useful for cleaning applications and in methods of cleaning. In one aspect, the present invention provides variant proteases, including *Bacillus sp.* variant subtilisin polypeptides, and cleaning compositions comprising one or more such variants.

**BACKGROUND OF THE INVENTION**

Serine proteases are a subgroup of carbonyl hydrolases comprising a diverse class of enzymes having a wide range of specificities and biological functions (see, e.g., Stroud, Sci. Amer. 131:74-88). Much research has been conducted on serine proteases (e.g., subtilisins), due largely to their usefulness in cleaning and feed applications.

WO 2008/112258 describes modified proteases and method for altering the expression of proteases in microorganisms. WO 02/077187 describes proteins, including subtilisin proteases, that produce an altered immunogenic response. WO 2010/123754 describes proteases with modified pro regions and methods for enhancing the production of mature proteases in bacterial host cells. WO 2010/056640 describes serine protease variants and cleaning compositions comprising the serine protease variants.

Although a number of useful variant proteases have been developed to address needs relating to these applications, there remains a need for new improved proteases and variant proteases for a variety of uses.

**SUMMARY OF THE INVENTION**

In one aspect, the invention provides an isolated variant protease comprising an amino acid sequence having at least 90% sequence identity to the amino acid sequence of SEQ ID NO:1, said variant protease amino acid sequence comprising a substitution of each of the amino acid residues in positions 76, 87, 118, 128, 129, 130, 188, and 244 of SEQ ID NO:1 with a different amino acid residue to yield a variant protease having an overall charge of -2, -1, 0, +1, +2, +3, or +4, wherein:

(i) the amino acid residue at each of positions 76 and 188 is substituted with a negatively charged amino acid residue selected from the group consisting of aspartic acid and glutamic acid;
(ii) the amino acid residue at each of positions 87, 118, and 244 is substituted with a positively charged amino acid residue selected from the group consisting of arginine, histidine, and lysine; and
(iii) the amino acid residue at each of positions 128, 129, and 130 is substituted with a neutral amino acid residue selected from the group consisting of serine, threonine, asparagine, glutamine, alanine, isoleucine, leucine, methionine, phenylalanine, tryptophan, tyrosine, and valine,

and wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence.

In some cases the amino acid sequence of said variant protease comprises amino acid substitutions N76D+S87R+G118R+S128L+P129Q+S130A+S188D+V244R. In particular, the variant protease may comprise the amino acid sequence set forth in SEQ ID NO:8.

In some cases the isolated variant protease comprises an amino acid sequence having at least 90% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue to yield a variant protease having an overall charge of -2, -1, 0, +1, +2, +3, or +4, wherein:

(i) the amino acid residue at position 76 is substituted with a negatively charged amino acid residue selected from the group of consisting of aspartic acid and glutamic acid,
(ii) the amino acid residue at each of positions 87 and 118 is substituted with a positively charged amino acid residue selected from the group consisting of arginine, histidine, and lysine, and
(iii) the amino acid residue at each of positions 128, 129, and 130 is substituted with an uncharged amino acid residue selected form the group consisting of serine, threonine, asparagine, glutamine, alanine, isoleucine, leucine, methionine, phenylalanine, tryptophan, tyrosine, and valine, and wherein said variant protease further comprises
(iv) a serine residue at amino acid position 188 or a substitution of the serine residue at position 188 with a different amino acid residue that is or is not an aspartic acid residue, and/or
(v) an asparagine residue at amino acid position 248 or a substitution of the asparagine residue at position 248 with a different amino acid residue that is or is not an arginine residue,

and wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence.

In some cases, the variant protease amino acid sequence comprises a serine residue at amino acid position 188 and/or an arginine residue at amino acid position 248.

In certain cases, the variant protease has an overall charge of +1.

In some cases, the amino acid sequence of said variant protease comprises amino acid substitutions N76D+S87R+G118R+S128L+P129Q+S130A. In particular, the variant protease may comprise the amino acid sequence set forth in SEQ ID NO:7.

In some cases, the variant protease comprises one of the following sets of amino acid substitutions relative to SEQ ID NO:1:

(i) N76D+S87R+G118R+S128L+P129Q+S130A, with the proviso that said variant protease does not comprise N248R+S188D, or

(ii) N76D+S87R+G118R+S128L+P129Q+S130A+S188D+V244R.

In a second aspect, the present invention provides a composition comprising at least one variant protease of the first aspect of the invention. In some cases, the composition is a detergent and/or cleaning composition.

In some cases, the cleaning composition is a laundry cleaning composition or a laundry detergent composition, respectively.

In some cases, the cleaning composition is a dishwashing detergent composition, optionally an automatic dishwashing detergent composition or a hand dishwashing detergent composition.

In some cases, the cleaning composition is a liquid detergent composition.

In some cases, the cleaning composition is a gel, tablet, powder, solid, or granule detergent composition.

In some cases, the cleaning composition does not contain phosphate;

In some cases, the composition is a liquid laundry cleaning composition or a powder laundry cleaning composition.

In some cases, the composition is a laundry booster cleaning composition, laundry additive cleaning composition, or laundry pre-spotter cleaning composition.

In some cases, the composition is a contact lens cleaning composition.

In some cases, the composition is not an automatic dishwashing composition.

In some cases, the composition of the invention comprises at least one additional enzyme, optionally wherein the at least one additional enzyme, optionally two or more additional enzymes, is or are selected from the group consisting of hemicellulase, cellulase, amylase, peroxidase, protease, xylanase, lipase, phospholipase, esterase, cutinase, pectinase, pectate lyase, mannanase, keratinase, reductase, oxidase, phenoloxidase, lipoxygenase, ligninase, pullulanase, tannase, pentosanase, malanase, ß-glucanase, arabinosidase, hyaluronidase, chondroitinase, and laccase.

In a third aspect, the present invention provides a method for cleaning an item or surface in need of cleaning, the method comprising contacting the item or surface with a variant protease of the first aspect of the invention or a composition of the second aspect of the invention, optionally wherein the method further comprises rinsing the item or surface with water.

In a fourth aspect, the present invention provides an isolated nucleic acid comprising a polynucleotide sequence that encodes the variant protease of the first aspect of the invention.

In a fifth aspect, the present invention provides an expression vector comprising at least one nucleic acid of the fourth aspect of the invention, optionally wherein the at least one nucleic acid is operably linked to a promoter.

In a sixth aspect, the present invention provides a recombinant host cell comprising the expression vector of the fifth aspect of the invention or the nucleic acid of the fourth aspect of the invention or the variant protease of the first aspect of the invention, optionally wherein:

the host cell is a bacterial cell;
the host cell is a *Bacillus* cell; or
the host cell is a *Bacillus* subtilis cell.

In a seventh aspect, the present invention provides a method of producing a variant protease, the method comprising cultivating the recombinant host cell of the sixth aspect of the invention under conditions conducive to the production of the variant protease, the method optionally further comprising recovering the variant protease from the culture.

## BRIEF DESCRIPTION OF THE FIGURES

Figure 1 presents an alignment of the mature amino acid sequence of *B. lentus* subtilisin GG36, the mature amino acid sequence of *B. amyloliquefaciens* subtilisin BPN', and amino acid sequences of exemplary variant protease polypeptides of the invention designated as PX3, PX4, and PX5, respectively.

Figure 2 provides a plasmid map of the pHPLT-GG36 *B. subtilis* expression plasmid.

## DETAILED DESCRIPTION OF THE INVENTION

The present invention provides variant proteases, as defined in the claims, that are particularly well suited to and useful in a variety of cleaning applications. In one aspect, the invention includes compositions comprising at least one of the variant proteases (e.g., variant subtilisins) set forth herein. Some such compositions comprise detergent compositions. In one aspect, the invention provides *Bacillus species* variant subtilisins and compositions comprising one or more such variant subtilisins. The invention also provides enzyme compositions, as defined in the claims, having comparable or improved wash performance, as compared to known proteases, such as, e.g., known serine proteases and/or subtilisin proteases.

Unless otherwise indicated, the practice of the present invention involves conventional techniques commonly used in molecular biology, microbiology, protein purification, protein engineering, protein and DNA sequencing, recombinant DNA fields, and industrial enzyme use and development, all of which are within the skill of the art.

Furthermore, the headings provided herein are not limitations of the various aspects or embodiments of the invention which can be had by reference to the specification as a whole. Accordingly, the terms defined immediately below are more fully defined by reference to the specification as a whole. Nonetheless, in order to facilitate understanding of the invention, definitions for a number of terms are provided below.

Unless defined otherwise herein, all technical and scientific terms used herein have the same meaning as commonly understood by one of ordinary skill in the art to which this invention pertains. Although many methods and materials similar or equivalent to those described herein find use in the practice of the present invention, some methods and materials are described herein. Accordingly, the terms defined immediately below are more fully described by reference to the specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, as these may vary, depending upon the context in which they are used by those of skill in the art.

It is intended that every maximum numerical limitation given throughout this specification include every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

A protease (also known as a proteinase) is an enzyme protein that has the ability to break down other proteins. A protease has the ability to conduct proteolysis, which begins protein catabolism by hydrolysis of peptide bonds that link amino acids together in a peptide or polypeptide chain forming the protein. This activity of a protease as a protein-digesting enzyme is termed a proteolytic activity. Many well known procedures exist for measuring proteolytic activity (Kalisz, "Microbial Proteinases," In: Fiechter (ed.), Advances in Biochemical Engineering/Biotechnology, (1988)). For example, proteolytic activity may be ascertained by comparative assays which analyze the respective protease's ability to hydrolyze a commercial substrate. Exemplary substrates useful in the analysis of protease or proteolytic activity, include, but are not limited to, di-methyl casein (Sigma C-9801), bovine collagen (Sigma C-9879), bovine elastin (Sigma E-1625), and bovine keratin (ICN Biomedical 902111). Colorimetric assays utilizing these substrates are well known in the art (see, e.g., WO 99/34011 and U.S. Pat. No. 6,376,450,). The pNA assay (see, e.g., Del Mar et al., Anal. Biochem. 99:316-320 (1979)) also finds use in determining the active enzyme concentration for fractions collected during gradient elution. This assay measures the rate at which p-nitroaniline is released as the enzyme hydrolyzes the soluble synthetic substrate, succinyl-alanine-alanine-proline-phenylalanine-p-nitroanilide (suc-AAPF-pNA). The rate of production of yellow color from the hydrolysis reaction is measured at 410 nm on a spectrophotometer and is proportional to the active enzyme concentration. In addition, absorbance measurements at 280 nanometers (nm) can be used to determine the total protein concentration. The active enzyme/total protein ratio gives the enzyme purity.

As used herein, "subtilisin" and "subtilisin protease" refer to any member of the S8 serine protease family as described in MEROPS - The Peptidase Data base (Rawlings et al., MEROPS: the peptidase database, Nucl. Acids Res.

34 Database issue, D270-272 (2006)). As described therein, the peptidase family S8 contains the serine endopeptidase subtilisin and its homologues (Biochem. J. 290:205-218 (1993)). Family S8, also known as the subtilase family, is the second largest family of serine peptidases. The tertiary structures for several members of family S8 have now been determined. A typical S8 protein structure consists of three layers with a seven-stranded β sheet sandwiched between two layers of helices. Subtilisin (S08.001) is the type structure for clan SB (SB). Despite the different structure, the active sites of subtilisin and chymotrypsin (S01.001) can be superimposed, which suggests the similarity is the result of convergent rather than divergent evolution. Many *Bacillus* species (*Bacillus sp.*) secrete large amounts of subtilisins. As used herein, the terms "protease variant," "variant protease," "mutant protease," and "protease mutant" are used interchangeably in reference to a protease that differs in amino acid sequence from the amino acid sequence of a reference protease (which may be a wild-type protease) by at least one amino acid, such as by at least one amino acid substitution, deletion, or addition. The proteolytic activity of a protease variant, variant protease, mutant protease, or protease mutant may be determined using procedures well known in the art and/or described herein.

As used herein, the terms "variant subtilisin," "subtilisin variant," "mutant subtilisin," and "subtilisin mutant" are used interchangeably in reference to a subtilisin protease that differs in amino acid sequence from the amino acid sequence of a reference subtilisin protease (which may be a wild-type subtilisin protease) by at least one amino acid substitution, deletion, or addition. The proteolytic activity of a subtilisin variant, variant subtilisin, mutant subtilisin, or subtilisin mutant may be determined using procedures well known in the art and/or described herein.

As used herein, the genus "*Bacillus*" includes all species within the genus *Bacillus,* as known to those of skill in the art, including, but not limited to, e.g., *B. subtilis*, *B. licheniformis*, *B. lentus*, *B. brevis*, *B. stearothermophilus*, *B. alkalophilus*, *B. amyloliquefaciens*, *B. clausii*, *B. halodurans*, *B. megaterium*, *B. coagulans*, *B. circulans*, *B. lautus*, and *B. thuringiensis.* It is recognized that the genus *Bacillus* continues to undergo taxonomical reorganization. Thus, it is intended that the genus include species that have been reclassified, including, but not limited to, such organisms as *B. stearothermophilus*, which is now named "*Geobacillus stearothermophilus*." The production of resistant endospores in the presence of oxygen is considered a defining feature of the genus *Bacillus,* although this characteristic also applies to the recently named *Alicyclobacillus*, *Amphibacillus*, *Aneurinibacillus*, *Anoxybacillus*, *Brevibacillus*, *Filobacillus*, *Gracilibacillus*, *Halobacillus*, *Paenibacillus*, *Salibacillus*, *Thermobacillus*, *Ureibacillus*, and *Virgibacillus.*

The terms "polynucleotide" and "nucleic acid," which are used interchangeably herein, refer to a polymer of any length of nucleotide monomers covalently bonded in a chain. DNA (deoxyribonucleic acid), a polynucleotide comprising deoxyribonucleotides, and RNA (ribonucleic acid), a polymer of ribonucleotides, are examples of polynucleotides or nucleic acids having distinct biological function. Polynucleotides or nucleic acids include, but are not limited to, a single-, double- or triple-stranded DNA, genomic DNA, cDNA, RNA, DNA-RNA hybrid, or a polymer comprising purine and pyrimidine bases, or other natural, chemically, biochemically modified, non-natural or derivatized nucleotide bases. The following are non-limiting examples of polynucleotides: genes, gene fragments, chromosomal fragments, expressed sequence tag(s) (EST(s)), exons, introns, messenger RNA (mRNA), transfer RNA (tRNA), ribosomal RNA (rRNA), ribozymes, complementary DNA (cDNA), recombinant polynucleotides, branched polynucleotides, plasmids, vectors, isolated DNA of any sequence, isolated RNA of any sequence, nucleic acid probes, and primers. In some embodiments, polynucleotides comprise modified nucleotides, such as methylated nucleotides and nucleotide analogs, uracil, other sugars and linking groups such as fluororibose and thioate, and nucleotide branches. In a particular embodiment, a sequence of nucleotides is interrupted by non-nucleotide components.

As used herein, the term "vector" refers to a nucleic acid construct or polynucleotide construct used to introduce or transfer nucleic acid(s) or polynucleotide(s) into a target cell or tissue. A vector is typically used to introduce foreign DNA into another cell or tissue. A vector generally comprises a DNA sequence that is a transgene and a larger polynucleotide sequence that serves as the "backbone" of the vector. The vector typically serves to transfers genetic information, such as the inserted transgene, to a target cell or tissue so as to isolate, multiply, or express the insert in the target cell or tissue. Vectors include plasmids, cloning vectors, bacteriophages, viruses (e.g., viral vector), cosmids, expression vectors, shuttle vectors, cassettes, and the like. A vector typically includes an origin of replication, a multicloning site, and a selectable marker. The process of inserting a vector into a target cell is typically referred to as transfection. The transfection of a cell with a viral vector is typically referred to as transduction. The present invention includes, in one aspect, a vector comprises a DNA sequence encoding a variant protease (e.g., precursor or mature variant protease) that is operably linked to a suitable prosequence (e.g., secretory, signal peptide sequence, etc.) capable of effecting the expression of the DNA sequence in a suitable host.

As used herein, the term "expression cassette" or "expression vector" refers to a nucleic acid construct or vector generated recombinantly or synthetically for the expression of a nucleic acid of interest (e.g., a foreign nucleic acid or transgene) in a target cell. The nucleic acid of interest typically expresses a protein of interest. An expression vector or expression cassette typically comprises a promoter nucleotide sequence that drives or promotes expression of the foreign nucleic acid. The expression vector or cassette also typically includes and other specified nucleic acid elements that permit transcription of a particular nucleic acid in a target cell. A recombinant expression cassette can be incorporated into a plasmid, chromosome, mitochondrial DNA, plastid DNA, virus, or nucleic acid fragment. Some expression vectors have the ability to incorporate and express heterologous DNA fragments in a host cell. Many prokaryotic and eukaryotic expression vectors are commercially available. Selection of appropriate expression vectors is within the knowledge of those of skill in the art. Selection of appropriate expression vectors for expression of a protein from a nucleic acid sequence incorporated into the expression vector is within the knowledge of those of skill in the art.

A DNA construct is an artificially constructed segment of nucleic acid that may be introduced into a target cell or tissue. A DNA construct typically comprises a DNA insert comprising a nucleotide sequence encoding a protein of interest that has been subcloned into a vector. The vector may contain bacterial resistance genes for growth in bacteria and a promoter for expression of the protein of interest in an organism. The DNA may be generated *in vitro* by PCR or any other suitable technique(s) known to those in the art. In some embodiments, the DNA construct comprises a nucleic acid sequence of interest. In some embodiments, the sequence is operably linked to additional elements such as control elements (e.g., promoters, etc.). The DNA construct may further comprise a selectable marker and may further comprise an incoming sequence flanked by homology boxes. The construct may comprise other non-homologous sequences, added to the ends (e.g., stuffer sequences or flanks). In some embodiments, the ends of the sequence are closed such that the DNA construct forms a closed circle. The nucleic acid sequence of interest, which is incorporated into the DNA construct, using techniques well known in the art, may be a wild-type, mutant, or modified nucleic acid. In some embodiments, the DNA construct comprises one or more nucleic acid sequences homologous to the host cell chromosome. In other embodiments, the DNA construct comprises one or more non-homologous nucleotide sequences. Once the DNA construct is assembled *in vitro* it may be used, for example, to: 1) insert heterologous sequences into a desired target sequence of a host cell; and/or 2) mutagenize a region of the host cell chromosome (i.e., replace an endogenous sequence with a heterologous sequence); 3) delete target genes; and/or 4) introduce a replicating plasmid into the host. "DNA construct" is used interchangeably herein with expression cassette."

As used herein, a "plasmid" refers to an extrachromosomal DNA molecule which is capable of replicating independently from the chromosomal DNA. A plasmid is double stranded (ds) and may be circular, is typically used as a cloning vector.

As used herein in the context of introducing a nucleic acid sequence into a cell, the term "introduced" refers to any method suitable for transferring the nucleic acid sequence into the cell. Such methods for introduction include but are not limited to protoplast fusion, transfection, transformation, electroporation, conjugation, and transduction (see, e.g., Ferrari et al., "Genetics," in Hardwood et al. (eds.), Bacillus, Plenum Publishing Corp., pp. 57-72 (1989)).

Transformation refers to the genetic alteration of a cell which results from the uptake, genomic incorporation, and expression of genetic material (e.g., DNA).

As used herein, a nucleic acid is "operably linked" with another nucleic acid sequence when it is placed into a functional relationship with another nucleic acid sequence. For example, a promoter or enhancer is operably linked to a nucleotide coding sequence if the promoter affects the transcription of the coding sequence. A ribosome binding site may be operably linked to a coding sequence if it is positioned so as to facilitate translation of the coding sequence. Typically, "operably linked" DNA sequences are contiguous. However, enhancers do not have to be contiguous. Linking is accomplished by ligation at convenient restriction sites. If such sites do not exist, synthetic oligonucleotide adaptors or linkers may be used in accordance with conventional practice.

As used herein the term "gene" refers to a polynucleotide (e.g., a DNA segment), that encodes a polypeptide and includes regions preceding and following the coding regions as well as intervening sequences (introns) between individual coding segments (exons).

As used herein, "recombinant" when used with reference to a cell typically indicates that the cell has been modified by the introduction of a heterologous nucleic acid sequence or that the cell is derived from a cell so modified. For example, a recombinant cell may comprise a gene not found in identical form within the native (non-recombinant) form of the cell, or a recombinant cell may comprise a native gene (found in the native form of the cell) but which has been modified and re-introduced into the cell. A recombinant cell may comprise a nucleic acid endogenous to the cell that has been modified without removing the nucleic acid from the cell; such modifications include those obtained by gene replacement, site-specific mutation, and related techniques known to those of ordinary skill in the art. Recombinant DNA technology includes techniques for the production of recombinant DNA in vitro and transfer of the recombinant DNA into cells where it may be expressed or propagated, thereby producing a recombinant polypeptide. "Recombination," "recombining," and "recombined" of polynucleotides or nucleic acids refer generally to the assembly or combining of two or more nucleic acid or polynucleotide strands or fragments to generate a new polynucleotide or nucleic acid. The recombinant polynucleotide or nucleic acid is sometimes referred to as a chimera. A nucleic acid or polypeptide is "recombinant" when it is artificial or engineered, or derived from an artificial or engineered protein or nucleic acid.

As used herein, the term nucleic acid or gene "amplification" refers to a process by which specific DNA sequences are disproportionately replicated such that the amplified nucleic acid or gene becomes present in a higher copy number than was initially present in the genome. In some embodiments, selection of cells by growth in the presence of a drug (e.g., an inhibitor of an inhibitable enzyme) results in the amplification of either the endogenous gene encoding the gene product required for growth in the presence of the drug or by amplification of exogenous (i.e., input) sequences encoding this nucleic acid or gene product or both.

"Amplification" is a special case of nucleic acid replication involving template specificity. It is to be contrasted with non-specific template replication (i.e., replication that is template-dependent but not dependent on a specific template). Template specificity is here distinguished from fidelity of replication (i.e., synthesis of the proper polynucleotide sequence) and nucleotide (ribo- or deoxyribo-) specificity. Template specificity is frequently described in terms of "target" specificity. Target sequences are "targets" in the sense that they are sought to be sorted out from other nucleic acid. Amplification techniques have been designed primarily for this sorting out.

As used herein, the term "primer" refers to an oligonucleotide (a polymer of nucleotide residues), whether occurring naturally as in a purified restriction digest or produced synthetically, which is capable of acting as a point of initiation of synthesis when placed under conditions in which synthesis of a primer extension product which is complementary to a nucleic acid strand is induced (i.e., in the presence of nucleotides and an inducing agent such as DNA polymerase and at a suitable temperature and pH). A primer is preferably single stranded for maximum efficiency in amplification, but may alternatively be double stranded. If double stranded, the primer is first treated to separate its strands before being used to prepare extension products. In one aspect, the primer is an oligodeoxyribonucleotide. The primer must be sufficiently long to prime the synthesis of extension products in the presence of the inducing agent. The exact length of a primer depends on a variety of factors, including temperature, source of primer, and the use of the method.

As used herein, the term "probe" refers to an oligonucleotide, whether occurring naturally as in a purified restriction digest or produced synthetically, recombinantly or by PCR amplification, which is typically capable of hybridizing to another oligonucleotide of interest. A probe may be single-stranded or double-stranded. Probes are useful in the detection, identification and isolation of particular gene sequences. It is contemplated that any probe used in the present invention will be labeled with any "reporter molecule," so that is detectable in any detection system, including, but not limited to enzyme (e.g., ELISA, as well as enzyme-based histochemical assays), fluorescent, radioactive, and luminescent systems. It is not intended that the present invention be limited to any particular detection system or label.

As used herein, the term "target," when used in reference to the polymerase chain reaction, refers to the region of nucleic acid bounded by the primers used for polymerase chain reaction. Thus, the "target" is sought to be sorted out from other nucleic acid sequences. A nucleotide "segment" is a region of a nucleic acid within the target nucleic acid sequence.

As used herein, the term "polymerase chain reaction" (PCR) refers to the methods of U.S. Patent Nos. 4,683,195 4,683,202, and 4,965,188, which include methods for increasing the concentration of a segment of a target sequence in a mixture of genomic DNA without cloning or purification. This process for amplifying the target sequence is well known in the art.

As used herein, the term "amplification reagents" refers to those reagents (e.g., deoxyribonucleotide triphosphates, buffer, etc.), needed for amplification except for primers, nucleic acid template, and the amplification enzyme. Typically, amplification reagents along with other reaction components are placed and contained in a reaction vessel (test tube, microwell, etc.).

As used herein, the term "restriction endonuclease" or "restriction enzyme" refers to an enzyme (e.g., bacterial enzyme) that is capable of cutting double-stranded or single-stranded DNA at or near a specific sequence of nucleotides known as a restriction site. The nucleotide sequence comprising the restriction site is recognized and cleaved by a given restriction endonuclease or restriction enzyme and is frequently the site for insertion of DNA fragments. A restriction site can be engineered into an expression vector or DNA construct.

"Homologous recombination" refers to the exchange of DNA fragments between two DNA molecules or paired chromosomes at the site of identical or nearly identical nucleotide sequences. In some embodiments, chromosomal integration is homologous recombination.

A nucleic acid or polynucleotide is said to "encode" a polypeptide if, in its native state or when manipulated by methods known to those of skill in the art, it can be transcribed and/or translated to produce the polypeptide or a fragment thereof. The anti-sense strand of such a nucleic acid is also said to encode the sequence.

As is known in the art, a DNA sequence can be transcribed by an RNA polymerase to produce an RNA sequence, but an RNA sequence can be reverse transcribed by reverse transcriptase to produce a DNA sequence.

"Host strain" or "host cell" refers to a suitable host for an expression vector comprising a DNA sequence of interest. The DNA sequence of interest may express a protein of interest in the host strain or host cell.

A "protein" or "polypeptide" comprises a polymeric sequence of amino acid residues. The terms "protein" and "polypeptide" are used interchangeability herein. The 3-letter code for amino acids as defined in conformity with the IUPAC-IUB Joint Commission on Biochemical Nomenclature (JCBN) is used through out this disclosure. It is also understood that a polypeptide may be coded for by more than one nucleotide sequence due to the degeneracy of the genetic code.

A "prosequence" or "propetide sequence" refers to an amino acid sequence between the signal peptide sequence and mature protease sequence that is necessary for the secretion of the protease. Cleavage of the prosequence or propeptide sequence results in a mature active protease.

The term "signal sequence" or "signal peptide" refers to a sequence of amino acid residues that may participate in the secretion or direct transport of the mature or precursor form of a protein. The signal sequence is typically located N-terminal to the precursor or mature protein sequence. The signal sequence may be endogenous or exogenous. One exemplary exogenous signal sequence comprises the first seven amino acid residues of the signal sequence from *Bacillus subtilis* subtilisin fused to the remainder of the signal sequence of the subtilisin from *Bacillus lentus* (ATCC 21536). A signal sequence is normally absent from the mature protein. A signal sequence is typically cleaved from the protein by a signal peptidase after the protein is transported.

The term "hybrid signal sequence" refers to signal sequences in which part of sequence is obtained from the expression host fused to the signal sequence of the gene to be expressed. In some embodiments, synthetic sequences are utilized.

The term "mature" form of a protein, polypeptide, or peptide refers to the functional form of the protein, polypeptide, or peptide without the signal peptide sequence and propeptide sequence.

The term "precursor" form of a protein or peptide refers to a mature form of the protein having a prosequence operably linked to the amino or carbonyl terminus of the protein. The precursor may also have a "signal" sequence operably linked, to the amino terminus of the prosequence. The precursor may also have additional polynucleotides that are involved in post-translational activity (e.g., polynucleotides cleaved therefrom to leave the mature form of a protein or peptide).

The term "wild-type" in reference to an amino acid sequence or nucleic acid sequence indicates that the amino acid sequence or nucleic acid sequence is native or naturally occurring sequence.

As used herein with regard to amino acid residue positions, "corresponding to" or "corresponds to" or "corresponds" refers to an amino acid residue at the enumerated position in a protein or peptide, or an amino acid residue that is analogous, homologous, or equivalent to an enumerated residue in a protein or peptide. As used herein, "corresponding region" generally refers to an analogous position along related proteins or a reference protein.

The terms "derived from" and "obtained from" refer to not only a protease produced or producible by a strain of the organism in question, but also a protease encoded by a DNA sequence isolated from such strain and produced in a host organism containing such DNA sequence. Additionally, the term refers to a protease which is encoded by a DNA sequence of synthetic and/or cDNA origin and which has the identifying characteristics of the protease in question. To exemplify, "proteases derived from *Bacillus*" refers to those enzymes having proteolytic activity which are naturally-produced by *Bacillus*, as well as to serine proteases like those produced by *Bacillus* sources but which through the use of genetic engineering techniques are produced by non-*Bacillus* organisms transformed with a nucleic acid encoding the serine proteases.

The term "identical" in the context of two nucleic acids or polypeptide sequences refers to the residues in the two sequences that are the same when aligned for maximum correspondence, as measured using one of the following sequence comparison or analysis algorithms.

As used herein, "homologous genes" refers to a pair of genes from different, but usually related species, which correspond to each other and which are identical or very similar to each other. The term encompasses genes that are separated by speciation (i.e., the development of new species) (e.g., orthologous genes), as well as genes that have been separated by genetic duplication (e.g., paralogous genes).

As used herein, "homology" refers to sequence similarity or identity, with identity being preferred. Homology may be determined using standard techniques known in the art (see, e.g., Smith and Waterman, Adv. Appl. Math. 2:482 (1981); Needleman and Wunsch, J. Mol. Biol. 48:443 (1970); Pearson and Lipman, Proc. Natl. Acad. Sci. USA 85:2444 (1988); software programs such as GAP, BESTFIT, FASTA, and TFASTA in the Wisconsin Genetics Software Package (Genetics Computer Group, Madison, WI); and Devereux et al., Nucl. Acid Res. 12:387-395 (1984)). One example of a useful algorithm is PILEUP. PILEUP creates a multiple sequence alignment from a group of related sequences using progressive, pair-wise alignments. It can also plot a tree showing the clustering relationships used to create the alignment. PILEUP uses a simplification of the progressive alignment method of Feng and Doolittle (Feng and Doolittle, J. Mol. Evol. 35:351-360 (1987)). The method is similar to that described by Higgins and Sharp (Higgins and Sharp, CABIOS 5:151-153 (1989)). Useful PILEUP parameters including a default gap weight of 3.00, a default gap length weight of 0.10, and weighted end gaps. Another example of a useful algorithm is the BLAST algorithm, described by Altschul et al., (Altschul et al., J. Mol. Biol. 215:403-410, (1990); and Karlin et al., Proc. Natl. Acad. Sci. USA 90:5873-5787 (1993)). A particularly useful BLAST program is the WU-BLAST-2 program (Altschul et al., Meth. Enzymol. 266:460-480 (1996)). WU-BLAST-2 uses several search parameters, most of which are set to the default values. The adjustable parameters are set with the following values: overlap span = 1, overlap fraction = 0.125, word threshold (T) = 11. The HSP S and HSP S2 parameters are dynamic values and are established by the program itself depending upon the composition of the particular sequence and composition of the particular database against which the sequence of interest is being searched. However, the values may be adjusted to increase sensitivity.

The percent sequence identity between a reference sequence and a test sequence of interest may be readily determined by one skilled in the art. The percent identity shared by polynucleotide or polypeptide sequences is determined by direct comparison of the sequence information between the molecules by aligning the sequences and determining the identity by methods known in the art. An example of an algorithm that is suitable for determining sequence similarity is the BLAST algorithm, which is described in Altschul, et al., J. Mol. Biol., 215:403-410 (1990). Software for performing BLAST analyses is publicly available through the National Center for Biotechnology Information. This algorithm involves first identifying high scoring sequence pairs (HSPs) by identifying short words of length W in the query sequence that either match or satisfy some positive-valued threshold score T when aligned with a word of the same length in a database sequence. These initial neighborhood word hits act as starting points to find longer HSPs containing them. The word hits are expanded in both directions along each of the two sequences being compared for as far as the cumulative alignment score can be increased. Extension of the word hits is stopped when: the cumulative alignment score falls off by the quantity X from a maximum achieved value; the cumulative score goes to zero or below; or the end of either sequence is reached. The BLAST algorithm parameters W, T, and X determine the sensitivity and speed of the alignment. The BLAST program uses as defaults a wordlength (W) of 11, the BLOSUM62 scoring matrix (see Henikoff & Henikoff, Proc. Natl. Acad. Sci. USA 89:10915 (1992)) alignments (B) of 50, expectation (E) of 10, M'5, N'-4, and a comparison of both strands.

The BLAST algorithm then performs a statistical analysis of the similarity between two sequences (see e.g., Karlin and Altschul, Proc. Natl. Acad. Sci. USA 90:5873-5787 (1993)). One measure of similarity provided by the BLAST algorithm is the smallest sum probability (P(N)), which provides an indication of the probability by which a match between two nucleotide or amino acid sequences would occur by chance. For example, a nucleic acid is considered similar to a serine protease nucleic acid of this invention if the smallest sum probability in a comparison of the test nucleic acid to a serine protease nucleic acid is less than about 0.1, more preferably less than about 0.01, and most preferably less than about 0.001. Where the test nucleic acid encodes a serine protease polypeptide, it is considered similar to a specified serine protease nucleic acid if the comparison results in a smallest sum probability of less than about 0.5, and more preferably less than about 0.2.

Percent "identical" or "identity" in the context of two or more nucleic acid or polypeptide sequences refers to two or more sequences that are the same or have a specified percentage of nucleic acid residues or amino acid residues, respectively, that are the same, when compared and aligned for maximum similarity, as determined using a sequence comparison algorithm or by visual inspection. "Percent sequence identity" or "% identity" or "% sequence identity or "% amino acid sequence identity" of a subject amino acid sequence to a reference (i.e. query) amino acid sequence means that the subject amino acid sequence is identical (i.e., on an amino acid-by-amino acid basis) by a specified percentage to the query amino acid sequence over a comparison length when the sequences are optimally aligned. Thus, 80% amino acid sequence identity or 80% identity with respect to two amino acid sequences means that 80% of the amino acid residues in two optimally aligned amino acid sequences are identical.

"Percent sequence identity" or "% identity" or "% sequence identity or "% nucleotide sequence identity" of a subject nucleic acid sequence to a reference (i.e. query) nucleic acid sequence means that the subject nucleic acid sequence is identical (i.e., on a nucleotide-by-nucleotide basis for a polynucleotide sequence) by a specified percentage to the query sequence over a comparison length when the sequences are optimally aligned. Thus, 80% nucleotide sequence identity or 80% identity with respect to two nucleic acid sequences means that 80% of the nucleotide residues in two optimally aligned nucleic acid sequences are identical.

In one aspect, the percent sequence identity or % sequence identity" or "% identity" of a subject sequence to a query sequence can be calculated by optimally aligning the two sequences and comparing the two optimally aligned sequences over the comparison length. The number of positions in the optimal alignment at which identical residues occur in both sequences are determined, thereby providing the number of matched positions, and the number of matched positions is then divided by the total number of positions of the comparison length (which, unless otherwise specified, is the length of the query sequence). The resulting number is multiplied by 100 to yield the percent sequence identity of the subject sequence to the query sequence.

"Optimal alignment" or "optimally aligned" refers to the alignment of two (or more) sequences giving the highest percent identity score. For example, optimal alignment of two protein sequences can be achieved by manually aligning the sequences such that the maximum number of identical amino acid residues in each sequence are aligned together or by using software programs or procedures described herein or known in the art. Optimal alignment of two nucleic acid sequences can be achieved by manually aligning the sequences such that the maximum number of identical nucleotide residues in each sequence are aligned together or by using software programs or procedures described herein or known in the art.

In one exemplary aspect, two polypeptide sequences are deemed "optimally aligned" when they are aligned using defined parameters, such as a defined amino acid substitution matrix, gap existence penalty (also termed gap open penalty), and gap extension penalty, so as to achieve the highest similarity score possible for that pair of sequences. The BLOSUM62 scoring matrix (Henikoff and Henikoff, Proc. Natl. Acad. Sci. USA 89(22):10915 (1992)) is often used as a default scoring substitution matrix in polypeptide sequence alignment algorithms (such as BLASTP. The gap existence penalty is imposed for the introduction of a single amino acid gap in one of the aligned sequences, and the gap extension penalty is imposed for each residue position in the gap. Exemplary alignment parameters employed are: BLOSUM62 scoring matrix, gap existence penalty=11, and gap extension penalty=1. The alignment score is defined by the amino acid positions of each sequence at which the alignment begins and ends (e.g., the alignment window), and optionally by the insertion of a gap or multiple gaps into one or both sequences, so as to achieve the highest possible similarity score.

Optimal alignment between two or more sequences can be determined manually by visual inspection or by using a computer, such as, but not limited to, e.g., the BLASTP program for amino acid sequences and the BLASTN program for nucleic acid sequences (see, e.g., Altschul et al., Nucleic Acids Res. 25(17):3389-3402 (1997); see also National Center for Biotechnology Information (NCBI) website).

A polypeptide of interest may be said to be "substantially identical" to a reference polypeptide if the polypeptide of interest comprises an amino acid sequence having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the amino acid sequence of the reference polypeptide. The percent identity between two such polypeptides can be determined manually by inspection of the two optimally aligned polypeptide sequences or by using software programs or algorithms (e.g., BLAST, ALIGN, CLUSTAL) using standard parameters. One indication that two polypeptides are substantially identical is that the first polypeptide is immunologically cross-reactive with the second polypeptide. Typically, polypeptides that differ by conservative amino acid substitutions are immunologically cross-reactive. Thus, a polypeptide is substantially identical to a second polypeptide, for example, where the two peptides differ only by a conservative amino acid substitution or one or more conservative amino acid substitutions.

A nucleic acid of interest may be said to be "substantially identical" to a reference nucleic acid if the nucleic acid of interest comprises a nucleotide sequence having at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, or at least about 99.5% sequence identity to the nucleotide sequence of the reference nucleic acid. The percent identity between two such nucleic acids can be determined manually by inspection of the two optimally aligned nucleic acid sequences or by using software programs or algorithms (e.g., BLAST, ALIGN, CLUSTAL) using standard parameters. One indication that two nucleic acid sequences are substantially identical is that the two nucleic acid molecules hybridize to each other under stringent conditions (e.g., within a range of medium to high stringency). A variant protease encoded by a nucleic acid comprising a polynucleotide sequence capable of hybridizing to the polynucleotide sequence of SEQ ID NO:9, SEQ ID NO:10, or SEQ ID NO:11 under conditions of from medium stringency to high stringency or highest stringency may be deemed to be "equivalent" to the variant protease having the polypeptide sequence of any of SEQ ID NO:6, SEQ ID NO:7, or SEQ ID NO:8, respectively.

A nucleic acid or polynucleotide is "isolated" when it is partially or completely separated from other components (such as, but not limited to, e.g., other proteins, nucleic acids, cells, etc.). Similarly, a polypeptide, protein or peptide is "isolated" when it is partially or completely separated from other components (such as, but not limited to, e.g., other proteins, nucleic acids, cells, etc.). On a molar basis, an isolated species is more abundant than are other species in a composition. For example, an isolated species may comprise at least about 50% 70%, 80%, 85%, 90%, 91%, 92%, 93%, 94%, 95%, 96%, 97%, 98%, 99%, or 100% (on a molar basis) of all macromolecular species present. Preferably, the species of interest is purified to essential homogeneity (i.e., contaminant species cannot be detected in the composition by conventional detection methods). Purity and homogeneity can be determined using a number of techniques well known in the art, such as agarose or polyacrylamide gel electrophoresis of a protein or nucleic acid sample, followed by visualization upon staining. If desired, a high-resolution technique, such as high performance liquid chromatography (HPLC) or a similar means can be utilized for purification of the material.

The term "purified" as applied to nucleic acids or polypeptides generally denotes a nucleic acid or polypeptide that is essentially free from other components as determined by analytical techniques well known in the art (e.g., a purified polypeptide or polynucleotide forms a discrete band in an electrophoretic gel, chromatographic eluate, and/or a media subjected to density gradient centrifugation). For example, a nucleic acid or polypeptide that gives rise to essentially one band in an electrophoretic gel is "purified." A purified nucleic acid or polypeptide is at least about 50% pure, usually at least about 75%, 80%, 85%, 90%, 91%, 92%, 93%, 94%, 95%, 96%, 97%, 98%, 99%, 99.5%, 99.6%, 99.7%, 99.8% or more pure (e.g., percent by weight on a molar basis). In a related sense, the invention provides methods of enriching compositions for one or more molecules of the invention, such as one or more polypeptides or polynucleotides of the invention. A composition is enriched for a molecule when there is a substantial increase in the concentration of the molecule after application of a purification or enrichment technique. A substantially pure polypeptide or polynucleotide of the invention (e.g., substantially pure variant protease or polynucleotide encoding a variant protease of the invention, respectively) will typically comprise at least about 55%, 60%, 70%, 80%, 85%, 90%, 91%, 92%, 93%, 94%, 95%, 96%, 97%, 98, 99%, 99.5% or more by weight (on a molar basis) of all macromolecular species in a particular composition.

In a related sense, the invention provides methods of enriching compositions for one or more molecules of the invention, such as one or more polypeptides of the invention (e.g., one or more variant proteases of the invention) or one or more nucleic acids of the invention (e.g., one or more nucleic acids encoding one or more variant proteases of the invention). A composition is enriched for a molecule when there is a substantial increase in the concentration of the molecule after application of a purification or enrichment technique. A substantially pure polypeptide or polynucleotide will typically comprise at least about 55%, 60%, 70%, 80%, 85%, 90%, 91%, 92%, 93%, 94%, 95%, 96%, 97%, 98, 99%, 99.5% or more by weight (on a molar basis) of all macromolecular species in a particular composition.

As used herein, the term "combinatorial mutagenesis" refers to methods in which libraries of nucleic acid variants of a reference nucleic acid sequence are generated. In these libraries, the variants contain one or several mutations chosen from a predefined set of mutations. The methods also provide means to introduce random mutations which were not members of the predefined set of mutations. Some such methods include those set forth in U.S. Patent No. 6,582,914,. Some such combinatorial mutagenesis methods include encompass methods embodied in commercially available kits (e.g., QuikChange® Multi Site-Directed Mutagenesis Kit (Stratagene)).

As used herein, "having improved properties" used in connection with a variant protease refers to a variant protease with improved or enhanced wash or cleaning performance, and/or improved or enhanced stability optionally with retained wash or cleaning performance, relative to the corresponding reference protease (e.g., wild-type or naturally-occurring protease). The improved properties of a variant protease may comprise improved wash or cleaning performance and/or improved stability. In one aspect, the invention provides variant proteases of the invention that exhibit one of more of the following properties: improved hand wash performance, improved hand or manual dishwashing performance, improved automatic dishwashing performance, improved laundry performance, and/or improved stability relative to a reference protease (e.g., wild-type protease, such as a wild-type subtilisin).

As used herein, the term "functional assay" refers to an assay that provides an indication of a protein's activity. In some embodiments, the term refers to assay systems in which a protein is analyzed for its ability to function in its usual capacity. For example, in the case of enzymes, a functional assay involves determining the effectiveness of the enzyme in catalyzing a reaction.

As used herein, the term "target property" refers to the property of the starting gene that is to be altered. It is not intended that the present invention be limited to any particular target property. However, in some embodiments, the target property is the stability of a gene product (e.g., resistance to denaturation, proteolysis or other degradative factors), while in other embodiments, the level of production in a production host is altered.

The term "property" or grammatical equivalents thereof in the context of a nucleic acid, as used herein, refer to any characteristic or attribute of a nucleic acid that can be selected or detected. These properties include, but are not limited to, a property affecting binding to a polypeptide, a property conferred on a cell comprising a particular nucleic acid, a property affecting gene transcription (e.g., promoter strength, promoter recognition, promoter regulation, enhancer function), a property affecting RNA processing (e.g., RNA splicing, RNA stability, RNA conformation, and post-transcriptional modification), a property affecting translation (e.g., level, regulation, binding of mRNA to ribosomal proteins, post-translational modification). For example, a binding site for a transcription factor, polymerase, regulatory factor, etc., of a nucleic acid may be altered to produce desired characteristics or to identify undesirable characteristics.

The term "property" or grammatical equivalents thereof in the context of a polypeptide (including proteins), as used herein, refer to any characteristic or attribute of a polypeptide that can be selected or detected. These properties include, but are not limited to oxidative stability, substrate specificity, catalytic activity, enzymatic activity, thermal stability, alkaline stability, pH activity profile, resistance to proteolytic degradation, $K_M$, $k_{cat}$, $k_{cat}/k_M$ ratio, protein folding, inducing an immune response, ability to bind to a ligand, ability to bind to a receptor, ability to be secreted, ability to be displayed on the surface of a cell, ability to oligomerize, ability to signal, ability to stimulate cell proliferation, ability to inhibit cell proliferation, ability to induce apoptosis, ability to be modified by phosphorylation or glycosylation, and/or ability to treat disease, etc.

As used herein, the term "screening" has its usual meaning in the art. In one exemplary screening process, a mutant nucleic acid or variant polypeptide encoded therefrom is provided and a property of the mutant nucleic acid or variant polypeptide, respectively, is assessed or determined. The determined property of the mutant nucleic acid or variant polypeptide may be compared to a property of the corresponding precursor (parent) nucleic acid or to the property of the corresponding parent polypeptide, respectively.

It will be apparent to the skilled artisan that the screening procedure for obtaining a nucleic acid or protein with an altered property depends upon the property of the starting material the modification of which the generation of the mutant nucleic acid is intended to facilitate. The skilled artisan will therefore appreciate that the invention is not limited to any specific property to be screened for and that the following description of properties lists illustrative examples only. Methods for screening for any particular property are generally described in the art. For example, one can measure binding, pH, specificity, etc., before and after mutation, wherein a change indicates an alteration. Preferably, the screens are performed in a high-throughput manner, including multiple samples being screened simultaneously, including, but not limited to assays utilizing chips, phage display, and multiple substrates and/or indicators.

As used herein, in some embodiments, a screening process encompasses one or more selection steps in which variants of interest are enriched from a population of variants. Examples of these embodiments include the selection of variants that confer a growth advantage to the host organism, as well as phage display or any other method of display, where variants can be captured from a population of variants based on their binding or catalytic properties. In some embodiments, a library of variants is exposed to stress (heat, protease, denaturation) and subsequently variants that are still intact are identified in a screen or enriched by selection. It is intended that the term encompass any suitable means for selection. Indeed, it is not intended that the present invention be limited to any particular method of screening.

The terms "modified nucleic acid sequence" and "modified gene" are used interchangeably herein to refer to a nucleic acid sequence that includes a deletion, insertion or interruption of naturally occurring (i.e., wild-type) nucleic acid sequence. In some embodiments, the expression product of the modified nucleic acid sequence is a truncated protein (e.g., if the modification is a deletion or interruption of the sequence). In some embodiments, the truncated protein retains biological activity. In alternative embodiments, the expression product of the modified nucleic acid sequence is an elongated protein (e.g., modifications comprising an insertion into the nucleic acid sequence). In some embodiments, a nucleotide insertion in the nucleic acid sequence leads to a truncated protein (e.g., when the insertion results in the formation of a stop codon). Thus, an insertion may result in either a truncated protein or an elongated protein as an expression product.

A "mutant" nucleic acid sequence typically refers to a nucleic acid sequence that has an alteration in at least one codon occurring in a host cell's wild-type sequence such that the expression product of the mutant nucleic acid sequence is a protein with an altered amino acid sequence relative to the wild-type protein. The expression product may have an altered functional capacity (e.g., enhanced enzymatic activity).

As used herein, the phrase "alteration in substrate specificity" refers to changes in the substrate specificity of an enzyme. In some embodiments, a change in substrate specificity is defined as a change in $k_{cat}$ and/or $K_m$ for a particular substrate, resulting from mutations of the enzyme or alteration of reaction conditions. The substrate specificity of an enzyme is determined by comparing the catalytic efficiencies it exhibits with different substrates. These determinations find particular use in assessing the efficiency of mutant enzymes, as it is generally desired to produce variant enzymes that exhibit greater ratios of $k_{cat}/K_m$ for substrates of interest. However, it is not intended that the present invention be limited to any particular substrate composition or substrate specificity.

As used herein, "surface property" is used in reference to electrostatic charge, as well as properties such as the hydrophobicity and hydrophilicity exhibited by the surface of a protein.

The terms "thermally stable" and "thermostable" and "thermostability" refer to proteases that retain a specified amount of enzymatic activity after exposure to identified temperatures over a given period of time under conditions prevailing during the proteolytic, hydrolyzing, cleaning or other process of the invention, such as, e.g., while exposed to altered temperatures. Altered temperatures include increased or decreased temperatures. In some embodiments, the proteases retain at least about 50%, about 60%, about 70%, about 75%, about 80%, about 85%, about 90%, about 92%, about 95%, about 96%, about 97%, about 98%, or about 99% proteolytic activity after exposure to altered temperatures over a given time period, for example, at least about 60 minutes, about 120 minutes, about 180 minutes, about 240 minutes, about 300 minutes, etc.

The term "enhanced stability" in the context of an oxidation, chelator, thermal and/or pH stable protease refers to a higher retained proteolytic activity over time as compared to other proteases (e.g., subtilisin proteases) and/or wild-type enzymes.

The term "diminished stability" in the context of an oxidation, chelator, thermal and/or pH stable protease refers to a lower retained proteolytic activity over time as compared to other proteases (e.g., subtilisin proteases) and/or wild-type enzymes.

The term "cleaning activity" refers to a cleaning performance achieved by a variant protease or reference protease under conditions prevailing during the proteolytic, hydrolyzing, cleaning, or other process of the invention. In one aspect, cleaning performance of a variant protease or reference protease may be determined by using various assays for cleaning one or more various enzyme sensitive stains on an item or surface, such as, e.g., for example, a stain resulting from food, grass, blood, milk, or egg protein. Cleaning performance of a variant or reference protease can be determined by subjecting the stain on the item or surface to standard wash condition(s) and assessing the degree to which the stain is removed by using various chromatographic, spectrophotometric, or other quantitative methodologies. Exemplary cleaning assays and methods include, but are not limited to, e.g., those described in WO 99/34011 and U.S. Pat. 6,605,458, as well as those cleaning assays and methods included in the Examples presented below.

The term "cleaning effective amount" of a variant protease or reference protease refers to the amount of protease that achieves a desired level of enzymatic activity in a specific cleaning composition. Such effective amounts are readily ascertained by one of ordinary skill in the art and are based on many factors, such as the particular protease used, the cleaning application, the specific composition of the cleaning composition, and whether a liquid or dry (e.g., granular, bar) composition is required, etc.

The term "cleaning adjunct material" refers to any liquid, solid, or gaseous material included in cleaning composition other than a variant protease of the invention. A cleaning composition of the invention may include one of more cleaning adjunct materials. Each cleaning adjunct material is typically selected depending on the particular type and form of cleaning composition (e.g., liquid, granule, powder, bar, paste, spray, tablet, gel; or foam composition). Preferably, each cleaning adjunct material is compatible with the protease enzyme used in the composition.

The term "enhanced performance" in the context of cleaning activity refers to an increased or greater cleaning activity of certain enzyme sensitive stains such as egg, milk, grass or blood, as determined by usual evaluation after a standard wash cycle and/or multiple wash cycles.

The term "diminished performance" in the context of cleaning activity refers to a decreased or lesser cleaning activity of certain enzyme sensitive stains such as egg, milk, grass or blood, as determined by usual evaluation after a standard wash cycle.

The term "comparative performance" in the context of cleaning activity of a variant protease of the invention refers to at least about 60%, at least about 70%, at least about 75%, at least about 80%, at least about 85%, at least about 90%, at least about 91%, at least about 92%, at least about 93%, at least about 94%, at least about 95%, at least about 96%, at least about 97%, at least about 98%, at least about 99%, at least about 99.5% of the cleaning activity of a comparative or reference protease (e.g., commercially available proteases), including, but not limited to, e.g., OPTIMASE™ protease (Genencor), PURAFECT™ protease products (Genencor), SAVINASE™ protease (Novozymes), BPN'-variants (see, e.g., U.S. Pat. No. Re 34,606), RELASE™, DURAZYME™, EVERLASE™, KANNASE™ protease (Novozymes), MAXACAL™, MAXAPEM™, PROPERASE™ proteases (Genencor; see also U.S. Pat. No. Re 34,606, and U.S. Pat. Nos. 5,700,676; 5,955,340; 6,312,936; and 6,482,628), and B. *lentus* variant protease products (e.g., those described in WO 92/21760, WO 95/23221 and/or WO 97/07770). Cleaning performance can be determined by comparing the variant proteases of the present invention with reference subtilisin proteases in various cleaning assays concerning enzyme sensitive stains such as grass, blood or milk as determined by usual spectrophotometric or analytical methodologies after standard wash cycle conditions.

As used herein, "cleaning composition" or "cleaning formulation" of the invention refers to any composition of the invention useful for removing or eliminating a compound (e.g., undesired compound) from an object, item or surface to be cleaned, including, but not limited, to, e.g., a fabric, fabric item, dishware item, tableware item, glassware item, contact lens, other solid substrate, hair (shampoo) (including human or animal hair), skin (soap or and cream), teeth (mouthwashes, toothpastes), surface of an item or object (e.g., hard surface, such as, e.g., the hard surface of a table, table top, wall, furniture item, floor, ceiling, non-dishware item, non-tableware item, etc.), contact lenses, etc. The term encompasses any material and/or added compound selected for the particular type of cleaning composition desired and the form of the product (e.g., liquid, gel, granule, or spray composition), as long as the composition is compatible with the protease and other enzyme(s) used in the composition. The specific selection of cleaning composition materials are readily made by considering the surface, object, item, or fabric to be cleaned, and the desired form of the composition for the cleaning conditions during use.

Cleaning compositions and cleaning formulations include any composition that is suited for cleaning, bleaching, disinfecting, and/or sterilizing any object, item, and/or surface. Such compositions and formulations include, but are not limited to, e.g., liquid and/or solid compositions, including cleaning or detergent compositions (e.g., liquid, tablet, gel, granule, and/or solid laundry cleaning or detergent compositions and fine fabric detergent compositions; hard surface cleaning compositions and formulations, such as for glass, wood, ceramic and metal counter tops and windows; carpet cleaners; oven cleaners; fabric fresheners; fabric softeners; and textile, laundry booster cleaning or detergent compositions, laundry additive cleaning compositions, and laundry pre-spotter cleaning compositions; dishwashing compositions, including, e.g., hand or manual dishwash compositions (e.g., hand or manual dishwashing detergents) and automatic dishwashing compositions (e.g., automatic dishwashing detergents).

Cleaning composition or cleaning formulations, as used herein, include, unless otherwise indicated, granular or powder-form all-purpose or heavy-duty washing agents, especially cleaning detergents; liquid, granular, gel, solid, tablet, or paste-form all-purpose washing agents, especially the so-called heavy-duty liquid (HDL) detergent or heavy-duty powder detergent (HDD) types; liquid fine-fabric detergents; hand or manual dishwashing agents, including those of the high-foaming type; hand or manual dishwashing, automatic dishwashing, or dishware or tableware washing agents, including the various tablet, powder, solid, granular, liquid, gel, and rinse-aid types for household and institutional use; liquid cleaning and disinfecting agents, including antibacterial hand-wash types, cleaning bars, mouthwashes, denture cleaners, car shampoos, carpet shampoos, bathroom cleaners; hair shampoos and/or hair-rinses for humans and other animals; shower gels and foam baths and metal cleaners; as well as cleaning auxiliaries, such as bleach additives and "stain-stick" or pre-treat types. In some embodiments, granular compositions are in "compact" form; in some embodiments, liquid compositions are in a "concentrated" form.

As used herein, "fabric cleaning compositions" include hand and machine laundry detergent compositions including laundry additive compositions and compositions suitable for use in the soaking and/or pretreatment of stained fabrics (e.g., clothes, linens, and other textile materials).

As used herein, "non-fabric cleaning compositions" include non-textile (i.e., non-fabric) surface cleaning compositions, including, but not limited to, e.g., hand or manual or automatic dishwashing detergent compositions, oral cleaning compositions, denture cleaning compositions, and personal cleansing compositions.

As used herein, the term "detergent composition" or "detergent formulation" is used in reference to a composition intended for use in a wash medium for the cleaning of soiled or dirty objects, including particular fabric and/or non-fabric objects or items. Such compositions of the present invention are not limited to any particular detergent composition or formulation. Indeed, in some aspects, detergents of the invention comprise at least one variant protease of the invention and, in addition, one or more surfactants, transferase(s), hydrolytic enzymes, oxido reductases, builders (such as, e.g., a builder salt), bleaching agents, bleach activators, bluing agents, fluorescent dyes, caking inhibitors, masking agents, enzyme activators, antioxidants, and/or solubilizers. In some instances, a builder salt is a mixture of a silicate salt and a phosphate salt, preferably with more silicate (e.g., sodium metasilicate) than phosphate (e.g., sodium tripolyphosphate). Some compositions of the invention, such as, but not limited to, cleaning compositions or detergent compositions, do not contain any phosphate (e.g., phosphate salt or phosphate builder).

As used herein, the term "bleaching" refers to the treatment of a material (e.g., fabric, laundry, pulp, etc.) or surface for a sufficient length of time and/or under appropriate pH and/or temperature conditions to effect a brightening (i.e., whitening) and/or cleaning of the material. Examples of chemicals suitable for bleaching include, but are not limited to, e.g., $ClO_2$, $H_2O_2$, peracids, $NO_2$, etc.

As used herein, "wash performance" of a protease (e.g., a variant protease of the invention) refers to the contribution of a variant protease to washing that provides additional cleaning performance to the detergent as compared to the detergent without the addition of the variant protease to the composition. Wash performance is compared under relevant washing conditions. In some test systems, other relevant factors, such as detergent composition, sud concentration, water hardness, washing mechanics, time, pH, and/or temperature, can be controlled in such a way that condition(s) typical for household application in a certain market segment (e.g., hand or manual dishwashing, automatic dishwashing, dishware cleaning, tableware cleaning, fabric cleaning, etc.) are imitated.

The term "relevant washing conditions" is used herein to indicate the conditions, particularly washing temperature, time, washing mechanics, sud concentration, type of detergent and water hardness, actually used in households in a hand dishwashing, automatic dishwashing, or laundry detergent market segment.

The term "improved wash performance" is used to indicate that a better end result is obtained in stain removal under relevant washing conditions, or that less variant protease, on weight basis, is needed to obtain the same end result relative to the corresponding wild-type or starting parent protease.

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

The "compact" form of the cleaning compositions herein is best reflected by density and, in terms of composition, by the amount of inorganic filler salt. Inorganic filler salts are conventional ingredients of detergent compositions in powder form. In conventional detergent compositions, the filler salts are present in substantial amounts, typically about 17 to about 35% by weight of the total composition. In contrast, in compact compositions, the filler salt is present in amounts not exceeding about 15% of the total composition. In some embodiments, the filler salt is present in amounts that do not exceed about 10%, or more preferably, about 5%, by weight of the composition. In some embodiments, the inorganic filler salts are selected from the alkali and alkaline-earth-metal salts of sulfates and chlorides. In some embodiments, the filler salt is sodium sulfate.

The position of an amino acid residue in a given amino acid sequence is typically numbered herein using the numbering of the position of the corresponding amino acid residue of the *B. amyloliquefaciens* subtilisin BPN' amino acid sequence shown in SEQ ID NO:2. The *B. amyloliquefaciens* subtilisin BPN' amino acid sequence of SEQ ID NO:2 thus serves as a reference sequence. A given amino acid sequence, such as a variant protease amino acid sequence described herein, can be aligned with the BPN' sequence (SEQ ID NO:2) using an alignment algorithm as described herein, and an amino acid residue in the given amino acid sequence that aligns (preferably optimally aligns) with an amino acid residue in the BPN' sequence can be conveniently numbered by reference to the corresponding amino acid residue in the subtilisin BPN' sequence. For example, the PX4 variant protease of the invention can be described as a variant protease of the GG36 protease shown in SEQ ID NO:1 comprising an amino acid sequence comprising the six amino acid substitutions N76D+S87R+G118R+S128L+P129Q+S130A relative to SEQ ID NO:1, wherein amino acid residue positions of the PX4 variant protease are numbered according to the numbering of corresponding amino acid positions in the subtilisin BPN' amino acid sequence shown in SEQ ID NO:2 as determined by alignment of the PX4 amino acid sequence with the subtilisin BPN' amino acid sequence.

Alternatively, if amino acid residue positions of the PX4 variant protease sequence are numbered using the actual numbering of the amino acid residue positions in the GG36 amino acid sequence (SEQ ID NO:1), and not by reference to corresponding amino acid positions in the BPN' sequence upon alignment, the PX4 variant protease can be described as a variant protease of the GG36 protease shown SEQ ID NO:1 comprising an amino acid sequence comprising the six amino acid substitutions N74D+S85R+G116R+S126L+P127Q+S128A.

Generally, the nomenclature used herein and many of the laboratory procedures in cell culture, molecular genetics, molecular biology, nucleic acid chemistry, and protein chemistry described below are well known and commonly employed by those of ordinary skill in the art. Standard techniques, such as described in Sambrook et al., Molecular Cloning--A Laboratory Manual (2nd Ed.), Vols. 1-3, Cold Spring Harbor Laboratory, Cold Spring Harbor, N.Y., 1989 (hereinafter "Sambrook") and CURRENT PROTOCOLS IN MOLECULAR BIOLOGY, F. M. Ausubel et al., eds., Current Protocols, a joint venture between Greene Publishing Associates, Inc. and John Wiley & Sons, Inc. (1994, supplemented through 1999) (hereinafter "Ausubel"), are used for recombinant nucleic acid methods, nucleic acid synthesis, cell culture methods, and transgene incorporation, e.g., transfection, electroporation. Oligonucleotide synthesis and purification steps are typically performed according to specifications. Techniques and procedures are generally performed according to conventional methods well known in the art and various general references that are provided throughout this document. Procedures therein are believed to be well known to those of ordinary skill in the art and are provided for the convenience of the reader.

**Polypeptides of the Invention**

The present invention provides novel polypeptides, which may be collectively referred to as "polypeptides of the invention." Polypeptides of the invention include isolated, recombinant, substantially pure, or non-naturally occurring variant protease polypeptides, including, e.g., variant subtilisin polypeptides, having enzymatic activity (e.g., proteolytic activity). In one aspect, polypeptides of the invention are useful in cleaning applications and may be incorporated into cleaning compositions that are useful in methods of cleaning an item or a surface (e.g., of surface an item) in need of cleaning.

In one aspect, a variant protease of the invention comprises a "variant subtilisin", as defined in the claims. In one aspect, the invention provides a "*Bacillus sp.* variant protease." In one aspect, the invention provides a "*Bacillus sp.* variant subtilisin."

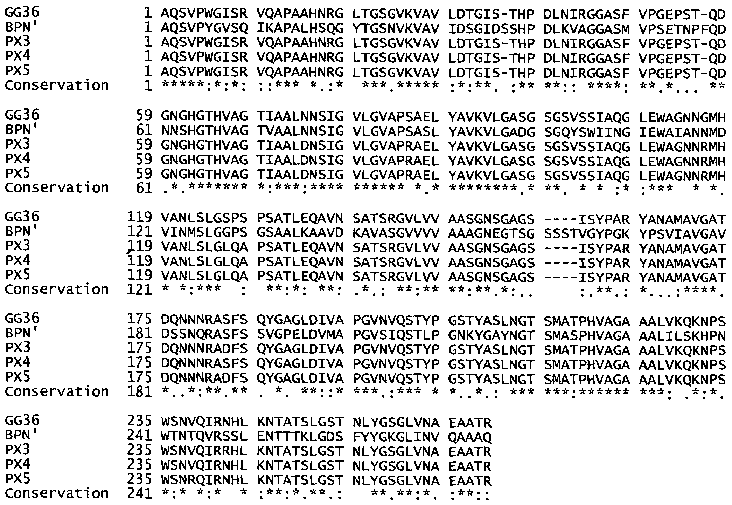

Also disclosed herein is an isolated, recombinant, substantially pure, or non-naturally occurring variant protease having proteolytic activity, which polypeptide comprises a polypeptide sequence having at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, at least about 99.5%, or 100% sequence identity to the amino acid sequence of SEQ ID NO:1, said variant protease amino acid sequence comprising a substitution of each of the amino acid residues in positions 76, 87, 118, 128, 129, 130, 188, and 244 of SEQ ID NO:1 with a different amino acid residue, wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). In one aspect, such variant proteases comprise variant subtilisins.

Also disclosed herein is an isolated, recombinant, substantially pure, or non-naturally occurring variant protease (e.g., variant subtilisin) having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5%, or 100% sequence identity to the amino acid sequence of SEQ ID NO:1, said variant protease amino acid sequence comprising a substitution of each of the amino acid residues in positions 76, 87, 118, 128, 129, 130, 188, and 244 of SEQ ID NO:1 with a different amino acid residue, wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). Also disclosed is an isolated, recombinant, or non-naturally occurring variant protease having proteolytic activity, said variant protease comprising an amino acid sequence having at least 95%, at least 96%, at least 97%, at least 98%, at least 99%, at least 99.5%, or 100% sequence identity to the amino acid sequence of SEQ ID NO:1, said variant protease amino acid sequence comprising a substitution of each of the amino acid residues in positions 76, 87, 118, 128, 129, 130, 188, and 244 of SEQ ID NO:1 with a different amino acid residue, wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence. For some such variant proteases, the amino acid sequence of the variant protease comprises a substitution of the amino acid residue in each of amino acid positions 76, 87, 118, 128, 129, 130, 188, and 244 of SEQ ID NO:1 with a different amino acid residue to yield a variant protease having an overall charge of - 2, -1, 0, +1, +2, +3, +4, +5, +6, +7, or +8, or, in one aspect, a variant protease having an overall charge of -2, -1, 0, +1, +2, +3, or +4, or, in one aspect, -2, -1, 0, +1, or +2, or in one aspect, +1. For some such variant proteases, in the amino acid sequence of the variant protease, (i) the amino acid residue at each of positions 76 and 188 is substituted with a negatively charged amino acid residue selected from the group consisting of aspartic acid and glutamic acid; (ii) the amino acid residue at each of positions 87, 118, and 244 is substituted with a positively charged amino acid residue selected from the group consisting of arginine, histidine, and lysine; and (iii) the amino acid residue at each of positions 128, 129, and 130 is substituted with a neutral amino acid residue selected from the group consisting of serine, threonine, asparagine, glutamine, alanine, isoleucine, leucine, methionine, phenylalanine, tryptophan, tyrosine, and valine. For some such variant proteases, the amino acid sequence of said variant protease comprises amino acid substitutions N76D+S87R+G118R+S128L+P129Q+S130A+S188D+V244R. In one aspect, the variant protease comprises the amino acid sequence set forth in SEQ ID NO:8.

Also disclosed herein is an isolated, recombinant, substantially pure, or non-naturally occurring polypeptide having proteolytic activity, which polypeptide comprises a polypeptide sequence having at least 98%, at least 99%, at least 99.5%, or 100% sequence identity to an amino acid sequence selected from the group consisting of SEQ ID NO:6, SEQ ID NO:7, and SEQ ID NO:8. Also disclosed herein is an isolated, recombinant, substantially pure, or non-naturally occurring variant protease (e.g., variant subtilisin) having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5%, or 100% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue, and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of *Bacillus amyloliquefaciens* subtilisin BPN' shown in SEQ ID NO:1, as determined by alignment of the variant protease amino acid sequence with the *Bacillus amyloliquefaciens* subtilisin BPN' amino acid sequence (see Figure 1).

Also disclosed is an isolated, recombinant, substantially pure, or non-naturally occurring variant protease having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5%, or 100% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue, and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). For some such variant proteases, the amino acid sequence of the variant protease comprises a serine residue at amino acid position 188 and/or an arginine residue at amino acid position 248. For some such variant proteases, the variant protease amino acid sequence comprises a substitution of the amino acid residue in each of amino acid positions 76, 87, 118, 128, 129, and 130 relative to SEQ ID NO:2 with a different amino acid to yield a variant protease having an overall charge of -2, -1, 0, +1, +2, +3, +4, +5, +6, +7, or +8, or, in one aspect, a variant protease having an overall charge of -2, -1, 0, +1, +2, +3, or +4, or in one aspect, a variant protease having an overall charge of +1. For some such variant proteases, in the amino acid sequence of the variant protease, (i) the amino acid residue at position 76 is substituted with a negatively charged amino acid residue selected from the group of consisting of aspartic acid and glutamic acid, (ii) the amino acid residue at each of positions 87 and 118 is substituted with a positively charged amino acid residue selected from the group consisting of arginine, histidine, and lysine, and (iii) the amino acid residue at each of positions 128, 129, and 130 is substituted with an uncharged (neutral) amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions N76D+S87R+G118R+S128L+P129Q+S130A. In one aspect, the variant protease comprises the amino acid sequence set forth in SEQ ID NO:7.

Also disclosed is an isolated, recombinant, substantially pure, or non-naturally occurring variant protease having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue, and (ii) substitution of the asparagine residue at position 248 with an arginine residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). For some such variant proteases, the variant protease amino acid sequence comprises a substitution of the amino acid residue in each of amino acid positions 76, 87, 118, 128, 129, and 130 relative to SEQ ID NO:2 with a different amino acid to yield a variant protease having an overall charge of -2, -1, 0, +1, +2, +3, +4, +5, +6, +7, or +8, or, in one aspect, a variant protease having an overall charge of -2, -1, 0, +1, +2, +3, or +4, or in one aspect, a variant protease having an overall charge of +1. For some such variant proteases, in the amino acid sequence of the variant protease, (i) the amino acid residue at position 76 is substituted with a negatively charged amino acid residue selected from the group of consisting of aspartic acid and glutamic acid, (ii) the amino acid residue at each of positions 87 and 118 is substituted with a positively charged amino acid residue selected from the group consisting of arginine, histidine, and lysine, and (iii) the amino acid residue at each of positions 128, 129, and 130 is substituted with an uncharged (neutral) amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions N76D+S87R+G118R+S128L+P129Q+S130A+N248R.

Also disclosed is an isolated, recombinant, substantially pure, or non-naturally occurring variant protease having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a substitution of the serine residue at position 188 with an aspartic acid residue, and (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). For some such variant proteases, the variant protease amino acid sequence comprises a substitution of the amino acid residue in each of amino acid positions 76, 87, 118, 128, 129, and 130 relative to SEQ ID NO:2 with a different amino acid to yield a variant protease having an overall charge of -2, -1, 0, +1, +2, +3, +4, +5, +6, +7, or +8, or, in one aspect, a variant protease having an overall charge of -2, -1, 0, +1, +2, +3, or +4, or in one aspect, a variant protease having an overall charge of +1. For some such variant proteases, in the amino acid sequence of the variant protease, (i) the amino acid residue at position 76 is substituted with a negatively charged amino acid residue selected from the group of consisting of aspartic acid and glutamic acid, (ii) the amino acid residue at each of positions 87 and 118 is substituted with a positively charged amino acid residue selected from the group consisting of arginine, histidine, and lysine, and (iii) the amino acid residue at each of positions 128, 129, and 130 is substituted with an uncharged (neutral) amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions N76D+S87R+G118R+S128L+P129Q+S130A+S188D.

Also disclosed is an isolated, recombinant, substantially pure, or non-naturally occurring variant protease having proteolytic activity, said variant protease comprising an amino acid sequence having at least 95%, at least 96%, at least 97%, at least 98%, at least 99%, at least 99.5%, or 100% sequence identity to the amino acid sequence of SEQ ID NO:1, said variant protease amino acid sequence comprising a substitution of the amino acid residue in each of amino acid positions 76, 87, 118, 128, 129, 130, 188, and 248 relative to SEQ ID NO:1 with a different amino acid residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). For some such variant proteases, the variant protease amino acid sequence comprises a substitution of the amino acid residue in each of amino acid positions 76, 87, 118, 128, 129, and 130 relative to SEQ ID NO:2 with a different amino acid to yield a variant protease having an overall charge of -2, -1, 0, +1, +2, +3, +4, +5, +6, +7, or +8, or, in one aspect, a variant protease having an overall charge of -2, -1, 0, +1, +2, +3, or +4, or in one aspect, a variant protease having an overall charge of +1. For some such variant proteases, in the amino acid sequence of the variant protease, (i) the amino acid residue at positions 76 and 188 is substituted with a negatively charged amino acid residue selected from the group of consisting of aspartic acid and glutamic acid, (ii) the amino acid residue at each of positions 87, 118, and 248 is substituted with a positively charged amino acid residue selected from the group consisting of arginine, histidine, and lysine, and (iii) the amino acid residue at each of positions 128, 129, and 130 is substituted with an uncharged (neutral) amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions N76D+S87R+G118R+S128L+P129Q+S130A+S188D+N248R. In one aspect, the variant protease comprises the amino acid sequence set forth in SEQ ID NO:6.

Also disclosed herein is an isolated, recombinant, substantially pure, or non-naturally occurring variant protease (e.g., variant subtilisin) having proteolytic activity, said variant protease comprising an amino acid sequence having at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, at least 99.5%, or 100% sequence identity to the amino acid sequence of SEQ ID NO:1, said variant protease comprising one of the following sets of amino acid substitutions relative to SEQ ID NO:1:

    (i) N76D+S87R+G118R+S128L+P129Q+S130A, with the proviso that said variant protease does not comprise both N248R+S188D, or

    (ii) N76D+S87R+G118R+S128L+P129Q+S130A+S188D+V244R,

wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1).

Also disclosed herein is an isolated, recombinant, substantially pure, or non-naturally occurring variant protease (e.g., variant subtilisin) having proteolytic activity, said variant protease comprising an amino acid sequence having at least 90%, at least 91%, at least 92%, at least 93%, at least 94%, at least 95%, at least 96%, at least 97%, at least 98%, at least 99%, at least 99.5%, or 100% sequence identity to the amino acid sequence of SEQ ID NO:1, said variant protease comprising one of the following sets of amino acid substitutions relative to SEQ ID NO:1:

    (i) N76D+S87R+G118R+S128L+P129Q+S130A, with the proviso that said variant protease does not comprise both N248R+S188D, or

    (ii) N76D+S87R+G118R+S128L+P129Q+S130A+S188D+V244R,

wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1).

Also disclosed herein isan isolated, recombinant, substantially pure, or non-naturally occurring variant protease (e.g., variant subtilisin) having proteolytic activity, said variant protease comprising an amino acid sequence having at least 95%, at least 96%, at least 97%, at least 98%, at least 99%, at least 99.5%, or 100% sequence identity to the amino acid sequence of SEQ ID NO:1, said variant protease comprising one of the following sets of amino acid substitutions relative to SEQ ID NO:1:

    (i) N76D+S87R+G118R+S128L+P129Q+S130A, with the proviso that said variant protease does not comprise both N248R and S188D, or

    (ii) N76D+S87R+G118R+S128L+P129Q+S130A+S188D+V244R,

wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1).

Also disclosed herein isan isolated, substantially pure, or recombinant variant protease (e.g., variant subtilisin) of a parent protease (e.g., subtilisin), said variant protease comprising an amino acid sequence comprising one of the following sets of amino acid substitutions relative to the amino acid sequence of the parent protease:

    (i) N76D+S87R+G118R+S128L+P129Q+S130A, with the proviso that said variant protease does not comprise both N248R and S188D, or

    (ii) N76D+S87R+G118R+S128L+P129Q+S130A+S188D+V244R.

Also disclosed herein isan isolated, substantially pure, or recombinant variant subtilisin of a parent subtilisin, said variant subtilisin comprising an amino acid sequence comprising one of the following sets of amino acid substitutions relative to the amino acid sequence of the parent subtilisin:

    (i) N76D+S87R+G118R+S128L+P129Q+S130A, with the proviso that said variant subtilisin does not comprise both N248R and S188D, or

    (ii) N76D+S87R+G118R+S128L+P129Q+S130A+S188D+V244R.

**EP 2 558 573 B1**

Also disclosed herein isan isolated, recombinant, or non-naturally occurring variant protease (e.g., variant subtilisin) of a parent protease, said parent protease comprising an amino acid sequence having at least 95%, at least 96%, at least 97%, at least 98%, at least 99%, at least 99.5%, or 100% identity to the amino acid sequence of SEQ ID NO:1, said variant protease having proteolytic activity and comprising one of the following sets of amino acid substitutions versus said parent protease:

> (i) N76D+S87R+G118R+S128L+P129Q+S130A, with the proviso that said variant protease does not comprise both N248R and S188D, or
> (ii) N76D+S87R+G118R+S128L+P129Q+S130A+S188D+V244R,

wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1).

Also disclosed herein isan isolated, recombinant, or non-naturally occurring variant protease (e.g., variant subtilisin) having proteolytic activity, said variant protease comprising an amino acid sequence having at least 95%, at least 96%, at least 97%, at least 98%, at least 99%, at least 99.5%, or 100% sequence identity to the amino acid sequence of SEQ ID NO:1, said variant protease amino acid sequence comprising a substitution of the amino acid residue in each of amino acid positions 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue and/or (iii) an asparagine at position 76 or a substitution of the asparagine residue at position 248 with a different amino acid residue that is not an aspartic acid residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). In some such variant proteases, the amino acid sequence of the variant protease comprises a serine residue at amino acid position 188 or an arginine residue at amino acid position 248. For some such variant proteases, the variant protease amino acid sequence comprises a substitution of the amino acid residue in each of amino acid positions 87, 118, 128, 129, and 130 relative to SEQ ID NO:1 with a different amino acid to yield a variant protease having a net charge of -2, -1, 0, +1, +2, +3, or +4. For some such variant proteases, in the amino acid sequence of the variant protease, (i) the amino acid residue at position 76 comprises an asparagine residue, a glutamic acid residue or an uncharged (neutral) amino acid residue selected form the group consisting of serine, threonine, asparagine, glutamine, alanine, isoleucine, leucine, methionine, phenylalanine, tryptophan, tyrosine, and valine neutral, (ii) the amino acid residue at position 118 is substituted with a positively charged amino acid residue selected from the group consisting of arginine, histidine, and lysine, and (iii) the amino acid residue at each of positions 128, 129, and 130 is substituted with an uncharged amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions S87R+G118R+S128L+P129Q+S130A.

Also disclosed herein isan isolated, recombinant, or non-naturally occurring variant protease (e.g., variant subtilisin) having proteolytic activity, said variant protease comprising an amino acid sequence having at least 95%, at least 96%, at least 97%, at least 98%, at least 99%, at least 99.5%, or 100% sequence identity to the amino acid sequence of SEQ ID NO:1, said variant protease comprising one of the following sets of amino acid substitutions relative to SEQ ID NO:1:

> (i) S87R+G118R+S128L+P129Q+S130A, with the proviso that said variant protease does not comprise all of N248R, S188D, and N76D, or
> (ii) S87R+G118R+S128L+P129Q+S130A+S188D+V244R, with the proviso that said variant protease does not comprise N76D,

wherein amino acid positions are numbered according to the numbering of corresponding amino acid positions in the amino acid sequence of the subtilisin BPN' shown in SEQ ID NO:2 as determined by alignment of the variant protease amino acid sequence with the subtilisin BPN' amino acid sequence.

Also disclosed herein isan isolated, recombinant, or non-naturally occurring variant protease (e.g., variant subtilisin) having proteolytic activity, said variant protease comprising an amino acid sequence which (a) differs from the amino acid sequence shown in SEQ ID NO:1 from 9 to 15 amino acid residues and (b) comprises amino acid substitutions N76D+S87R+G118R+S128L+P129Q+S130A+S118D+N248R, wherein amino acid positions are num-

19

bered according to the numbering of corresponding amino acid positions in the amino acid sequence of the subtilisin BPN' shown in SEQ ID NO:2 as determined by alignment.

Also disclosed herein isan isolated, recombinant, or non-naturally occurring variant protease (e.g., variant subtilisin) having proteolytic activity, said variant protease comprising an amino acid sequence which (a) differs from the amino acid sequence shown in SEQ ID NO:1 by no more than 30, no more than 25, no more than 20, no more than 15, no more than 14, no more than 13, no more than 12, no more than 11, no more than 10, no more than 9, or no more than 8amino acid residue(s) and (b) comprises a substitution of the amino acid residue in each of amino acid positions 76, 87, 118, 128, 129, 130, 188, and 244 of SEQ ID NO:1 with a different amino acid residue, wherein amino acid positions are numbered according to the numbering of corresponding amino acid positions in the amino acid sequence of the subtilisin BPN' shown in SEQ ID NO:2 as determined by alignment of the variant protease amino acid sequence with the subtilisin BPN' amino acid sequence. Some such variant protease polypeptides differ from the polypeptide sequence of SEQ ID NO:1 by one or more amino acid deletion(s), amino acid addition(s) or insertion(s), and/or amino acid substitution(s). An amino acid substitution is typically made with one of the other 19 naturally occurring amino acids and may be a conservative or non-conservative amino acid substitution. Examples of conservative amino acid substitutions are discussed elsewhere herein. In one aspect, at least one amino acid is deleted and/or at least one amino acid is substituted in the sequence such that the resultant polypeptide exhibits enzymatic activity (e.g., proteolytic activity), as determined by standard assays well known in the art, including, e.g., an assay described herein.

Some such variant proteases (e.g., variant subtilisins) having proteolytic activity comprise an amino acid sequence which (a) differs from the amino acid sequence shown in SEQ ID NO:1 in no more than 25, no more than 20, no more than 15, no more than 14, no more than 13, no more than 12, no more than 11, no more than 10, no more than 9, or no more than 8 amino acid residues, and (b) comprise a substitution of the amino acid residue in each of amino acid at positions 76, 87, 118, 128, 129, 130, 188, and 244 of SEQ ID NO:1, wherein amino acid positions are numbered according to the numbering of corresponding amino acid positions in the amino acid sequence of the subtilisin BPN' shown in SEQ ID NO:2 as determined by alignment of the variant protease amino acid sequence with the subtilisin BPN' amino acid sequence. Some such variant protease polypeptides differ from the polypeptide sequence of SEQ ID NO:1 by one or more amino acid deletion(s), amino acid addition(s) or insertion(s), and/or amino acid substitution(s). An amino acid substitution is typically made with one of the other 19 naturally occurring amino acids and may be a conservative or non-conservative amino acid substitution. Exemplary conservative amino acid substitutions are discussed elsewhere herein. In one aspect, at least one amino acid is deleted and/or at least one amino acid is substituted in the sequence such that the resultant polypeptide exhibits enzymatic activity (e.g., proteolytic activity), as determined by an assay well known in the art, including, e.g., an assay described herein.

Also disclosed is an isolated, recombinant, or non-naturally occurring variant protease (e.g., variant subtilisin) having proteolytic activity, said variant protease comprising an amino acid sequence which:

(a) differs from the amino acid sequence shown in SEQ ID NO:1 in or by no more than 30, no more than 25, no more than 20, no more than 15, no more than 14, no more than 13, no more than 12, no more than 11, no more than 10, no more than 9, no more than 8, no more than 7, or no more than 6 amino acid residue(s); and

(b) comprises a substitution of the amino acid residue in each of amino acid positions 76, 87, 118, 128, 129, and 130 of SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of subtilisin BPN' shown in SEQ ID NO:2, as determined by alignment of the variant protease amino acid sequence with the subtilisin BPN' amino acid sequence. Some such variant protease polypeptides differ from the polypeptide sequence of SEQ ID NO:1 by one or more amino acid deletion(s), addition(s) or insertion(s), and/or amino acid substitution(s). An amino acid substitution is typically made with one of the other 19 naturally occurring amino acids and may be a conservative or non-conservative substitution. Exemplary conservative amino acid substitutions are discussed elsewhere herein. In one aspect, at least one amino acid is deleted and/or at least one amino acid is substituted in the sequence such that the resultant polypeptide exhibits enzymatic activity (e.g., proteolytic activity), as determined by an assay well known in the art, including, e.g., an assay described herein.

Also disclosed is an isolated, recombinant, or non-naturally occurring variant protease (e.g., variant subtilisin) having proteolytic activity, said variant protease comprising an amino acid sequence which:

(a) differs from the amino acid sequence of SEQ ID NO:1 in no more than 25, no more than 20, no more than 15, no more than 14, no more than 13, no more than 12, no more than 11, no more than 10, no more than 9, no more than 8, no more than 7, or no more than 6 amino acid residues; and

(b) comprises a substitution of the amino acid residue in each of amino acid positions 76, 87, 118, 128, 129, and 130 of SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of the subtilisin BPN' shown in SEQ ID NO:2 as determined by alignment of the variant protease amino acid sequence with the subtilisin BPN' amino acid sequence. In one aspect, some such variant proteases comprise (i) a serine residue at amino acid position 188 or a substitution of the serine residue at position 188 with a different amino acid residue that is not an aspartic acid residue, and (ii) a substitution of the asparagine residue at position 248 with an arginine residue. In one aspect, some such variant proteases comprise (i) a substitution of the serine residue at position 188 with an aspartic acid residue, and (ii) an asparagine residue at position 248 or a substitution of the asparagine residue at position 248 with a different amino acid residue that is not an arginine residue.

Some such variant protease polypeptides differ from the polypeptide sequence of SEQ ID NO:1 by one or more amino acid deletion(s), addition(s) or insertion(s), and/or amino acid substitution(s). An amino acid substitution is typically made with one of the other 19 naturally occurring amino acids and may be a conservative or non-conservative substitution. Examples of conservative amino acid substitutions are discussed elsewhere herein. In one aspect, at least one amino acid is deleted and/or at least one amino acid is substituted in the sequence such that the resultant polypeptide exhibits enzymatic activity (e.g., proteolytic activity), as determined by an assay well known in the art, including, e.g., an assay described herein. For some such variant proteases, the amino acid sequence of the variant protease comprises a serine residue at amino acid position 188 or an arginine residue at amino acid position 248. For some such variant proteases, the variant protease amino acid sequence comprises a substitution of the amino acid residue in each of amino acid positions 76, 87, 118, 128, 129, and 130 relative to SEQ ID NO:2 with a different amino acid to yield a variant protease having a net charge of -2, -1, 0, +1, +2, +3, or +4. For some such variant proteases, in the amino acid sequence of the variant protease, (i) the amino acid residue at position 76 is substituted with a negatively charged amino acid residue selected from the group of consisting of aspartic acid and glutamic acid, (ii) the amino acid residue at each of positions 87 and 118 is substituted with a positively charged amino acid residue selected from the group consisting of arginine, histidine, and lysine, and (iii) the amino acid residue at each of positions 128, 129, and 130 is substituted with an uncharged (neutral) amino acid residue selected form the group consisting of serine, threonine, asparagine, glutamine, alanine, isoleucine, leucine, methionine, phenylalanine, tryptophan, tyrosine, and valine. For some such variant proteases, the amino acid sequence of the variant protease comprises amino acid substitutions N76D+S87R+G118R+S128L+P129Q+S130A.

Also disclosed herein isan isolated, recombinant, or non-naturally occurring variant protease (e.g., variant subtilisin) having proteolytic activity, said variant protease comprising an amino acid sequence having at least 99% or 99.5% sequence identity to SEQ ID NO:7 or SEQ ID NO:8.

Also disclosed herein isan isolated, recombinant, or non-naturally occurring variant protease having proteolytic activity, said variant protease comprising an amino acid sequence which differs from the amino acid sequence of SEQ ID NO:7 or SEQ ID NO:8 in no more than 1 amino acid residue. For example, the variant protease sequence may differ from the amino acid sequence of SEQ ID NO:7 or SEQ ID NO:8 by an amino acid substitution (such as a non-conservative or conservative amino acid substitution), amino acid deletion, or amino acid insertion.

Also disclosed herein isan isolated, recombinant, or non-naturally occurring variant protease having proteolytic activity, said variant protease comprising an amino acid sequence which differs from the amino acid sequence of SEQ ID NO:7 in no more than 1 amino acid substitution (such as a non-conservative or conservative amino acid substitution), in no more than 15, 14, 13, 12, 11, 10, 9, 8, 7, 6, 5, 4, 3, 2, or 1 amino acid deletion(s), and/or in no more than 15, 14, 13, 12, 11, 10, 9, 8, 7, 6, 5, 4, 3, 2, or 1 amino acid deletion amino acid insertion(s) or addition(s).

Also disclosed herein isan isolated, recombinant, or non-naturally occurring variant protease having proteolytic activity, said variant protease comprising an amino acid sequence which differs from the amino acid sequence of SEQ ID NO:8 in no more than 1 amino acid substitution (such as a non-conservative or conservative amino acid substitution), in no more than 15, 14, 13, 12, 11, 10, 9, 8, 7, 6, 5, 4, 3, 2, or 1 amino acid deletion(s), and/or in no more than 15, 14, 13, 12, 11, 10, 9, 8, 7, 6, 5, 4, 3, 2, or 1 amino acid deletion amino acid insertion(s) or addition(s).

As noted above, the variant protease polypeptides of the invention have enzymatic activities (such as, e.g., proteolytic activities) and thus are useful in cleaning applications, such as, e.g., but not limited to, methods for cleaning dishware items, tableware items, fabrics, and items having hard surfaces (e.g., the hard surface of a table, table top, wall, furniture item, floor, ceiling, etc.). Exemplary cleaning compositions comprising one or more variant protease polypeptides of the invention are described *infra.* The enzymatic activity (e.g., protease activity) of a variant protease polypeptide of the invention can be determined readily using procedures well known to those of ordinary skill in the art. The Examples presented *infra* describe methods for evaluating the enzymatic activity, cleaning performance, and/or washing performance. The performance of variant proteases of the invention in removing stains, such as, e.g., a stain induced by a protein(s), cleaning hard surfaces, or cleaning laundry, dishware or tableware item(s) can be readily determined using procedures well known in the art and/or by using procedures set forth in the Examples.

A polypeptide disclosed herein can be subject to various changes, such as one or more amino acid insertions, deletions, and/or substitutions, either conservative or non-conservative, including where, e.g., such changes do not substantially alter the enzymatic activity of the polypeptide. Similarly, a nucleic acid of the invention can also be subject to various changes, such as one or more substitutions of one or more nucleic acids in one or more codons such that a particular codon encodes the same or a different amino acid, resulting in either a silent variation (e.g., mutation in a nucleotide sequence results in a silent mutation in the amino acid sequence, e.g., when the encoded amino acid is not altered by the nucleic acid mutation) or non-silent variation, one or more deletions of one or more nucleic acids (or codons) in the sequence, one or more additions or insertions of one or more nucleic acids (or codons) in the sequence, and/or cleavage of or one or more truncations of one or more nucleic acids (or codons) in the sequence. Many such changes in the nucleic acid sequence may not substantially alter the enzymatic activity of the resulting encoded variant protease compared to the variant protease encoded by the original nucleic acid sequence. A nucleic acid of the invention can also be modified to include one or more codons that provide for optimum expression in an expression system (e.g., bacterial expression system), while, if desired, said one or more codons still encode the same amino acid(s).

In one aspect, a genus of polypeptides disclosed herein may comprise variant protease polypeptides having the desired enzymatic activity (e.g., protease activity or cleaning performance activity) which comprise sequences having the amino acid substitutions described herein and also which comprise one or more additional amino acid substitutions, such as conservative and non-conservative substitutions, wherein the polypeptide exhibits, maintains, or approximately maintains the desired enzymatic activity (e.g., protease activity or subtilisin activity), e.g., as reflected in the cleaning activity or performance of the variant protease. Amino acid substitutions in accordance with the present disclosure may include, e.g., but are not limited to, one or more non-conservative substitutions and/or one or more conservative amino acid substitutions. A conservative amino acid residue substitution typically involves exchanging a member within one functional class of amino acid residues for a residue that belongs to the same functional class (identical amino acid residues are considered functionally homologous or conserved in calculating percent functional homology). A conservative amino acid substitution typically involves the substitution of an amino acid in an amino acid sequence with a functionally similar amino acid. For example, alanine, glycine, serine, and threonine are functionally similar and thus may serve as conservative amino acid substitutions for one another. Aspartic acid and glutamic acid may serve as conservative substitutions for one another. Asparagine and glutamine may serve as conservative substitutions for one another. Arginine, lysine, and histidine may serve as conservative substitutions for one another. Isoleucine, leucine, methionine, and valine may serve as conservative substitutions for one another. Phenylalanine, tyrosine, and tryptophan may serve as conservative substitutions for one another.

Other conservative amino acid substitution groups can be envisioned. For example, amino acids can be grouped by similar function or chemical structure or composition (e.g., acidic, basic, aliphatic, aromatic, sulfur-containing). For instance, an Aliphatic grouping may comprise: Glycine (G), Alanine (A), Valine (V), Leucine (L), Isoleucine (I). Other groups containing amino acids that are considered conservative substitutions for one another include: Aromatic: Phenylalanine (F), Tyrosine (Y), Tryptophan (W); Sulfur-containing: Methionine (M), Cysteine (C); Basic: Arginine (R), Lysine (K), Histidine (H); Acidic: Aspartic acid (D), Glutamic acid (E); Non-polar uncharged residues, Cysteine (C), Methionine (M), and Proline (P); Hydrophilic Uncharged Residues: Serine (S), Threonine (T), Asparagine (N), and Glutamine (Q). See also Creighton (1984) Proteins: STRUCTURE AND MOLECULAR PROPERTIES (2d Ed. 1993), W.H. Freeman and Company for additional groupings of amino acids. Listing of a polypeptide sequence herein, in conjunction with the above substitution groups, provides an express listing of all conservatively substituted polypeptide sequences.

More conservative substitutions exist within the amino acid residue classes described above, which also or alternatively can be suitable. Conservation groups for substitutions that are more conservative include: valine-leucine-isoleucine, phenylalanine-tyrosine, lysine-arginine, alanine-valine, and asparagine-glutamine. Thus, for example, disclosed herein is an isolated or recombinant variant protease polypeptide (e.g., variant subtilisin) having proteolytic activity, said variant protease polypeptide comprising an amino acid sequence having at least 90%, 95%, 96%, 97%, 98%, 99%, or 99.5% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of the subtilisin BPN' shown in SEQ ID NO:2 as determined by optimal alignment of the variant protease amino acid sequence with the BPN' sequence, and wherein an amino acid residue in the variant protease sequence at an amino acid position other than at position 76, 87, 118, 128, 129, or 130 differs from the corresponding amino acid position in SEQ ID NO:1 (as determined by alignment using the BPN' numbering scheme) by a conservative amino acid substitution, wherein the resultant variant protease exhibits or maintains a desired enzymatic activity or cleaning performance activity. A conservative substitution of one amino acid for another in a variant protease of the invention is not expected to alter significantly the enzymatic activity or cleaning performance activity of the variant protease. Enzymatic activity or cleaning performance activity of the resultant protease can be readily determined using the standard assays and the assays described herein. Accordingly, the variant protease may include one or more additional conservative amino acid substitutions other than at the positions specified above while maintaining the desired enzymatic activity or cleaning performance activity. In some aspects, at least 10%, 20%, 50%, 60%, 70%, or more (e.g., at least 75%, 80%, 90%, 95%, 96%, 97%, 98%, 99%, 99.5%) of the substitutions in an amino acid sequence variant protease comprise substitutions of one or more amino acid residues at amino acid positions other than positions 76, 87, 118, 128, 129, and 130 in a variant protease polypeptide sequence of the invention relative to SEQ ID NO:1, wherein amino acid positions are numbering by alignment with SEQ ID NO:2 and using the BPN' numbering scheme, with one or more amino acid residues, respectively, that are within the same functional homology class (as determined by any suitable classification system) as the amino acid residues of the polypeptide sequence that they each replace.

Conservatively substituted variations of a polypeptide sequence of the disclosure (e.g., variant protease of the disclosure) include substitutions of a small percentage, sometimes less than 25%, 20%, 15%, 14%, 13%, 12%, 11%, 10%, 9%, 8%, 7%, or 6% of the amino acids of the polypeptide sequence, or less than 5%, 4%, 3%, 2%, or 1%, of the amino acids of the polypeptide sequence, with a conservatively selected amino acid of the same conservative substitution group.

As described elsewhere herein in greater detail (and in the Examples set forth below), polypeptides of the invention may have cleaning abilities that may be compared to known proteases, including known subtilisins. Exemplary known subtilisin proteases include, but are not limited to, for example, *B. lentus* subtilisin GG36 (also referred to herein as "GCI-P036"), *B. amyloliquefaciens* subtilisin BPN', *B. amyloliquefaciens* subtilisin BPN'-Y217L, and *B. clausii* PB92 (also referred to herein as "GCI-P037"). Another known Subtilisin protease used for comparison reference herein is referred to as "GCI-P038".

The amino acid sequence of the mature *B. lentus* subtilisin GG36 protein (GCI-P036) is:

AQSVPWGISRVQAPAAHNRGLTGSGVKVAVLDTGIS:THPDLNIRGGASFVPGEPST:QDGNGHG

THVAGTIAALNNSIGVLGVAPSAELYAVKVLGASGSGSVSSIAQGLEWAGNNGMHVANLSLGS

PSPSATLEQAVNSATSRGVLVVAASGNSGAGSISYPARYANAMAVGATDQNNNRASFSQYGAG

LDIVAPGVNVQSTYPGSTYASLNGTSMATPHVAGAAALVKQKNPSWSNVQIRNHL

KNTATSLGSTNLYGSGLVNAEAATR (SEQ ID NO:1)

The amino acid sequence of mature *B. amyloliquefaciens* subtilisin BPN' protein is:

AQSVPYGVSQIKAPALHSQGYTGSNVKVAVIDSGIDSSHPDLKVAGGASMVPSETNPFQDNNSH

GTHVAGTVAALNNSIGVLGVAPSASLYAVKVLGADGSGQYSWIINGIEWAIANNMDVINMSLG

GPSGSAALKAAVDKAVASGVVVVAAAGNEGTSGSSSTVGYPGKYPSVIAVGAVDSSNQRASFS

SVGPELDVMAPGVSIQSTLPGNKYGAYNGTSMASPHVAGAAALILSKHPNWTNTQVRSSL

ENTTTKLGDSFYYGKGLINVQAAAQ (SEQ ID NO:2)

The amino acid sequence of mature *B. amyloliquefaciens* subtilisin BPN'-Y217L protein (also referred to as "FNA") is:

AQSVPYGVSQIKAPALHSQGYTGSNVKVAVIDSGIDSSHPDLKVAGGASMVPSETNPFQDNNSH

GTHVAGTVAALNNSIGVLGVAPSASLYAVKVLGADGSGQYSWIINGIEWAIANNMDVINMSLG

GPSGSAALKAAVDKAVASGVVVVAAAGNEGTSGSSSTVGYPGKYPSVIAVGAVDSSNQRASFS

SVGPELDVMAPGVSIQSTLPGNKYGALNGTSMASPHVAGAAALILSKHPNWTNTQVRSSL

ENTTTKLGDSFYYGKGLINVQAAAQ (SEQ ID NO:3)

The amino acid sequence of the mature PB92 (GCI-P037) reference subtilisin protease protein is:

AQSVPWGISRVQAPAAHNRGLTGSGVKVAVLDTGISTHPDLNIRGGASFVPGEPSTQDGNGHGT
HVAGTIAALNNSIGVLGVAPNAELYAVKVLGASGSGSVSSIAQGLEWAGNNGMHVANLSLGSP
SPSATLEQAVNSATSRGVLVVAASGNSGAGSISYPARYANAMAVGATDQNNNRASFSQYGAGL
DIVAPGVNVQSTYPGSTYASLNGTSMATPHVAGAAALVKQKNPSWSNVQIRNHLKNTATSLGS
TNLYGSGLVNAEAATR (SEQ ID NO:4)

The amino acid sequence of the mature GCI-P038 reference subtilisin protease protein is:

AQSVPWGISRVQAPAAHNRGLTGSGVKVAVLDTGISTHPDLNIRGGASFVPGEPSTQDGNGHGT
HVAGTIAALNNSIGVLGVAPNAELYAVKVLGASGSGSVSSIAQGLEWAGNN**V**MHVANLSLG**LQ**
**A**PSATLEQAVNSATSRGVLVVAASGNSGAGSISYPARYANAMAVGATDQNNNRASFSQYGAGL
DIVAPGVNVQSTYPGSTYASLNGTSMATPHVAGAAALVKQKNPSWSNVQIRNHLKNTATSLGS
TNLYGSGLVNAEAATR (SEQ ID NO:5)

Nucleic Acids of the Invention

The invention provides isolated, non-naturally occurring, or recombinant nucleic acids (also referred to herein as polynucleotides), which may be collectively referred to as "nucleic acids of the invention" or "polynucleotides of the invention," which encode polypeptides of the invention, as defined in the claims. Nucleic acids of the invention, as defined in the claims, are useful in recombinant production (e.g., expression) of polypeptides of the invention, typically through expression of a plasmid expression vector comprising a sequence encoding the polypeptide of interest or fragment thereof. As discussed above, polypeptides include variant protease polypeptides, including, e.g., variant subtilisin polypeptides, having enzymatic activity (e.g., proteolytic activity) which are useful in cleaning applications and cleaning compositions for cleaning an item or a surface (e.g., surface of an item) in need of cleaning.

In one aspect, the invention provides an isolated, recombinant, substantially pure, or non-naturally occurring nucleic acid comprising a nucleotide sequence encoding any polypeptide (including any fusion protein, etc.) of the invention as defined in the claims. The invention also provides an isolated, recombinant, substantially pure, or non-naturally-occurring nucleic acid comprising a nucleotide sequence encoding a combination of two or more of any polypeptides of the invention described above and elsewhere herein.

For example, disclosed herein is an isolated, recombinant, substantially pure, or non-naturally-occurring nucleic acid comprising a polynucleotide sequence that encodes a variant protease (e.g., variant subtilisin) having proteolytic activity, said variant protease comprising an amino acid sequence having at least 85%, 90%, 91%, 92%, 93%, 94%, 95%, 96%, 97%, 98%, 99%, 99.5%, or 100% sequence identity to the amino acid sequence of SEQ ID NO:1, said variant protease amino acid sequence comprising a substitution of each of the amino acid residues in positions 76, 87, 118, 128, 129, 130, 188, and 244 of SEQ ID NO:1 with a different amino acid residue, wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence (see Figure 1). Some such variant proteases comprise an amino acid sequence comprising a substitution of the amino acid residue in each of amino acid positions 76, 87, 118, 128, 129, 130, 188, and 244 of SEQ ID NO:1 with a different amino acid residue to yield a variant protease having a net charge of -2, -1, 0, +1, +2, +3, or +4. In some such variant proteases: (i) the amino acid residue at each of positions 76 and 188 is substituted with a negatively charged amino acid residue selected from the group consisting of aspartic acid and glutamic acid; (ii) the amino acid residue at each of positions 87, 118, and 244 is substituted with a positively charged amino acid residue selected from the group consisting of arginine, histidine, and lysine; and (iii) the amino acid residue at each of positions 128, 129, and 130 is substituted with a neutral amino acid residue selected from the group consisting of serine, threonine, asparagine, glutamine, alanine, isoleucine, leucine, methionine, phenylalanine, tryptophan, tyrosine, and valine. For some such variant proteases, wherein the amino acid sequence of said variant protease comprises amino acid substitutions N76D+S87R+G118R+S128L+P129Q+S130A+S188D+V244R.

Also disclosed herein is an isolated, recombinant, substantially pure, or non-naturally occurring nucleic acid comprising a polynucleotide sequence which encodes a variant protease (e.g., variant subtilisin) comprising the amino acid sequence set forth in SEQ ID NO:6. In another aspect, the invention provides an isolated, recombinant, substantially pure, or non-naturally occurring nucleic acid comprising a polynucleotide sequence having at least 90%, 91%, 92%, 93%, 94%, 95%, 96%, 97%, 98%, 99%, or 100% nucleotide sequence identity to the polynucleotide sequence of SEQ ID NO:9 or a complementary polynucleotide sequence thereof. Also disclosed herein isan isolated, recombinant, substantially pure, or non-naturally occurring nucleic acid comprising the polynucleotide sequence of SEQ ID NO:9 or a complementary polynucleotide sequence thereof.

Also disclosed herein isan isolated, recombinant, substantially pure, or non-naturally occurring nucleic acid comprising a polynucleotide sequence which encodes a variant protease (e.g., variant subtilisin) comprising the amino acid sequence set forth in SEQ ID NO:8. Also disclosed herein isan isolated, recombinant, substantially pure, or non-naturally occurring nucleic acid comprising a polynucleotide sequence having at least 90%, 91%, 92%, 93%, 94%, 95%, 96%, 97%, 98%, 99%, or 100% nucleotide sequence identity to the polynucleotide sequence of SEQ ID NO:11 or a complementary polynucleotide sequence thereof. Also disclosed herein isan isolated, recombinant, substantially pure, or non-naturally occurring nucleic acid comprising the polynucleotide sequence of SEQ ID NO:11 or a complementary polynucleotide sequence thereof.

Also disclosed herein isan isolated, recombinant, substantially pure, or non-naturally-occurring nucleic acid comprising a polynucleotide sequence that encodes a variant protease (e.g., variant subtilisin) having proteolytic activity, said variant protease comprising an amino acid sequence having at least 85%, 90%, 91%, 92%, 93%, 94%, 95%, 96%, 97%, 98%, 99%, 99.5%, or 100% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of the subtilisin BPN' shown in SEQ ID NO:2 as determined by alignment of the variant protease amino acid sequence with the subtilisin BPN' amino acid sequence. Some such encoded variant proteases comprise an amino acid sequence comprising a serine residue at amino acid position 188 or an arginine residue at amino acid position 248. Some such variant proteases comprise (i) a serine residue at position 188 or a substitution of the serine residue at position 188 with a different amino acid residue that is not an aspartic acid residue, and (ii) a substitution of the asparagine residue at position 248 with an arginine residue. Some such variant proteases comprise (i) a substitution of the serine residue at position 188 with an aspartic acid residue, and (ii) an asparagine residue at position 248 or a substitution of the asparagine residue at position 248 with a different amino acid residue that is not an arginine residue. Some such variant proteases comprise an amino acid sequence comprising a substitution of the amino acid residue in each of amino acid positions 76, 87, 118, 128, 129, and 130 relative to SEQ ID NO:1 with a different amino acid to yield a variant protease having a net charge of -2, -1, 0, +1, +2, +3, or +4. In some such variant proteases: (i) the amino acid residue at position 76 is substituted with a negatively charged amino acid residue selected from the group of consisting of aspartic acid and glutamic acid, (ii) the amino acid residue at each of positions 87 and 118 is substituted with a positively charged amino acid residue selected from the group consisting of arginine, histidine, and lysine, and (iii) the amino acid residue at each of positions 128, 129, and 130 is substituted with an uncharged amino acid residue selected form the group consisting of serine, threonine, asparagine, glutamine, alanine, isoleucine, leucine, methionine, phenylalanine, tryptophan, tyrosine, and valine.

In one aspect, the invention provides an isolated, recombinant, substantially pure, or non-naturally occurring nucleic acid comprising a polynucleotide sequence which encodes a variant protease (e.g., variant subtilisin) comprising the amino acid sequence set forth in SEQ ID NO:7.

Also disclosed herein isan isolated, recombinant, substantially pure, or non-naturally occurring nucleic acid comprising a polynucleotide sequence having at least 90%, 91%, 92%, 93%, 94%, 95%, 96%, 97%, 98%, 99%, or 100% nucleotide sequence identity to the polynucleotide sequence of SEQ ID NO:10 or a complementary polynucleotide sequence thereof. Also disclosed herein isan isolated, recombinant, substantially pure, or non-naturally occurring nucleic acid comprising the polynucleotide sequence of SEQ ID NO:10 or a complementary polynucleotide sequence thereof.

Also disclosed is an isolated, recombinant, substantially pure, or non-naturally occurring nucleic acid comprising a polynucleotide sequence which encodes a variant protease having proteolytic activity, said variant protease (e.g., variant subtilisin) comprising an amino acid sequence which:

(a) differs from the amino acid sequence of SEQ ID NO:1 by no more than 25, no more than 20, no more than 15, no more than 14, no more than 13, no more than 12, no more than 11, no more than 10, no more than 9, no more than 8, no more than 7, or no more than 6 amino acid residues; and

(b) comprises a substitution of the amino acid residue in each of amino acid positions 76, 87, 118, 128, 129, and 130 of SEQ ID NO:1 with a different amino acid residue, and (i) a serine residue at amino acid position 188 or a substitution of the serine residue at position 188 with a different amino acid residue that is not an aspartic acid residue and/or (ii) an asparagine residue at amino acid position 248 or a substitution of the asparagine residue at position 248 with a different amino acid residue that is not an arginine residue, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence. Some such encode variant proteases comprise an amino acid sequence comprising (i) a serine residue at amino acid position 188 or a substitution of the serine residue at position 188 with a different amino acid residue that is not an aspartic acid residue, and (ii) a substitution of the asparagine residue at position 248 with an arginine residue. Some such encoded variant proteases comprise an amino acid sequence comprising a substitution of the serine residue at position 188 with an aspartic acid residue, and (ii) an asparagine residue at position 248 or a substitution of the asparagine residue at position 248 with a different amino acid residue that is not an arginine residue.

Also disclosed herein isan isolated, recombinant, substantially pure, or non-naturally occurring variant protease (e.g., variant subtilisin) having proteolytic activity, said variant protease comprising an amino acid sequence which (a) differs from the amino acid sequence shown in SEQ ID NO:1 by no more than 25, no more than 20, no more than 15, no more than 14, no more than 13, no more than 12, no more than 11, no more than 10, no more than 9, or no more than 8 amino acid residues, and (b) comprise a substitution of the amino acid residue in each of amino acid at positions 76, 87, 118, 128, 129, 130, 188, and 244 of SEQ ID NO:1, wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence.

Also disclosed herein isan isolated, recombinant, substantially pure, or non-naturally-occurring nucleic acid comprising a polynucleotide sequence having at least 85%, 90%, 91%, 92%, 93%, 94%, 95%, 96%, 97%, 98%, 99%, 99.5%, or 100% sequence identity to the polynucleotide sequence of any of SEQ ID NOS:9, 10, and 11.

Nucleic acids of the invention can be generated by using any suitable synthesis, manipulation, and/or isolation techniques, or combinations thereof. For example, a polynucleotide of the invention may be produced using standard nucleic acid synthesis techniques, such as solid-phase synthesis techniques that are well-known to those skilled in the art. In such techniques, fragments of up to 50 or more nucleotide bases are typically synthesized, then joined (e.g., by enzymatic or chemical ligation methods, or polymerase mediated recombination methods) to form essentially any desired continuous nucleic acid sequence. The synthesis of the nucleic acids of the invention can be also facilitated (or alternatively accomplished), by chemical synthesis using, e.g., the classical phosphoramidite method, which is described in, e.g., Beaucage et al. (1981) Tetrahedron Letters 22:1859-69, or the method described by Matthes et al. (1984) EMBO J. 3:801-05, e.g., as is typically practiced in automated synthetic methods. Nucleic acids of the invention also can be produced by using an automatic DNA synthesizer. Customized nucleic acids can be ordered from a variety of commercial sources, such as The Midland Certified Reagent Company (mcrc@oligos.com), the Great American Gene Company (worldwide website address genco.com), Operon Technologies Inc. (Alameda, Calif.), and DNA2.0 (Menlo Park, CA). Other techniques for synthesizing nucleic acids and related principles are described in, e.g., Itakura et al., Annu. Rev. Biochem. 53:323 (1984) and Itakura et al., Science 198:1056 (1984).

Recombinant DNA techniques useful in modification of nucleic acids are well known in the art. For example, techniques such as restriction endonuclease digestion, ligation, reverse transcription and cDNA production, and polymerase chain reaction (e.g., PCR) are known and readily employed by those of skill in the art. Useful recombinant DNA technology techniques and principles related thereto are described in Sambrook et al. (1989) Molecular Cloning: A Laboratory Manual, Cold Spring Harbor Press, and the third edition thereof (2001); Ausubel et al. (1994-1999) Current Protocols in Molecular Biology, Wiley Interscience Publishers; and Berger and Kimmel, "Guide to Molecular Cloning Techniques," in Methods in Enzymol. Vol. 152, Acad. Press, Inc., San Diego, CA.

Nucleotides of the invention may also be obtained by screening cDNA libraries (generated using mutagenesis techniques commonly used in the art, including those described herein) using one or more oligonucleotide probes that can hybridize to or PCR-amplify polynucleotides which encode a variant protease polypeptide(s) of the invention. Procedures for screening and isolating cDNA clones and PCR amplification procedures are well known to those of skill in the art and described in, e.g., Berger, Sambrook, and Ausubel, all *supra*. Some nucleic acids of the invention can be obtained by altering a naturally occurring polynucleotide backbone (e.g., that encodes an enzyme or parent protease) by, e.g., a known mutagenesis procedure (e.g., site-directed mutagenesis, site saturation mutagenesis, and *in vitro* recombination).

Methods for Making Modified Variant Proteases of the Invention

A variety of methods are known in the art that are suitable for generating modified polynucleotides of the invention that encode variant proteases of the invention, including, but not limited to, e.g., site-saturation mutagenesis, scanning mutagenesis, insertional mutagenesis, deletion mutagenesis, random mutagenesis, site-directed mutagenesis, and directed-evolution, as well as various other recombinatorial approaches. Methods for making modified polynucleotides and proteins (e.g., variant proteases) include DNA shuffling methodologies (see, e.g., Stemmer WP, 91(22):10747-51 (1994)), methods based on non-homologous recombination of genes e.g. ITCHY (Ostermeier et al., 7(10):2139-44 (1999)), SCRACHY (Lutz et al. 98(20):11248-53 (2001)), SHIPREC (Sieber et al., 19(5):456-60 (2001)), andNRR (Bittker et al., 20(10):1024-9 (2001); Bittker et al., 101(18):7011-6 (2004)), and methods that rely on the use of oligonucleotides to insert random and targeted mutations, deletions and/or insertions (Ness et al., 20(12):1251-5 (2002); Coco et al., 20(12):1246-50 (2002); Zha et al., 4(1):34-9 (2003), Glaser et al., 149(12):3903-13 (1992), and, Proc. Natl. Acad. Sci. USA 89(8):3581-5 (1992), Yáñez et al., 32(20):e158 (2004), Osuna et al., 32(17):e136 (2004), Gaytán et al., 29(3):E9 (2001), and Gaytán et al., 30(16):e84 (2002)).

## Vectors, Cells, and Methods for Producing Variant Proteases of the Invention

The present invention provides isolated or recombinant vectors comprising at least one polynucleotide of the invention described herein (e.g., a polynucleotide encoding a variant protease of the invention described herein), isolated or recombinant expression vectors or expression cassettes comprising at least one nucleic acid or polynucleotide of the invention, isolated, substantially pure, or recombinant DNA constructs comprising at least one nucleic acid or polynucleotide of the invention, isolated or recombinant cells comprising at least one polynucleotide of the invention, cell cultures comprising cells comprising at least one polynucleotide of the invention, cell cultures comprising at least one nucleic acid or polynucleotide of the invention, and compositions comprising one or more such vectors, nucleic acids, expression vectors, expression cassettes, DNA constructs, cells, cell cultures, or any combination or mixtures thereof.

In one aspect, the invention provides recombinant cells comprising at least one vector (e.g., expression vector or DNA construct) of the invention which comprises at least one nucleic acid or polynucleotide of the invention, as defined in the claims. Some such recombinant cells are transformed or transfected with such at least one vector. Such cells are typically referred to as host cells. Some such cells comprise bacterial cells, including, but are not limited to, e.g., *Bacillus sp.* cells, such as, e.g., *Bacillus subtilis* cells. The invention also provides recombinant cells (e.g., recombinant host cells) comprising at least one variant protease of the invention.

In one aspect, the invention provides a vector comprising a nucleic acid or polynucleotide of the invention. In one aspect, the vector is an expression vector or expression cassette in which a polynucleotide sequence of the invention which encodes a variant protease of the invention is operably linked to one or additional nucleic acid segments required for efficient gene expression (e.g., a promoter operably linked to the polynucleotide of the invention which encodes a variant protease of the invention). A vector may include a transcription terminator and/or a selection gene, such as an antibiotic resistance gene that enables continuous cultural maintenance of plasmid-infected host cells by growth in antimicrobial-containing media.

An expression vector may be derived from plasmid or viral DNA, or in alternative embodiments, contains elements of both. Exemplary vectors include, but are not limited to pXX, pC194, pJH101, pE194, pHP13 (Harwood and Cutting (eds.)), Molecular Biological Methods for Bacillus, John Wiley & Sons (1990), see, e.g., chapter 3; suitable replicating plasmids for *B. subtilis* include those listed on p. 92; Perego, M. (1993) Integrational Vectors for Genetic Manipulations in Bacillus subtilis, pp. 615-624; A. L. Sonenshein, J. A. Hoch, and R. Losick (ed.), Bacillus subtilis and other Gram-positive bacteria: biochemistry, physiology and molecular genetics, American Society for Microbiology, Washington, D.C.

For expression and production of a protein of interest (e.g., variant protease) in a cell, at least one expression vector comprising at least one copy of a polynucleotide encoding the modified protease, and preferably comprising multiple copies, is transformed into the cell under conditions suitable for expression of the protease. In one aspect, a polynucleotide sequence encoding the variant protease (as well as other sequences included in the vector) is integrated into the genome of the host cell, while in another aspect, a plasmid vector comprising a polynucleotide sequence encoding the variant protease remains as autonomous extra-chromosomal element within the cell. The invention provides both extrachromosomal nucleic acid elements as well as incoming nucleotide sequences that are integrated into the host cell genome. The vectors described herein are useful for production of the variant proteases of the invention. In one aspect, a polynucleotide construct encoding the variant protease is present on an integrating vector that enables the integration and optionally the amplification of the polynucleotide encoding the variant protease into the bacterial chromosome. Examples of sites for integration include are well known to those skilled in the art. In some embodiments, transcription of a polynucleotide encoding a variant protease of the invention is effectuated by a promoter that is the wild-type promoter for the selected precursor protease. In some other embodiments, the promoter is heterologous to the precursor protease, but is functional in the host cell. Specifically, examples of suitable promoters for use in bacterial host cells include, but are not limited to, e.g., the amyE, amyQ, amyL, pstS, sacB, pSPAC, pAprE, pVeg, pHpaII promoters, the promoter of the *B. stearothermophilus* maltogenic amylase gene, the *B. amyloliquefaciens* (BAN) amylase gene, the *B. subtilis* alkaline protease gene, the *B. clausii* alkaline protease gene the *B. pumilis* xylosidase gene, the *B. thuringiensis* cryIIIA, and the *B. licheniformis* alpha-amylase gene. Additional promoters include, but are not limited to the A4 promoter, as well as phage Lambda $P_R$ or $P_L$ promoters, and the E. coli lac, trp or tac promoters.

Variant proteases of the invention can be produced in host cells of any suitable Gram-positive microorganism, including bacteria and fungi. For example, in some embodiments, the variant protease is produced in host cells of fungal and/or bacterial origin. In some embodiments, the host cells are *Bacillus sp., Streptomyces sp., Escherichia sp. or Aspergillus sp.* In some embodiments, the variant proteases are produced by *Bacillus sp.* host cells. Examples *of Bacillus* sp. host cells that find use in the production of the variant proteases of the invention include, but are not limited to *B. licheniformis, B. lentus, B. subtilis, B. amyloliquefaciens, B. lentus, B. brevis, B. stearothermophilus, B. alkalophilus, B. coagulans, B. circulans, B. pumilis, B. thuringiensis, B. clausii*, and *B. megaterium*, as well as other organisms within the genus *Bacillus.* In some embodiments, *B. subtilis* host cells are used for production of variant proteases. U.S. Patents 5,264,366 and 4,760,025 (RE 34,606) describe various *Bacillus* host strains that can be used for producing variant proteases of the invention, although other suitable strains can be used.

Several industrial bacterial strains that can be used to produce variant proteases of the invention include non-recombinant (i.e., wild-type) *Bacillus sp.* strains, as well as variants of naturally-occurring strains and/or recombinant strains. In some embodiments, the host strain is a recombinant strain, wherein a polynucleotide encoding a polypeptide of interest has been introduced into the host. In some embodiments, the host strain is a *B. subtilis* host strain and particularly a recombinant *Bacillus subtilis* host strain. Numerous *B. subtilis* strains are known, including, but not limited to, e.g., 1A6 (ATCC 39085), 168 (1A01), SB19, W23, Ts85, B637, PB1753 through PB1758, PB3360, JH642, 1A243 (ATCC 39,087), ATCC 21332, ATCC 6051, MI113, DE100 (ATCC 39,094), GX4931, PBT 110, and PEP 211strain (see, e.g., Hoch et al., Genetics 73:215-228 (1973)) (see also U.S. Patent Nos. 4,450,235 and 4,302,544, and EP 0134048). The use of *B. subtilis* as an expression host cells is well known in the art (see, e.g., Palva et al., Gene 19:81-87 (1982); Fahnestock and Fischer, J. Bacteriol., 165:796-804 (1986); and Wang et al., Gene 69:39-47 (1988)).

In some embodiments, the *Bacillus* host cell is a *Bacillus* sp. that includes a mutation or deletion in at least one of the following genes, *degU, degS, degR* and *degQ.* Preferably the mutation is in a *degU* gene, and more preferably the mutation is *degU(Hy)32.* See e.g., Msadek et al., J. Bacteriol. 172:824-834 (1990) and Olmos et al., Mol. Gen. Genet. 253:562-567 (1997)). A preferred host strain is a *Bacillus subtilis* carrying a *degU32(Hy)* mutation. In some embodiments, the *Bacillus* host comprises a mutation or deletion in *scoC4* (see, e.g., Caldwell et al., J. Bacteriol. 183:7329-7340 (2001)); *spoIIE* (see, e.g., Arigoni et al., Mol. Microbiol. 31:1407-1415 (1999)); and/or *oppA* or other genes of the *opp* operon (see, e.g., Perego et al., Mol. Microbiol. 5:173-185 (1991)). Indeed, it is contemplated that any mutation in the *opp* operon that causes the same phenotype as a mutation in the *oppA* gene will find use in some embodiments of the altered *Bacillus* strain of the invention. In some embodiments, these mutations occur alone, while in other embodiments, combinations of mutations are present. In some embodiments, an altered *Bacillus* host cell strain that can be used to produce a variant protease of the invention is a *Bacillus* host strain that already includes a mutation in one or more of the above-mentioned genes. In addition, *Bacillus* sp. host cells that comprise mutation(s) and/or deletions of endogenous protease genes find use. In some embodiments, the *Bacillus* host cell comprises a deletion of the *aprE* and the *nprE* genes. In other embodiments, the *Bacillus* sp. host cell comprises a deletion of 5 protease genes, while in other embodiments, the *Bacillus sp.* host cell comprises a deletion of 9 protease genes (see, e.g., U.S. Pat. Application Pub. No. 2005/0202535).

Host cells are transformed with at least one nucleic acid encoding at least one variant protease of the invention using any suitable method known in the art. Whether the nucleic acid is incorporated into a vector or is used without the presence of plasmid DNA, it is typically introduced into a microorganism, in some embodiments, preferably an *E. coli* cell or a competent *Bacillus* cell. Methods for introducing a nucleic acid (e.g., DNA) into *Bacillus* cells or *E. coli* cells utilizing plasmid DNA constructs or vectors and transforming such plasmid DNA constructs or vectors into such cells are well known. In some embodiments, the plasmids are subsequently isolated from *E. coli* cells and transformed into *Bacillus* cells. However, it is not essential to use intervening microorganisms such as *E. coli,* and in some embodiments, a DNA construct or vector is directly introduced into a *Bacillus* host.

Those of skill in the art are well aware of suitable methods for introducing nucleic acid or polynucleotide sequences of the invention into *Bacillus* cells (see e.g., Ferrari et al., "Genetics," in Harwood et al. (ed.), Bacillus, Plenum Publishing Corp. (1989), pp. 57-72; Saunders et al., J. Bacteriol. 157:718-726 (1984); Hoch et al., J. Bacteriol. 93:1925-1937 (1967); Mann et al., Current Microbiol. 13:131-135 (1986); and Holubova, Folia Microbiol. 30:97 (1985); Chang et al., Mol. Gen. Genet. 168:11-115 (1979); Vorobjeva et al., FEMS Microbiol. Lett. 7:261-263 (1980); Smith et al., Appl. Env. Microbiol. 51:634 (1986); Fisher et al., Arch. Microbiol. 139:213-217 (1981); and McDonald, J. Gen. Microbiol. 130:203 (1984)). Indeed, such methods as transformation, including protoplast transformation and congression, transduction, and protoplast fusion are well known and suited for use in the present invention. Methods of transformation are used to introduce a DNA construct or vector comprising a nucleic acid encoding a variant protease of the present invention into a host cell. Methods known in the art to transform *Bacillus* cells include such methods as plasmid marker rescue transformation, which involves the uptake of a donor plasmid by competent cells carrying a partially homologous resident plasmid (Contente et al., Plasmid 2:555-571 (1979); Haima et al., Mol. Gen. Genet. 223:185-191 (1990); Weinrauch et al., J. Bacteriol. 154:1077-1087 (1983); and Weinrauch et al., J. Bacteriol. 169:1205-1211 (1987)). In this method, the incoming donor plasmid recombines with the homologous region of the resident "helper" plasmid in a process that mimics chromosomal transformation.

In addition to commonly used methods, in some embodiments, host cells are directly transformed with a DNA construct or vector comprising a nucleic acid encoding a variant protease of the invention (i.e., an intermediate cell is not used to amplify, or otherwise process, the DNA construct or vector prior to introduction into the host cell). Introduction of the DNA construct or vector of the invention into the host cell includes those physical and chemical methods known in the art to introduce a nucleic acid sequence (e.g., DNA sequence) into a host cell without insertion into a plasmid or vector. Such methods include, but are not limited to calcium chloride precipitation, electroporation, naked DNA, liposomes and the like. In additional embodiments, DNA constructs or vector are co-transformed with a plasmid, without being inserted into the plasmid. In further embodiments, a selective marker is deleted from the altered *Bacillus* strain by methods known in the art (see Stahl et al., J. Bacteriol. 158:411-418 (1984); and Palmeros et al., Gene 247:255 -264 (2000)).

In some embodiments, the transformed cells of the present invention are cultured in conventional nutrient media. The suitable specific culture conditions, such as temperature, pH and the like are known to those skilled in the art. In addition, some culture conditions may be found in the scientific literature such as Hopwood (2000) Practical Streptomyces Genetics, John Innes Foundation, Norwich UK; Hardwood et al., (1990) Molecular Biological Methods for Bacillus, John Wiley and from the American Type Culture Collection (ATCC). In one aspect, the invention provides a culture (e.g., cell culture) comprising at least one variant protease or at least one nucleic acid of the invention. Also provided is a composition comprising at least one nucleic acid, vector, or DNA construct of the invention.

In some embodiments, host cells transformed with at least one polynucleotide sequence encoding at least one variant protease of the invention are cultured in a suitable nutrient medium under conditions permitting the expression of the present protease, after which the resulting protease is recovered from the culture. The medium used to culture the cells comprises any conventional medium suitable for growing the host cells, such as minimal or complex media containing appropriate supplements. Suitable media are available from commercial suppliers or may be prepared according to published recipes (e.g., in catalogues of the American Type Culture Collection). In some embodiments, the protease produced by the cells is recovered from the culture medium by conventional procedures, including, but not limited to, e.g., separating the host cells from the medium by centrifugation or filtration, precipitating the proteinaceous components of the supernatant or filtrate by means of a salt (e.g., ammonium sulfate), chromatographic purification (e.g., ion exchange, gel filtration, affinity, etc.). Any method suitable for recovering or purifying a variant protease of the invention can be used.

In one aspect, a variant protease produced by a recombinant host cell is secreted into the culture medium. A nucleic acid sequence that encodes a purification facilitating domain may be used to facilitate purification of soluble proteins. A vector or DNA construct comprising a polynucleotide sequence encoding a variant protease may further comprise a nucleic acid sequence encoding a purification facilitating domain to facilitate purification of the variant protease (see, e.g., Kroll, D.J. et al., DNA Cell Biol. 12:441-53 (1993)). Such purification facilitating domains include, but are not limited to, e.g., metal chelating peptides such as histidine-tryptophan modules that allow purification on immobilized metals (Porath J., Protein Expr. Purif. 3:263-281 (1992)), protein A domains that allow purification on immobilized immunoglobulin, and the domain utilized in the FLAGS extension/affinity purification system (Immunex Corp., Seattle, WA). The inclusion of a cleavable linker sequence such as Factor XA or enterokinase (Invitrogen, San Diego, CA) between the purification domain and the heterologous protein also find use to facilitate purification.

Assays for detecting and measuring the enzymatic activity of an enzyme, such as a variant protease of the invention, are well known. Various assays for detecting and measuring activity of proteases, such as, e.g., variant proteases of the invention, are also known to those of ordinary skill in the art. In particular, assays are available for measuring protease activity that are based on the release of acid-soluble peptides from casein or hemoglobin, measured as absorbance at 280 nm or colorimetrically using the Folin method (see e.g., Bergmeyer et al., "Methods of Enzymatic Analysis" vol. 5, Peptidases, Proteinases and their Inhibitors, Verlag Chemie, Weinheim (1984)). Other exemplary assays involve the solubilization of chromogenic substrates (see e.g., Ward, "Proteinases," in Fogarty (ed.)., Microbial Enzymes and Biotechnology, Applied Science, London, (1983), pp. 251-317). Other exemplary assays include, but are not limited to succinyl-Ala-Ala-Pro-Phe-para nitroanilide assay (SAAPFpNA) and the 2,4,6-trinitrobenzene sulfonate sodium salt assay (TNBS assay). Numerous additional references known to those in the art provide suitable methods (see e.g., Wells et al., Nucleic Acids Res. 11:7911-7925 (1983); Christianson et al., Anal. Biochem. 223:119 -129 (1994); and Hsia et al., Anal Biochem. 242:221-227 (1999)).

A variety of methods can be used to determine the level of production of a mature protease (e.g., mature variant protease of the invention) in a host cell. Such methods include, but are not limited to, e.g., methods that utilize either polyclonal or monoclonal antibodies specific for the protease. Exemplary methods include, but are not limited, e.g., to enzyme-linked immunosorbent assays (ELISA), radioimmunoassays (RIA), fluorescent immunoassays (FIA), and fluorescent activated cell sorting (FACS). These and other assays are well known in the art (see e.g., Maddox et al., J. Exp.

Med. 158:1211 (1983)).

In another aspect, the invention provides methods for making or producing a mature variant protease of the invention, as defined in the claims. A mature variant protease does not include a signal peptide or a propeptide sequence. Some such methods comprising making or producing a variant protease of the invention in a recombinant bacterial host cell, such as, e.g., a *Bacillus sp.* cell, including, e.g., *Bacillus subtilis* cell. In one aspect, the invention provides a method of producing a variant protease of the invention, the method comprising cultivating a recombinant host cell comprising a recombinant expression vector comprising a nucleic acid encoding a variant protease of the invention under conditions conducive to the production of the variant protease. Some such methods further comprise recovering the variant protease from the culture.

In one aspect, the invention provides a method of producing a variant protease of the invention, the method comprising: (a) introducing a recombinant expression vector comprising a nucleic acid encoding a variant protease of the invention into a population of cells (e.g., bacterial cells, such as *Bacillus subtilis* cells); and (b) culturing the cells in a culture medium under conditions conducive to produce the variant protease encoded by the expression vector. Some such methods further comprise: (c) isolating the variant protease from the cells or from the culture medium.

**Cleaning Compositions of the Invention**

In another aspect, the invention provides a composition (e.g., cleaning composition) comprising one or more polypeptides (e.g., variant proteases) of the invention, as defined in the claims. Such compositions may comprise at least one excipient or carrier and/or other substituent, component, or material. As further discussed herein, the polypeptides of the invention, including the variant proteases of the invention, are useful in a variety of cleaning applications, including laundry cleaning applications, automatic dishwashing applications, hand dishwashing applications, hard surface cleaning applications, and other applications described herein. Thus, for example, in one aspect, the invention provides cleaning compositions comprising at least one polypeptide (e.g., variant protease) of the invention, as defined in the claims. As noted above, such cleaning compositions include, but are not limited to, e.g., automatic and hand dishwashing detergent compositions, laundry detergent compositions (including, e.g., liquid and powder laundry detergent compositions), hard surface cleaning compositions (including, but not limited to, e.g., hard surface of a non-dishware item, non-tableware item, table, table top, furniture item, wall, floor, ceiling, etc.). Such cleaning compositions, which are useful in methods of cleaning an item or a surface in need of cleaning, may comprise, e.g., but not limited to, at least one excipient, carrier, and/or other substituent, component, or material. In one aspect, the invention provides any composition of the invention (e.g., cleaning composition or detergent composition) described herein and throughout, said composition comprising any polypeptide of the invention (e.g., any protease variant or subtilisin variant of the invention) described throughout and herein, but wherein said composition is not an automatic dishwashing composition(e.g., automatic dishwashing detergent composition).

Unless otherwise noted, all component or composition levels provided herein are made in reference to the active level of that component or composition, and are exclusive of impurities, for example, residual solvents or byproducts, which may be present in commercially available sources. Enzyme components weights are based on total active protein. All percentages and ratios are calculated by weight unless otherwise indicated. All percentages and ratios are calculated based on the total composition unless otherwise indicated. In the exemplified detergent compositions, the enzymes levels are expressed by pure enzyme by weight of the total composition and, unless otherwise specified, the detergent ingredients are expressed by weight of the total compositions.

As indicated herein, in some embodiments, the cleaning compositions of the present invention may further comprise one or more adjunct materials including, but not limited to, e.g., one or more surfactants, builders, bleaches, bleach activators, bleach catalysts, other enzymes, enzyme stabilizing systems, chelants, optical brighteners, soil release polymers, dye transfer agents, dispersants, suds suppressors, dyes, perfumes, colorants, filler salts, hydrotropes, photoactivators, fluorescers, fabric conditioners, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, rinse aid (e.g., a rinse aid containing at least one surfactant to prevent water droplet formation by making water drain from the surface of the item being cleaned in a thin sheet, rather than forming droplets), and/or pH control agents (see, e.g., U.S. Pat. Nos. 6,610,642, 6,605,458, 5,705,464, 5,710,115, 5,698,504, 5,695,679, 5,686,014 and 5,646,101). Embodiments of specific cleaning composition materials are exemplified in detail below. If a cleaning adjunct material(s) is not compatible with a variant protease of the present invention in a desired cleaning composition, then a suitable method of keeping the cleaning adjunct material(s) and the variant protease(s) separated (i.e., not in contact with one another) until combination of the two components is appropriate is used. Such separation methods include any suitable method known in the art (e.g., gelcaps, encapsulation, tablets, physical separation, etc.).

The cleaning compositions of the present invention are advantageously employed, for example, in laundry applications, hard surface cleaning applications, hand or manual dishwashing applications, automatic dishwashing applications, eyeglass cleaning applications, as well as cosmetic applications, such as for cleaning dentures, teeth, hair, and skin. Due to the unique advantages of increased effectiveness in lower temperature solutions, the variant protease enzymes of the present invention are suited for laundry applications and dishwashing applications, including hand and automatic dishwashing applications. Furthermore, the variant protease enzymes of the present invention find use in solid, gel, granular, and/or liquid compositions, including solid, gel, granular, and/or liquid detergent compositions and/or formulations.

The variant proteases of the present invention also find use cleaning additive product compositions. In some embodiments, a variant protease of the invention is useful in low temperature solution cleaning applications and methods. In one aspect, the invention provides cleaning additive product compositions which include at least one variant protease enzyme of the present invention and which are ideally suited for inclusion in a wash process when additional bleaching effectiveness is desired. Such instances include, but are not limited to, e.g., low temperature solution cleaning applications. In some embodiments, the additive product composition is in its simplest form - i.e., one or more variant proteases of the invention. In some embodiments, the additive product composition is packaged in dosage form for addition to a cleaning process. In some embodiments, the additive product composition is packaged in dosage form for addition to a cleaning process where a source of peroxygen is employed and increased bleaching effectiveness is desired. Any suitable single dosage unit form may be used, including but not limited to, e.g., pills, tablets, gelcaps, or other single dosage units, such as premeasured powders or liquids. Thus, in one aspect, the invention provides a cleaning product composition comprising at least one variant protease of the invention, wherein the product is formulated in suitable form (e.g., as a liquid, powder solid, pill, tablet, gelcap or other suitable form) in a suitable single dosage unit such that a single dose of the variant protease is provided. Such cleaning products are useful in a variety of cleaning methods and applications, including but not limited to, e.g., machine or hand laundry methods and applications, automatic dishwashing or hand dishwashing methods and applications, etc. Such cleaning methods and applications may be conducted at low temperature or low pH conditions. In some embodiments, at least one filler and/or at least one carrier material is included to increase the volume of such compositions. Suitable filler or carrier materials include, but are not limited to, e.g., various salts of sulfate, carbonate and silicate as well as talc, clay and the like. Suitable filler or carrier materials for liquid compositions include, but are not limited to, e.g., water or low molecular weight primary and secondary alcohols including polyols and diols. Examples of such alcohols include, but are not limited to, e.g., methanol, ethanol, propanol and isopropanol. In some embodiments, the compositions contain from about 5% to about 90% of such filler or carrier materials. Acidic fillers may be included in such compositions to reduce the pH of the resulting solution in the cleaning method or application. Alternatively, in some embodiments, the cleaning additive includes one or more adjunct ingredients, as more fully described below.

The present cleaning compositions and cleaning additives require an effective amount of at least one variant protease of the invention, alone or in combination with other proteases and/or additional enzymes. The required level of enzyme is achieved by the addition of one or more variant proteases of the invention. Typically, a cleaning composition comprises at least about 0.0001 weight percent to about 20 weight percent, from about 0.0001 to about 10 weight percent, from about 0.0001 to about 1 weight percent, from about 0.001 to about 1 weight percent, or from about 0.01 to about 0.1 weight percent of at least one variant protease of the invention. In one aspect, a composition of the invention (e.g., cleaning composition of the invention) comprises from about 0.01 milligram (mg) to about 10 mg, about 0.01 to about 5 mg, about 0.01 mg to about 2 mg, about 0.01 to about 1 mg, about 0.5 mg to about 10 mg, about 0.5 to about 5 mg, about 0.5 to about 4 mg, about 0.5 to about 4 mg, about 0.5 to about 3 mg, about 0.5 to about 2 mg, about 0.5 to about 1 mg, about 0.1 to about 10 mg, about 0.1 to about 5 mg, about 0.1 to about 4 mg, about 0.1 to about 3 mg, about 0.1 to about 2 mg, about 0.1 to about 2 mg, about 0.1 to about 1 mg, about 0.1 to about 0.5 mg of at least one active variant protease of the invention per gram of the composition.

The cleaning compositions of the invention are typically formulated such that, during use in aqueous cleaning operations, the wash water will have a pH of from about 5.0 to about 11.5 or even from about 7.5 to about 10.5. Liquid product compositions or formulations are typically formulated to have a neat pH from about 3.0 to about 9.0 or from about 3.0 to about 5.0. Granular laundry product compositions are typically formulated to have a pH from about 9 to about 11. Hand dishwashing and automatic dishwashing detergent compositions are typically formulated to have a pH from about 8 to about 11.5, including, but not limited to, e.g., pH ranges of about 8 to about 10, from about 9 to about 11.5, and from about 9.5 to about 11.5 depending on the method and specific application. Techniques for controlling pH at recommended usage levels include the use of buffers, alkalis, acids, etc., and are well known to those skilled in the art.

Suitable low pH cleaning compositions typically have a neat pH of from about 3 to about 5, and are typically free of surfactants that hydrolyze in such a pH environment. Such surfactants include sodium alkyl sulfate surfactants that comprise at least one ethylene oxide moiety or even from about 1 to about 16 moles of ethylene oxide. Such cleaning compositions typically comprise a sufficient amount of a pH modifier, such as sodium hydroxide, monoethanolamine, or hydrochloric acid, to provide such cleaning composition with a neat pH of from about 3 to about 5. Such compositions typically comprise at least one acid stable enzyme. In some embodiments, the compositions are liquids, while in other embodiments, they are solids. The pH of such liquid compositions is typically measured as a neat pH. The pH of such solid compositions is measured as a 10% solids solution of said composition wherein the solvent is distilled water. In these embodiments, all pH measurements are taken at 20°C, unless otherwise indicated.

In some embodiments, when the variant protease(s) of the invention is/are employed in a granular composition or liquid, it is desirable for the variant protease to be in the form of an encapsulated particle to protect the variant protease from other components of the granular composition during storage. In addition, encapsulation is also a means of controlling the availability of the variant protease during the cleaning process. In some embodiments, encapsulation enhances the performance of the variant protease(s) and/or additional enzymes. In this regard, the variant proteases of the present invention are encapsulated with any suitable encapsulating material known in the art. In some embodiments, the encapsulating material typically encapsulates at least part of the catalyst for the variant protease(s) of the invention. Typically, the encapsulating material is water-soluble and/or water-dispersible. In some embodiments, the encapsulating material has a glass transition temperature (Tg) of 0°C or higher. Glass transition temperature is described in more detail in WO 97/11151. The encapsulating material is typically selected from consisting of carbohydrates, natural or synthetic gums, chitin, chitosan, cellulose and cellulose derivatives, silicates, phosphates, borates, polyvinyl alcohol, polyethylene glycol, paraffin waxes, and combinations thereof. When the encapsulating material is a carbohydrate, it is typically selected from monosaccharides, oligosaccharides, polysaccharides, and combinations thereof. In some typical embodiments, the encapsulating material is a starch (see, e.g., EP 0 922 499; U.S. Patent Nos. 4,977,252, 5,354,559, and 5,935,826). In some embodiments, the encapsulating material is a microsphere made from plastic such as thermoplastics, acrylonitrile, methacrylonitrile, polyacrylonitrile, polymethacrylonitrile and mixtures thereof; commercially available microspheres that find use include, but are not limited to those supplied by EXPANCEL® (Stockviksverken, Sweden), and PM 6545, PM 6550, PM 7220, PM 7228, EXTENDOSPHERES®, LUXSIL®, Q-CEL®, and SPHERICEL® (PQ Corp., Valley Forge, PA).

As described herein, the variant proteases of the invention find particular use in the cleaning methods and applications, including, but not limited to, e.g., cleaning, laundry, hand dishwashing, and automatic dishwashing detergent compositions. These applications place enzymes under various environmental stresses. The variant proteases of the invention provide advantages over many currently used enzymes in such cleaning applications due to their proteolytic activity and stability under various conditions.

Indeed, there are a variety of wash conditions including varying detergent formulations, wash water volumes, wash water temperatures, and lengths of wash time, to which proteases involved in washing are exposed. In addition, detergent formulations used in different geographical areas have different concentrations of their relevant components present in the wash water. For example, European detergents typically have about 4500-5000 parts per million (ppm) of detergent components in the wash water, while Japanese detergents typically have approximately 667 ppm of detergent components in the wash water. In North America, particularly the United States, detergents typically have about 975 ppm of detergent components present in the wash water.

A low detergent concentration system includes detergents where less than about 800 ppm of the detergent components are present in the wash water. Japanese detergents are typically considered low detergent concentration system as they have approximately 667 ppm of detergent components present in the wash water.

A medium detergent concentration includes detergents where between about 800 ppm and about 2000ppm of the detergent components are present in the wash water. North American detergents are generally considered to be medium detergent concentration systems as they have approximately 975 ppm of detergent components present in the wash water. Brazil typically has approximately 1500 ppm of detergent components present in the wash water.

A high detergent concentration system includes detergents where greater than about 2000 ppm of the detergent components are present in the wash water. European detergents are generally considered to be high detergent concentration systems as they have approximately 4500-5000 ppm of detergent components in the wash water.

Latin American detergents are generally high suds phosphate builder detergents and the range of detergents used in Latin America can fall in both the medium and high detergent concentrations as they range from 1500 ppm to 6000 ppm of detergent components in the wash water. As mentioned above, Brazil typically has approximately 1500 ppm of detergent components present in the wash water. However, other high suds phosphate builder detergent geographies, not limited to other Latin American countries, may have high detergent concentration systems up to about 6000 ppm of detergent components present in the wash water.

In light of the foregoing, it is evident that concentrations of detergent compositions in typical wash solutions throughout the world varies from less than about 800 ppm of detergent composition ("low detergent concentration geographies"), for example, about 667 ppm in Japan, to between about 800 ppm to about 2000 ppm ("medium detergent concentration geographies"), for example, about 975 ppm in U.S. and about 1500 ppm in Brazil, to greater than about 2000 ppm ("high detergent concentration geographies"), for example, about 4500 ppm to about 5000 ppm in Europe and about 6000 ppm in high suds phosphate builder geographies.

The concentrations of the typical wash solutions are determined empirically. For example, in the U.S., a typical washing machine holds a volume of about 64.4 L of wash solution. Accordingly, in order to obtain a concentration of about 975 ppm of detergent within the wash solution about 62.79 g of detergent composition must be added to the 64.4 L of wash solution. This amount is the typical amount measured into the wash water by the consumer using the measuring cup provided with the detergent.

As a further example, different geographies use different wash temperatures. The temperature of the wash water in Japan is typically less than that used in Europe. For example, the temperature of the wash water in North America and Japan is typically between about 10 and about 30°C (e.g., about 20°C), whereas the temperature of wash water in Europe is typically between about 30 and about 60°C (e.g., about 40°C). However, in the interest of saving energy, many consumers are switching to using cold water washing. In addition, in some further regions, cold water is typically used for laundry, as well as in dishwashing applications. In some embodiments, the "cold water washing" of the present invention utilizes washing at temperatures from about 10°C to about 40°C, or from about 20°C to about 30°C, or from about 15°C to about 25°C, as well as all other combinations within the range of about 15°C to about 35°C, and all ranges within 10°C to 40°C.

As a further example, different geographies typically have different water hardness. Water hardness is usually described in terms of the grains per gallon mixed $Ca^{2+}/Mg^{2+}$. Hardness is a measure of the amount of calcium ($Ca^{2+}$) and magnesium ($Mg^{2+}$) in the water. Most water in the United States is hard, but the degree of hardness varies. Moderately hard (60-120 ppm) to hard (121-181 ppm) water has 60 to 181 parts per million (parts per million converted to grains per U.S. gallon is ppm # divided by 17.1 equals grains per gallon) of hardness minerals.

| Water | Grains per gallon | Parts per million |
|---|---|---|
| Soft | less than 1.0 | less than 17 |
| Slightly hard | 1.0 to 3.5 | 17 to 60 |
| Moderately hard | 3.5 to 7.0 | 60 to 120 |
| Hard | 7.0 to 10.5 | 120 to 180 |
| Very hard | greater than 10.5 | greater than 180 |

European water hardness is typically greater than about 10.5 (for example about 10.5 to about 20.0) grains per gallon mixed $Ca^{2+}/Mg^{2+}$ (e.g., about 15 grains per gallon mixed $Ca^{2+}/Mg^{2+}$). North American water hardness is typically greater than Japanese water hardness, but less than European water hardness. For example, North American water hardness can be between about 3 to about 10 grains, about 3 to about 8 grains or about 6 grains. Japanese water hardness is typically lower than North American water hardness, usually less than about 4, for example about 3 grains per gallon mixed $Ca^{2+}/Mg^{2+}$.

Accordingly, in some aspects, the invention provides variant proteases that show surprising wash performance in at least one set of wash conditions (e.g., water temperature, water hardness, and/or detergent concentration). In some aspects, the variant proteases of the invention are comparable in wash performance to other subtilisin proteases. In some aspects, the variant proteases of the present invention exhibit enhanced wash performance as compared to subtilisin proteases currently commercially available. Thus, in some aspects of the invention, the variant proteases provided herein exhibit enhanced oxidative stability, enhanced thermal stability, enhanced cleaning capabilities under various conditions, and/or enhanced chelator stability. In addition, the variant proteases of the invention find use in cleaning compositions that do not include detergents, again either alone or in combination with builders and stabilizers.

In one aspect, the invention provides a cleaning composition comprising at least one variant protease of the present invention that is present at a level from about 0.00001 % to about 10% by weight of the composition with the balance (e.g., about 99.999% to about 90.0%) comprising one or more cleaning adjunct materials by weight of composition. In another aspect, the invention provides a cleaning composition comprising at least one variant protease of the invention that is present at a level of about 0.0001 % to about 10%, about 0.001% to about 5%, about 0.001% to about 2%, or about 0.005% to about 0.5% by weight of the composition with the balance of the cleaning composition (e.g., about 99.9999% to about 90.0%, about 99.999 % to about 98%, about 99.995% to about 99.5% by weight) comprising one or more cleaning adjunct materials.

In some aspects, a cleaning composition of the invention comprises, in addition to at least one variant protease of the invention, one or more additional enzymes, which provide cleaning performance and/or fabric care and/or hand or manual dishwashing and/or automatic dishwashing benefits. Examples of suitable enzymes include, but are not limited to, e.g., hemicellulases, cellulases, peroxidases, proteases, xylanases, lipases, phospholipases, esterases, cutinases, pectinases, pectate lyases, mannanases, keratinases, reductases, oxidases, phenoloxidases, lipoxygenases, ligninases, pullulanases, tannases, pentosanases, malanases, β-glucanases, arabinosidases, hyaluronidase, chondroitinase, laccase, and/or amylases, neutral metalloprotease enzymes (abbreviated as "nprE"), or mixtures thereof. In some aspects, the cleaning composition comprises, in addition to at least one variant protease of the invention, a combination of additional enzymes (i.e., a "cocktail") comprising conventional applicable enzymes such as, e.g., at least one additional protease, lipase, cutinase, cellulose, and/or amylase.

In addition to the variant proteases provided herein, any other suitable protease may find use and be included in a composition of the invention. In one aspect, the invention provides a composition (e.g., cleaning composition) comprising at least one variant protease of the invention and at least one additional protease. Suitable proteases include those of animal, vegetable, or microbial origin. In one embodiment, a microbial protease may be included. A chemically or genetically modified mutant of a protease may be included. In one aspect, the at least one additional protease is a serine protease, preferably an alkaline microbial protease or a trypsin-like protease. Examples of alkaline proteases include subtilisins, especially those derived from *Bacillus* (e.g., subtilisin, *B. lentus* subtilisin (i.e., GG36), *B. amyloliquefaciens* subtilisin (i.e., BPN'), subtilisin Carlsberg, subtilisin 309, subtilisin 147, PB92, and subtilisin 168). Additional examples include those mutant proteases (i.e., variant proteases) described in U.S. Pat. Nos. RE 34,606, 5,955,340, 5,700,676, 6,312,936, and 6,482,628. Additional proteases include, but are not limited to trypsin (e.g., of porcine or bovine origin), and the *Fusarium* protease described in WO 89/06270. A composition of the invention comprising at least one variant protease of the invention may also comprise at least one commercially available protease enzyme. Commercially available protease enzymes that find use in compositions of the present invention include, but are not limited to, e.g., MAXATASE®, MAXACAL™, MAXAPEM™, OPTICLEAN®, OPTIMASE®, PROPERASE®, PURAFECT®, PURAFECT® OXP, PURAMAX™, EXCELLASE™, and PURAFAST™ (Genencor); ALCALASE®, SAVINASE®, PRIMASE®, DURAZYM™, POLARZYME®, OVOZYME®, KANNASE®, LIQUANASE®, NEUTRASE®, RELASE® and ESPERASE® (Novozymes); KAP *Bacillus* alkalophilus subtilisin with A230V+S256G+S259N (Kao); and BLAP™ *B. lentus* protease, BLAP X, and BLAP S (Henkel Kommanditgesellschaft auf Aktien, Duesseldorf, Germany). Additional proteases that may be included in compositions of the invention include those described in WO95/23221, WO 92/21760, U.S. Pat. Pub. No. 2008/0090747, and U.S. Pat. Nos. 5,801,039, 5,340,735, 5,500,364, 5,855,625, RE 34,606, 5,955,340, 5,700,676, 6,312,936, and 6,482,628, and various other patents. Metalloproteases may be included in compositions of the invention. Such metalloproteases, include, but are not limited to, e.g., the neutral metalloprotease enzyme (nprE) described in WO 07/044993.

In one aspect, the invention provides a composition (e.g., cleaning composition) comprising at least one variant protease of the invention and at least one lipase. Suitable lipases include, but are not limited to, e.g., those of bacterial or fungal origin. A chemically or genetically modified mutant of a lipase may be included in the composition. Examples of useful lipases include *Humicola lanuginosa* lipase (see, e.g., EP 258 068, and EP 305 216), *Rhizomucor miehei* lipase (see, e.g., EP 238 023), *Candida* lipase, such as *C. antartica* lipase (e.g., *C. antartica* lipase A or B; see, e.g., EP 214 761), *Pseudomonas* lipases such as *P. alcaligenes* lipase and *P. pseudoalcaligenes* lipase (see, e.g., EP 218 272), *P. cepacia* lipase (see, e.g., EP 331 376), *P. stutzeri* lipase (see, e.g., GB 1,372,034), *P. fluorescens* lipase, *Bacillus* lipase (e.g., *B. subtilis* lipase (Dartois et al., Biochem. Biophys. Acta 1131:253-260 (1993)); *B. stearothermophilus* lipase (see, e.g., JP 64/744992); and *B. pumilus* lipase (see, e.g., WO 91/16422)).

Furthermore, a number of cloned lipases find use in compositions (e.g., cleaning compositions) of the present invention, including, but not limited to, e.g., *Penicillium camembertii* lipase (Yamaguchi et al., Gene 103:61-67 (1991)), *Geotricum candidum* lipase (Schimada et al., J. Biochem. 106:383-388 (1989)), and various *Rhizopus* lipases such as *R. delemar* lipase (Hass et al., Gene 109:117-113 (1991)), a *R. niveus* lipase (Kugimiya et al., Biosci. Biotech. Biochem. 56:716-719 (1992)) *and R. oryzae* lipase.

Other types of lipolytic enzymes, such as cutinases, also find use in some embodiments of the present invention, including, but not limited to, e.g., the cutinase derived from *Pseudomonas mendocina* (see WO 88/09367), and the cutinase derived from *Fusarium solani pisi* (see WO 90/09446).

Additional suitable lipases include commercially available lipases such as M1 LIPASE™, LUMA FAST™, and LIPOMAX™ (Genencor); LIPOLASE® and LIPOLASE® ULTRA (Novozymes); and LIPASE P™ "Amano" (Amano Pharmaceutical Co. Ltd., Japan).

In some embodiments, the invention provides compositions (e.g., cleaning compositions) comprising at least one variant protease of the invention and at least one lipase that is present at a level from about 0.00001 % to about 10% of additional lipase by weight of the composition and the balance of one or more cleaning adjunct materials by weight of composition. In one aspect, a cleaning composition of the present invention comprises, in addition to at least one variant protease of the invention, at least one lipase at a level of about 0.0001 % to about 10%, about 0.001% to about 5%, about 0.001% to about 2%, or about 0.005% to about 0.5% lipase by weight of the composition.

Also included is a composition (e.g., cleaning composition) comprising at least one variant of the invention and at least one amylase. Any amylase (e.g., alpha and/or beta) suitable for use in alkaline solutions may be useful to include in such a composition. Suitable amylases include, but are not limited to, e.g., those of bacterial or fungal origin. A chemically or genetically modified mutant of an amylase may be included. Amylases that find use in compositions of the invention, include, but are not limited to, e.g., $\alpha$-amylases obtained from *B. licheniformis* (see, e.g., GB 1,296,839). Commercially available amylases that find use in compositions of the invention include, but are not limited to, e.g., DURAMYL®, TERMAMYL®, FUNGAMYL®, STAINZYME®, STAINZYME PLUS®, STAINZYME ULTRA®, and BAN™ (Novozymes), as well as POWERASE™, RAPIDASE® and MAXAMYL® P (Genencor).

In one aspect, the invention provides a cleaning composition comprising at least one variant protease or at least one amylase, wherein the amylase is present at a level from about 0.00001 % to about 10% of additional amylase by weight of the composition and the balance of one or more cleaning adjunct materials by weight of composition. In another aspect, the invention includes a cleaning composition comprising at least one variant protease and at least one amylase that is present at a level of about 0.0001 % to about 10%, about 0.001% to about 5%, about 0.001% to about 2%, about 0.005% to about 0.5% amylase by weight of the composition.

Any suitable cellulase may find used in a cleaning composition of the present invention. In one aspect, the invention provides a cleaning composition comprising at least one variant protease of the invention and one or at least one cellulase. Suitable cellulases include, but are not limited to, e.g., those of bacterial or fungal origin. A chemically or genetically modified mutant of a cellulase may be included in a composition of the invention. Suitable cellulases include, but are not limited to, e.g., *Humicola insolens* cellulases (see, e.g., U.S. Pat. No. 4,435,307) and cellulases having color care benefits (see, e.g., EP 0 495 257). Additional suitable cellulases are known in the art. Commercially available cellulases that find use and may be included in a composition of the invention include, but are not limited to, e.g., CELLUZYME®, CAREZYME® (Novozymes), and KAC-500(B)™ (Kao Corporation). In some embodiments, cellulases are incorporated as portions or fragments of mature wild-type or variant cellulases, wherein a portion of the N-terminus is deleted (see, e.g., U.S. Pat. No. 5,874,276). In one aspect, a cleaning composition of the invention comprises at least one variant protease of the invention and at least one cellulase at a level from about 0.00001 % to about 10% of additional cellulase by weight of the composition and the balance of one or more cleaning adjunct materials by weight of composition. In another aspect, the invention provides a cleaning composition comprising at least one variant protease of the invention and at least one cellulase at a level of about 0.0001 % to about 10%, about 0.001% to about 5%, about 0.001% to about 2%, about 0.005% to about 0.5% cellulase by weight of the composition.

Any mannanase suitable for use in detergent compositions also finds use in and thus may be included in a cleaning composition of the invention. In one aspect, the invention provides a cleaning composition comprising at least one variant protease of the invention and least one mannanase. Suitable mannanases include, but are not limited to, e.g., those of bacterial or fungal origin. A chemically or genetically modified mutant of a mannanase may be included in a composition of the invention. Various mannanases are known which are useful and may be included in a composition of invention (see, e.g., mannanases described in U.S. Pat. Nos. 6,566,114, 6,602,842, and 6,440,991). In one aspect, the invention provides a cleaning composition comprising at least one variant protease of the invention and at least one mannanase at a level from about 0.00001 % to about 10% of additional mannanase by weight of the composition and the balance of one or more cleaning adjunct materials by weight of composition. In some such cleaning compositions, each mannanase is present at a level of about 0.0001 % to about 10%, about 0.001% to about 5%, about 0.001% to about 2%, or about 0.005% to about 0.5% mannanase by weight of the composition.

A peroxidase may be used in combination with hydrogen peroxide or a source thereof (e.g., a percarbonate, perborate or persulfate) in a composition of the invention. An oxidase may be used in combination with oxygen in a composition of the invention. Both such types of enzymes are used for "solution bleaching" (i.e., to prevent transfer of a textile dye from a dyed fabric to another fabric when the fabrics are washed together in a wash liquor), preferably together with an enhancing agent (see, e.g., WO 94/12621 and WO 95/01426). Suitable peroxidases/oxidases that may be included in compositions of the invention include, but are not limited to, e.g., those of plant, bacterial, or fungal origin. A chemically or genetically modified mutant of a peroxidase or oxidase may be included in a composition of the invention. In one aspect, the invention provides a cleaning composition comprising at least one variant protease of the invention and at least one peroxidase and/or at least one oxidase enzyme. Each such peroxidase or oxidase may be present in the composition at a level from about 0.00001 % to about 10% of peroxidase or oxidase by weight of the composition and the balance of one or more cleaning adjunct materials by weight of composition. In another aspect, the invention provides a cleaning composition comprising at least one variant protease of the invention and at least one peroxidase and/or at least oxidase enzyme, wherein each such peroxidase or oxidase is present at a level of about 0.0001 % to about 10%, about 0.001% to about 5%, about 0.001% to about 2%, or about 0.005% to about 0.5% peroxidase or oxidase enzyme, respectively, by weight of the composition.

In one aspect, the invention provides a composition (e.g., cleaning composition) comprising at least one variant protease of the invention and one or more additional enzymes find use, including but not limited to, e.g., one or more perhydrolases (see, e.g., WO 05/056782).

In another aspect, the invention provides a composition (e.g., cleaning composition) comprising at least one variant protease of the invention and one or more mixtures of the above-mentioned enzymes are encompassed, such as, e.g., one or more additional proteases, amylases, lipases, mannanases, and/or cellulases. Indeed, it is contemplated that various mixtures of these enzymes will find use in compositions of the present invention. It is also contemplated that the varying levels of the variant protease(s) and one or more additional enzymes may both independently range to about 10%, the balance of the cleaning composition being one or more cleaning adjunct materials. The specific selection of a cleaning adjunct material is readily made by considering the surface or item (e.g., dishware item, tableware item, or fabric item) or fabric to be cleaned, and the desired form of the composition for the cleaning conditions during use (e.g., through the hand or automatic dishwashing detergent use).

In one aspect, the invention provides a composition (e.g., cleaning composition) comprising at least one variant protease of the invention (and optionally at least one additional enzyme, if desired) and one or more cleaning adjunct materials. Examples of suitable cleaning adjunct materials include, but are not limited to, e.g., surfactants, builders, bleaches, bleach activators, bleach catalysts, other enzymes, enzyme stabilizing systems, chelants, optical brighteners, soil release polymers, dye transfer agents, dye transfer inhibiting agents, catalytic materials, hydrogen peroxide, sources of hydrogen peroxide, preformed peracids, polymeric dispersing agents, clay soil removal agents, structure elasticizing agents, dispersants, suds suppressors, dyes, perfumes, colorants, filler salts, hydrotropes, photoactivators, fluorescers, fabric conditioners, fabric softeners, carriers, hydrotropes, processing aids, solvents, pigments, hydrolyzable surfactants, preservatives, anti-oxidants, anti-shrinkage agents, anti-wrinkle agents, germicides, fungicides, color speckles, silvercare, anti-tarnish and/or anti-corrosion agents, alkalinity sources, solubilizing agents, carriers, processing aids, pigments, and pH control agents (see, e.g., U.S. Pat. Nos. 6,610,642, 6,605,458, 5,705,464, 5,710,115, 5,698,504, 5,695,679, 5,686,014 and 5,646,101). Embodiments of specific cleaning composition materials are exemplified in detail below. As noted above, if a cleaning adjunct material is not compatible with a variant protease of the present invention included in a desired cleaning composition, then a suitable method of keeping the cleaning adjunct material(s) and the protease(s) separated (i.e., not in contact with each other) until combination of the components is appropriate is used. Such separation methods include any suitable method known in the art (e.g., gelcap, encapsulation, tablets, physical separation, etc.).

In some aspects, a composition (e.g., cleaning composition) of the invention comprises an effective amount of at least one variant protease of the invention that is useful or effective for cleaning a surface in need of proteinaceous stain removal. Such cleaning compositions include cleaning compositions for such applications as cleaning hard surfaces, laundry, fabrics, dishes, tableware, or dishware (e.g., by hand or manual dishwashing or automatic dishwashing). Indeed, in some embodiments, the present invention provides fabric cleaning compositions, while in other embodiments, the present invention provides non-fabric cleaning compositions. Notably, the present invention also provides cleaning compositions comprising at least one variant protease of the invention, wherein such cleaning compositions are suitable for personal care, including oral care (including dentrifices, toothpastes, mouthwashes, etc., as well as denture cleaning compositions), suitable for cleaning skin and hair, and suitable for cleaning eyeglasses. It is intended that the present invention encompass detergent compositions in any form (i.e., liquid, granular, bar, solid, semi-solid, gels, emulsions, tablets, capsules, etc.).

By way of example, several cleaning compositions wherein the variant proteases of the invention find use are described in greater detail below. In some embodiments in which the cleaning compositions of the invention are formulated as compositions suitable for use in laundry machine washing method(s), the compositions of the invention preferably contain at least one surfactant and at least one builder compound, as well as one or more cleaning adjunct materials, including, e.g., one or more cleaning adjunct materials selected from the group of organic polymeric compounds, bleaching agents, additional enzymes, suds suppressors, dispersants, lime-soap dispersants, soil suspension and anti-redeposition agents, and corrosion inhibitors. In some embodiments, laundry compositions also contain one or more softening agents (i.e., as additional cleaning adjunct materials). Additional exemplary laundry or fabric cleaning compositions and formulations to which one or more variant proteases of the invention can be added are presented in the Examples below.

The compositions of the invention also find use as detergent additive products in solid or liquid form. Such additive products are intended to supplement and/or boost the performance of conventional detergent compositions and can be added at any stage of the cleaning process. In some embodiments, the density of the laundry detergent composition ranges from about 400 to about 1200 g/liter, while in other embodiments, it ranges from about 500 to about 950 g/liter of composition measured at 20°C.

In one aspect, the invention provides cleaning compositions such as those provided in U.S. Pat. No. 6,605,458 comprising at least variant protease of the invention. In some embodiments, the composition comprising at least one variant protease of the invention is a compact granular fabric cleaning composition, while in other embodiments, the composition is a granular fabric cleaning composition useful in the laundering of colored fabrics; in other embodiments, the composition is a granular fabric cleaning composition which provides softening through the wash capacity, in additional embodiments, the composition is a heavy duty liquid fabric cleaning composition. In some embodiments, compositions comprising at least one variant protease of the invention are fabric cleaning compositions such as those described in U.S. Pat. Nos. 6,610,642 and 6,376,450. Also provided are granular laundry detergent compositions of particular utility under European or Japanese washing conditions (see, e.g., U.S. Pat. No. 6,610,642) which comprise at least one variant protease of the invention.

In some aspects, the invention provides hard surface cleaning compositions comprising at least one variant protease provided herein. Some such compositions comprise hard surface cleaning compositions such as those described in U.S. Pat. Nos. 6,610,642, 6,376,450, and 6,376,450 that include at least one such variant protease.

In one aspect, the invention provides hand dishwashing or automatic dishwashing detergent compositions comprising at least one variant protease provided herein. Some such compositions comprise hard surface cleaning compositions, such as those described in U.S. Pat. Nos. 6,610,642 and 6,376,450.

In one aspect, the invention provides cleaning compositions for use in manual or hand dishwashing or automatic dishwashing methods comprising at least one variant protease of the invention and/or at least one surfactant and/or at least one additional cleaning adjunct material selected from the group of organic polymeric compounds, suds enhancing agents, group II metal ions, solvents, hydrotropes, and additional enzymes.

In some embodiments in which the cleaning compositions of the invention are formulated as compositions suitable for use in automatic dishwashing machine method(s), the compositions of the invention typically contain at least one surfactant and/or at least one builder compound and may contain one or more cleaning adjunct materials preferably selected from organic polymeric compounds, bleaching agents, additional enzymes, suds suppressors, dispersants, lime-soap dispersants, soil suspension and anti-redeposition agents and corrosion inhibitors. Additional exemplary dishwashing compositions and formulations to which one or more variant proteases of the invention can be added are presented in the Examples below.

In another aspect, the invention provides oral care compositions comprising at least one variant protease of the present invention that are useful for oral care (e.g., cleaning teeth and dentures); components of oral care compositions that may be useful and included in such compositions include those described in U.S. Pat. No. 6,376,450. Compositions of the invention may further comprise cleaning adjunct materials and compounds described in the U.S. Pat. Nos. 6,376,450, 6,605,458, and 6,610,642.

The cleaning compositions of the invention are formulated into any suitable form and prepared by any process chosen by the formulator, non-limiting examples of which are described in U.S. Pat. Nos. 5,879,584, 5,691,297, 5,574,005, 5,569,645, 5,565,422, 5,516,448, 5,489,392, and 5,486,303. When a low pH cleaning composition is desired, the pH of such composition is adjusted via the addition of a material such as monoethanolamine or an acidic material such as hydrogen chloride (HCl).

While not essential for the purposes of the present invention, the non-limiting list of adjuncts illustrated hereinafter are suitable for use in the instant cleaning compositions. In some embodiments, these adjuncts are incorporated, for example, to assist or enhance cleaning performance for treatment of the substrate to be cleaned or to modify the aesthetics of the cleaning composition as is the case with perfumes, colorants, dyes or the like. It is understood that such adjuncts are in addition to the variant proteases of the present invention. The precise nature of these additional components, and levels of incorporation thereof, will depend on the physical form of the composition and the nature of the cleaning operation for which it is to be used. Suitable adjunct materials include, but are not limited to, e.g., surfactants, builders, chelating agents, dye transfer inhibiting agents, deposition aids, dispersants, additional enzymes, and enzyme stabilizers, catalytic materials, bleach activators, bleach boosters, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning compositions of the present invention.

In some aspects, cleaning compositions of the invention comprise at least one surfactant and/or a surfactant system, wherein the surfactant is selected from nonionic surfactants, anionic surfactants, cationic surfactants, ampholytic surfactants, zwitterionic surfactants, semi-polar nonionic surfactants and mixtures thereof. In some low pH cleaning composition embodiments (e.g., compositions having a neat pH of from about 3 to about 5), the composition typically does not contain alkyl ethoxylated sulfate, as it is believed that such surfactant may be hydrolyzed by such compositions the acidic contents. In some embodiments, the surfactant is present at a level of from about 0.1% to about 60%, while in alternative embodiments the level is from about 1% to about 50%, while in still further embodiments the level is from about 5% to about 40%, by weight of the cleaning composition.

In some aspects, cleaning compositions of the invention comprise one or more detergent builders or builder systems. In some such compositions incorporating at least one builder, the cleaning compositions comprise at least about 1%, from about 3% to about 60% or even from about 5% to about 40% builder by weight of the cleaning composition. Builders include, but are not limited to, e.g., alkali metal, ammonium and alkanolammonium salts of polyphosphates, alkali metal silicates, alkaline earth and alkali metal carbonates, aluminosilicates, polycarboxylate compounds, ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates, such as mellitic acid, succinic acid, citric acid, oxydisuccinic acid, polymaleic acid, benzene 1,3,5-tricarboxylic acid, carboxymethyloxysuccinic acid, and soluble salts thereof. It is contemplated that any suitable builder will find use in various compositions of the invention.

In some such compositions, the builders form water-soluble hardness ion complexes (e.g., sequestering builders), such as citrates and polyphosphates (e.g., sodium tripolyphosphate and sodium tripolyphospate hexahydrate, potassium tripolyphosphate, and mixed sodium and potassium tripolyphosphate, etc.). It is contemplated that any suitable builder will find use in the present invention, including those known in the art (see, e.g., EP 2 100 949).

Some cleaning compositions of the invention comprise at least one chelating agent in addition to at least one variant protease. Suitable chelating agents include, but are not limited to, e.g., copper, iron, and/or manganese chelating agents and mixtures thereof. In embodiments in which at least one chelating agent is used, the cleaning compositions of the present invention comprise from about 0.1% to about 15% or even from about 3% to about 10% chelating agent by weight of the subject cleaning composition.

Some cleaning compositions provided herein comprise at least one deposition aid in addition to at least one variant protease. Suitable deposition aids include, but are not limited to, e.g., polyethylene glycol, polypropylene glycol, polycarboxylate, soil release polymers such as polytelephthalic acid, clays such as kaolinite, montmorillonite, atapulgite, illite, bentonite, halloysite, and mixtures thereof.

As indicated herein, in some embodiments, anti-redeposition agents find use in some embodiments of the present invention. In some embodiments, non-ionic surfactants find use. These non-ionic surfactants also find use in preventing the re-deposition of soils. In some embodiments, the anti-redeposition agent is a non-ionic surfactant as known in the art (see, e.g., EP 2 100 949).

In some embodiments, the cleaning compositions of the present invention include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof. In embodiments in which at least one dye transfer inhibiting agent is used, the cleaning compositions of the present invention comprise from about 0.0001% to about 10%, from about 0.01% to about 5%, or even from about 0.1% to about 3% by weight of the cleaning composition.

In some embodiments, silicates are included within the compositions of the present invention. In some such embodiments, sodium silicates (e.g., sodium disilicate, sodium metasilicate, and crystalline phyllosilicates) find use. In some embodiments, silicates are present at a level of from about 1% to about 20%. In some embodiments, silicates are present at a level of from about 5% to about 15% by weight of the composition.

In some still additional embodiments, the cleaning compositions of the invention also comprise dispersants. Suitable water-soluble organic materials include, but are not limited to, e.g., the homo- or co-polymeric acids or their salts, in which the polycarboxylic acid comprises at least two carboxyl radicals separated from each other by not more than two carbon atoms.

In some further embodiments, the enzymes (e.g., variant proteases of the invention or other additional enzymes) used in the cleaning compositions are stabilized any suitable technique. In some embodiments, the enzymes employed herein are stabilized by the presence of water-soluble sources of calcium and/or magnesium ions in the finished compositions that provide such ions to the enzymes. In some embodiments, the enzyme stabilizers include oligosaccharides, polysaccharides, and inorganic divalent metal salts, including alkaline earth metals, such as calcium salts. It is contemplated that various techniques for enzyme stabilization will find use in the present invention. For example, in some embodiments, the enzymes employed herein are stabilized by the presence of water-soluble sources of zinc (II), calcium (II) and/or magnesium (II) ions in the finished compositions that provide such ions to the enzymes, as well as other metal ions (e.g., barium (II), scandium (II), iron (II), manganese (II), aluminum (III), tin (II), cobalt (II), copper (II), nickel (II), and oxovanadium (IV). Chlorides and sulfates also find use in some embodiments of the present invention. Examples of suitable oligosaccharides and polysaccharides (e.g., dextrins) are known in the art (see, e.g., WO 07/145964). In some embodiments, reversible protease inhibitors also find use, such as boron-containing compounds (e.g., borate, 4-formyl phenyl boronic acid) and/or a tripeptide aldehyde find use in compositions of the invention to further improve stability, as desired.

In some embodiments, bleaches, bleach activators, and/or bleach catalysts are included in the compositions of the invention. In some embodiments, the cleaning compositions of the invention comprise inorganic and/or organic bleaching compound(s). Inorganic bleaches include, but are not limited to perhydrate salts (e.g., perborate, percarbonate, perphosphate, persulfate, and persilicate salts). In some embodiments, inorganic perhydrate salts are alkali metal salts. In some embodiments, inorganic perhydrate salts are included as the crystalline solid, without additional protection, although in some other embodiments, the salt is coated. Any suitable salt known in the art finds use in the compositions of the invention (see, e.g., EP 2 100 949).

In some embodiments, bleach activators are used in the compositions of the invention. Bleach activators are typically organic peracid precursors that enhance the bleaching action in the course of cleaning at temperatures of 60°C and below. Bleach activators suitable for use herein include compounds which, under perhydrolysis conditions, give aliphaic peroxycarboxylic acids having preferably from about 1 to about 10 carbon atoms, in particular from about 2 to about 4 carbon atoms, and/or optionally substituted perbenzoic acid. Additional bleach activators are known in the art and find use in the composition of the invention (see, e.g., EP 2 100 949).

In addition, in some embodiments and as further described herein, the cleaning compositions of the invention further comprise at least one bleach catalyst. In some embodiments, the manganese triazacyclononane and related complexes find use, as well as cobalt, copper, manganese, and iron complexes. Additional bleach catalysts find use in the present invention (see, e.g., U.S. Pat Nos. 4,246,612, 5,227,084, and 4,810410; WO 99/06521; and EP 2 100 949).

In some embodiments, the cleaning compositions of the invention comprise one or more catalytic metal complexes. In some embodiments, a metal-containing bleach catalyst finds use. In some embodiments, the metal bleach catalyst comprises a catalyst system comprising a transition metal cation of defined bleach catalytic activity (e.g., copper, iron, titanium, ruthenium, tungsten, molybdenum, or manganese cations), an auxiliary metal cation having little or no bleach catalytic activity (e.g., zinc or aluminum cations), and a sequestrate having defined stability constants for the catalytic and auxiliary metal cations, particularly ethylenediaminetetraacetic acid, ethylenediaminetetra (methylenephosphonic acid) and water-soluble salts thereof are used (see, e.g., U.S. Pat. No. 4,430,243). In some embodiments, the cleaning compositions of the invention are catalyzed by means of a manganese compound. Such compounds and levels of use are well known in the art (see, e.g., U.S. Patent No. 5,576,282). In additional embodiments, cobalt bleach catalysts find use and are included in the cleaning compositions of the invention. Various cobalt bleach catalysts are known in the art (see, e.g., U.S. Patent Nos. 5,597,936 and 5,595,967) and are readily prepared by known procedures.

In some additional embodiments, the cleaning compositions of the invention include a transition metal complex of a macropolycyclic rigid ligand (MRL). As a practical matter, and not by way of limitation, in some embodiments, the compositions and cleaning processes provided by the invention are adjusted to provide on the order of at least one part per hundred million of the active MRL species in the aqueous washing medium, and in some embodiments, provide from about 0.005 ppm to about 25 ppm, from about 0.05 ppm to about 10 ppm, and from about 0.1 ppm to about 5 ppm, of the MRL in the wash liquor.

In some embodiments, transition-metals in the instant transition-metal bleach catalyst include, but are not limited to, e.g., manganese, iron and chromium. Preferred MRLs also include, but are not limited to, e.g., special ultra-rigid ligands that are cross-bridged (e.g., 5,12-diethyl-1,5,8,12-tetraazabicyclo(6.6.2)hexadecane). Suitable transition metal MRLs are readily prepared by known procedures (see, e.g., WO 2000/32601 and U.S. Pat. No. 6,225,464).

In one aspect, the invention provides an automatic dishwashing detergent composition formulated as a detergent tablet. Such tablet comprises at least one variant protease of the invention and a builder, such as, e.g., a builder salt. Some such tablets have an alkalinity of at least equivalent to 3 grams (g) of sodium hydroxide per 100 grams of the tablet composition and a density of at least 1.4 grams/cubic centimeter. The builder salt can comprise a mixture of a silicate salt and a phosphate salt, preferably with more silicate (e.g., sodium metasilicate) than phosphate (e.g., sodium tripolyphosphate). Some such tablets are free of surfactant materials and are especially adapted for use in automatic dishwashing machines.

In one aspect, the cleaning compositions of the present invention comprise metal care agents. Metal care agents are useful in preventing and/or reducing the tarnishing, corrosion, and/or oxidation of metals, including aluminum, stainless steel, and non-ferrous metals (e.g., silver and copper). Suitable metal care agents include those described in EP 2 100 949, WO 9426860, and WO 94/26859). In some such cleaning compositions, the metal care agent is a zinc salt. Some such cleaning compositions comprise from about 0.1% to about 5% by weight of one or more metal care agent.

As indicated above, the cleaning compositions of the present invention are formulated into any suitable form and prepared by any process chosen by the formulator, non-limiting examples of which are described in U.S. Pat. Nos. 5,879,584, 5,691,297, 5,574,005, 5,569,645, 5,516,448, 5,489,392, and 5,486,303. In some embodiments in which a low pH cleaning composition is desired, the pH of such composition is adjusted via the addition of an acidic material such as hydrogen chloride (HCl).

The cleaning compositions disclosed herein find use in cleaning a situs (e.g., a surface, dishware, tableware, or fabric). Typically, at least a portion of the situs is contacted with a present cleaning composition of the invention in neat form or diluted in a wash liquor, and then the situs is optionally washed and/or rinsed. For purposes of the present invention, "washing" includes, but is not limited to, e.g., scrubbing and mechanical agitation. In some embodiments, the cleaning compositions are employed at concentrations of from about 500 ppm to about 15,000 ppm in solution. When the wash solvent is water, the water temperature typically ranges from about 5°C to about 90°C and when the situs comprises a fabric, the water to fabric mass ratio is typically from about 1:1 to about 30:1.

**Processes of Making and Using Cleaning Compositions**

The cleaning compositions of the invention described herein and throughout can be formulated into any suitable form and prepared by any suitable process chosen by the formulator (see e.g., US Patent Nos. 5,879,584, 5,691,297, 5,574,005, 5,569,645, 5,565,422, 5,516,448, 5,489,392, 5,486,303, 4,515,705, 4,537,706, 4,515,707, 4,550,862, 4,561,998, 4,597,898, 4,968,451, 5,565,145, 5,929,022, 6,294,514 and 6,376,445).

In some embodiments, the cleaning compositions of the invention are provided in unit dose form, including tablets, capsules, sachets, pouches, and multi-compartment pouches. In some embodiments, the unit dose format is designed to provide controlled release of the ingredients within a multi-compartment pouch (or other unit dose format). Suitable unit dose and controlled release formats are known in the art (*See e.g.*, EP 2 100 949, WO 02/102955, U.S. Pat. Nos. 4,765,916 and 4,972,017, and WO 04/111178 for materials suitable for use in unit dose and controlled release formats). In some embodiments, the unit dose form is provided by tablets wrapped with a water-soluble film or water-soluble pouches. Various formats for unit doses are provided in EP 2 100 947, and are known in the art.

## Methods of the Invention

The invention provides methods for cleaning or washing an item or surface (e.g., hard surface) in need of cleaning, including, but not limited to, e.g., methods for cleaning or washing a dishware item, a tableware item, a fabric item, a laundry item, personal care item, etc., or the like, and methods for cleaning or washing a hard or soft surface, such as, e.g., a hard surface of an item.

In one aspect, the invention provides a method for cleaning an item, object, or surface in need of cleaning, the method comprising contacting the item or surface (or a portion of the item or surface desired to be cleaned) with a variant protease of any of the invention or a composition of the invention for a sufficient time and/or under conditions suitable or effective to clean the item, object, or surface to a desired degree. Some such methods further comprise rinsing the item, object, or surface with water. For some such methods, the cleaning composition is a dishwashing detergent composition and the item or object to be cleaned is a dishware item or tableware item. A dishware item is a dish item generally used in serving or eating food. A dishware item can be, but is not limited to, e.g., a dish, plate, cup, bowl, etc., and the like. Tableware is a broader term that includes, but is not limited, to, e.g., dishes, cutlery, knives, forks, spoons, chopsticks, glassware, pitchers, sauce boats, drinking vessels, etc., and the like; a tableware item includes any of these or similar items for serving or eating food. For some such methods, the cleaning composition is an automatic dishwashing detergent composition or a hand dishwashing detergent composition and the item or object to be cleaned is a dishware or tableware item. For some such methods, the cleaning composition is a laundry detergent composition, such as, e.g., a power laundry detergent composition or a liquid laundry detergent composition, and the item to be cleaned is a fabric item.

In one aspect, the invention provides methods for cleaning or washing a fabric item optionally in need of cleaning or washing, respectively. Some such methods comprise providing a composition comprising the variant protease (such as, but not limited to, e.g., a fabric or laundry cleaning composition) and a fabric item or laundry item in need of cleaning, and contacting the fabric item or laundry item (or a portion of the item desired to be cleaned) with the composition under conditions sufficient or effective to clean or wash the fabric or laundry item to a desired degree.

In one aspect, the invention provides a method for cleaning or washing an item or surface (e.g., hard surface) optionally in need of cleaning, the method comprising providing an item or surface to be cleaned or washed and contacting the item or surface (or a portion of the item or surface desired to be cleaned or washed) with at least one variant protease of the invention or a composition of the invention comprising at least one such variant protease for a sufficient time and/or under conditions sufficient or effective to clean or wash the item or surface to a desired degree. Such compositions include, but are not limited to, e.g., a cleaning composition or detergent composition of the invention (including, but not limited to, e.g., hand dishwashing detergent composition, hand dishwashing cleaning composition, laundry detergent or fabric detergent or laundry or fabric cleaning composition, liquid laundry detergent, liquid laundry cleaning composition, powder laundry detergent composition, powder laundry cleaning composition, automatic dishwashing detergent composition, laundry booster cleaning or detergent composition, laundry cleaning additive, and laundry pre-spotter composition, etc.). In some instances, if desired, the method can be repeated one or more times, particularly if additional cleaning or washing is desired. For example, in some instance, the method optionally further comprises allowing the item or surface to remain in contact with the at least one variant protease or composition for a period of time sufficient or effective to clean or wash the item or surface to the desired degree. Some such methods further comprise rinsing the item or surface with water. Some such methods further comprise contacting the item or surface with at least one variant protease of the invention or a composition of the invention again and allowing the item or surface to remain in contact with the at least one variant protease or composition for a period of time sufficient to clean or wash the item or surface to the desired degree. For some such methods, the cleaning composition is a dishwashing detergent composition and the item to be cleaned is a dishware or tableware item. For some such methods, the cleaning composition is an automatic dishwashing detergent composition or a hand dishwashing detergent composition and the item to be cleaned is a dishware or tableware item. For some such methods, the cleaning composition is a laundry detergent composition and the item to be cleaned is a fabric item.

In one aspect, the invention provides a method of cleaning a tableware or dishware item in an automatic dishwashing machine, the method comprising providing an automatic dishwashing machine, placing an amount of an automatic dishwashing composition comprising at least one variant protease of the invention or a composition of the invention sufficient to clean the tableware or dishware item in the machine (e.g., by placing the composition in an appropriate or provided detergent compartment or dispenser in the machine), putting a dishware or tableware item in the machine, and operating the machine so as to clean the tableware or dishware item (e.g., as per the manufacturer's instructions). Such method can include any automatic dishwashing composition described herein, which comprises, but is not limited to, e.g., any variant protease described herein. The amount of automatic dishwashing composition to be used can be readily determined according to manufacturer's instructions or suggestions and any form of automatic dishwashing composition comprising at least one variant protease of the invention (e.g., liquid, powder, solid, gel, table, etc.), including any described herein, may be employed.

In one aspect, the invention provides a method for cleaning a surface, item or object optionally in need of cleaning, the method comprises contacting the item or surface (or a portion of the item or surface desired to be cleaned) with at least one variant protease of the invention or a cleaning composition of the invention in neat form or diluted in a wash liquor for a sufficient time and/or under conditions sufficient or effective to clean or wash the item or surface to a desired degree. The surface, item, or object may then be (optionally) washed and/or rinsed if desired. For purposes of the present invention, "washing" includes, but is not limited to, e.g., scrubbing and mechanical agitation. In some embodiments, the cleaning compositions are employed at concentrations of from about 500 ppm to about 15,000 ppm in solution (e.g., aqueous solution). When the wash solvent is water, the water temperature typically ranges from about 5°C to about 90°C and when the situs comprises a fabric, the water to fabric mass ratio is typically from about 1:1 to about 30:1.

In one aspect, the invention provides a method of cleaning a laundry or fabric item in an washing machine, the method comprising providing an washing machine, placing an amount of a laundry detergent composition comprising at least one variant protease of the invention sufficient to clean the laundry or fabric item in the machine (e.g., by placing the composition in an appropriate or provided detergent compartment or dispenser in the machine), putting the laundry or fabric item in the machine, and operating the machine so as to clean the tableware or dishware item (e.g., as per the manufacturer's instructions). Such method can include any laundry washing detergent composition described herein, which comprises, but is not limited to, e.g., any variant protease described herein. The amount of laundry detergent composition to be used can be readily determined according to manufacturer's instructions or suggestions and any form of laundry detergent composition comprising at least one variant protease of the invention (e.g., solid, powder, liquid, tablet, gel, etc.), including any described herein, may be employed.

Additional exemplary cleaning methods are provided in the Examples below.

## Additional Aspects of the Invention

In one aspect, the invention provides any composition of the invention (e.g., cleaning composition or detergent composition) described herein and throughout, said composition comprising any polypeptide of the invention (e.g., any protease variant or subtilisin variant of the invention) as defined in the claims, but wherein said composition is not an automatic dishwashing composition. Also disclosed herein is a composition comprising any variant protease of the invention described throughout and herein, but wherein said composition does not include an automatic dishwashing detergent composition comprising a variant protease of a parent protease, said parent protease comprising an amino acid sequence having at least 97%, at least 99%, or 100% identity to the amino acid sequence of SEQ ID NO:1, said variant protease of said parent protease comprising one of the following sets of amino acid substitutions versus said parent protease:

(i) N76D+S87R+G118R+S128L+P129Q+S130A, with the proviso that said variant protease does not comprise N248R+S188D, or
(ii) N76D+S87R+G118R+S128L+P129Q+S130A+S188D+V244R,
(iii) and a builder,

wherein amino acid positions are numbered according to the numbering of corresponding amino acid positions in the amino acid sequence of Bacillus amyloliquefaciens subtilisin BPN' shown in SEQ ID NO:2 as determined by alignment of the variant protease amino acid sequence with the Bacillus amyloliquefaciens subtilisin BPN' amino acid sequence. In one aspect, such composition is detergent composition. In one aspect, such composition is a cleaning composition. In one aspect, such composition is a liquid detergent or powder detergent composition. In one aspect, such composition is a cleaning composition in liquid, gel, tablet, powder, solid, or granule form. In one aspect, such composition is an automatic dishwashing detergent composition. In one aspect, such composition is a cleaning composition that does not contain phosphate. In any aspect, said composition comprises at least one additional enzyme. In any aspect of said composition, said composition comprises at least such at least one additional enzyme can be selected from the group consisting of hemicellulase, cellulase, amylase, peroxidase, protease, xylanase, lipase, phospholipase, esterase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, and laccase. In any aspect of said composition, said composition may comprise two or more additional enzymes selected from the group consisting of hemicellulase, cellulase, amylase, peroxidase, protease, xylanase, lipase, phospholipase, esterase, cutinase, pectinase, pectate lyase, mannanase, keratinase, reductase, oxidase, phenoloxidase, lipoxygenase, ligninase, pullulanase, tannase, pentosanase, malanase, β-glucanase, arabinosidase, hyaluronidase, chondroitinase, and laccase. In any aspect of said composition, said composition may further comprise at least one builder and/or at least one surfactant.

In one aspect, the invention provides a method for cleaning an item or surface in need of cleaning, the method comprising contacting the item or surface with any of said aforementioned compositions. In one aspect of said method, said method further comprises rinsing the item or surface with water. In one aspect of said method, the cleaning composition is a dishwashing detergent composition and the item to be cleaned is a dishware or tableware item. In one aspect of said method, the cleaning composition is an automatic dishwashing detergent composition and the item to be cleaned is a dishware or tableware item.

In one aspect, the invention provides a method for cleaning a surface, item, or object, the method comprising contacting at least a portion of the item or surface to be cleaned with any of said aforementioned compositions, for a sufficient time and/or under conditions sufficient or effective to clean or wash the item or surface to a desired degree, and optionally further comprising rinsing the surface, item, or object with water.

In one aspect, the invention provides a method of cleaning a tableware or dishware item in an automatic dishwashing machine, the method comprising (i) providing an automatic dishwashing machine, (ii) placing an amount of any of said aforementioned compositions or automatic dishwashing compositions sufficient to clean the tableware or dishware item in the machine, wherein said composition is optionally placed in a compartment or dispenser in said machine, (iii) putting a dishware or tableware item in the machine, and (iv) operating the machine to clean the tableware or dishware item.

## EXAMPLES

The following examples are provided in order to demonstrate and further illustrate certain aspects of the present invention and are not to be construed as limiting the scope thereof.

In the experimental disclosure which follows and elsewhere herein, the following abbreviations apply: PI (proteinase inhibitor), ppm (parts per million); M (molar); mM (millimolar); $\mu$M (micromolar); nM (nanomolar); mol (mole); mmol (millimole); $\mu$mol (micromole); nmol (nanomole); gm (gram); mg (milligram); $\mu$g (microgram); pg (picogram); L or l (liter); ml and mL (milliliters); $\mu$l or $\mu$L (microliter); cm (centimeter); mm (millimeter); $\mu$m (micrometer); nm (nanometer); U (units); V (volt); MW (molecular weight); sec (second); min(s) (minute/minutes); h(s) or hr(s) (hour/hours); °C (degrees Centigrade); ND (not determined); rpm (revolutions per minute); GH (degrees German hardness); $H_2O$ (water); $dH_2O$ (deionized water); HCl (hydrochloric acid); aa (amino acid); bp (base pair); kb (kilobase pair); kD (kilodaltons); cDNA (copy or complementary DNA); DNA (deoxyribonucleic acid); ssDNA (single stranded DNA); dsDNA (double stranded DNA); RNA (ribonucleic acid); $MgCl_2$ (magnesium chloride); NaCl (sodium chloride); BPN' (*Bacillus amyloliquefaciens* subtilisin); PB92 (*Bacillus clausii* subtilisin); w/v (weight to volume); v/v (volume to volume); w/w (weight to weight); g (gravity); OD (optical density); ppm (parts per million); $OD_{280}$ (optical density at 280 nm); $OD_{600}$ (optical density at 600 nm); $A_{405}$ (absorbance at 405 nm); PAGE (polyacrylamide gel electrophoresis); PBS (phosphate buffered saline [150 mM NaCl, 10 mM sodium phosphate buffer, pH 7.2]); PEG (polyethylene glycol); PCR (polymerase chain reaction); SDS (sodium dodecyl sulfate); TRIS or Tris (tris(hydroxymethyl)aminomethane); HEPES (N-[2-Hydroxyethyl]piperazine-N-[2-ethanesulfonic acid]); HBS (HEPES buffered saline); Tris-HCl (tris[Hydroxymethyl]aminomethane-hydrochloride); DMSO (dimethyl sulfoxide); SA (sinapinic acid (s,5-dimethoxy-4-hydroxy cinnamic acid); TCA (trichloroacetic acid); HPLC (high pressure liquid chromatography); *Taq* (*Thermus aquaticus* DNA polymerase); Klenow (DNA polymerase I large (Klenow) fragment); EDTA (ethylenediaminetetracetic acid); bla ($\beta$-lactamase or ampicillin-resistance gene); HDL (high density liquid); HDD (heavy duty powder detergent); HSG (high suds granular detergent); CEE (Central and Eastern Europe); WE (Western Europe); NA, when used in reference to detergents (North America); Japan and JPN, when used in reference to detergents (Japan); CFT (Center for Test Materials, Vlaardingen, the Netherlands); P&G and Procter & Gamble (Procter & Gamble, Inc., Cincinnati, OH); DNA2.0 (DNA2.0, Menlo Park, CA); Corning (Corning Life Sciences, Corning, NY); ATCC (American Type Culture Collection, Rockville, MD); Sigma (Sigma Chemical Co., St. Louis, MO); NCBI (National Center for Biotechnology Information); Operon Technologies (Operon Technologies, Inc., Alameda, CA); Invitrogen (Invitrogen Corp., San Diego, CA); Qiagen (Qiagen, Inc., Valencia, CA); Molecular Devices (Molecular Devices Corp., Sunnyvale, CA); Siegfried Handel (Siegfried Handel AG, Zofingen, Switzerland); Stratagene (Stratagene Cloning Systems, La Jolla, CA); Monsanto (Monsanto Co., St. Louis, MO); Wintershall (Wintershall AG, Kassel, Germany); BASF (BASF Co., Florham Park, NJ); Huntsman (Huntsman Petrochemical Corp., Salt Lake City, UT); Enichem (Enichem Iberica, Barcelona, Spain); Fluka Chemie AG (Fluka Chemie AG, Buchs, Switzerland); Gist-Brocades (Gist-Brocades, NV, Delft, the Netherlands); Dow Corning (Dow Corning Corp., Midland, MI); RB (Reckitt-Benckiser, Slough, UK).

As used herein, in some lists, a leading "0" is indicated, in order to provide a three number designation for each site (*e.g.*, "001" is the same as "1," so "A001C" is the same as "A1C"). In some lists, the leading "0" is not included. In addition, as used herein, "X" refers to any amino acid.

In the exemplified detergent compositions provided herein, the enzymes levels are expressed by pure enzyme by weight of the total composition and unless otherwise specified, the detergent ingredients are expressed by weight of the total compositions. The abbreviated component identifications therein have the following meanings:

| Abbreviation | Ingredient |
|---|---|
| LAS | : Sodium linear $C_{11-13}$ alkyl benzene sulfonate |
| NaC16-17HSAS | : Sodium $C_{16-17}$ highly soluble alkyl sulfate |
| TAS | : Sodium tallow alkyl sulphate |
| CxyAS | : Sodium $C_{1x}$ - $C_{1y}$ alkyl sulfate |
| CxyEz | : $C_{1x}$ - $C_{1y}$ predominantly linear primary alcohol condensed with an average of z moles of ethylene oxide |
| CxyAEzS | : $C_{1x}$ - $C_{1y}$ sodium alkyl sulfate condensed with an average of z moles of ethylene oxide. Added molecule name in the examples. |
| Nonionic | : Mixed ethoxylated/propoxylated fatty alcohol e.g. Plurafac LF404 being an alcohol with an average degree of ethoxylation of 3.8 and an average degree of propoxylation of 4.5 |
| QAS | : $R_2.N+(CH_3)_2(C_2H_4OH)$ with $R_2$ = $C_{12}$-$C_{14}$. |
| Silicate | : Amorphous Sodium Silicate ($SiO_2$:$Na_2O$ ratio = 1.6-3.2:1) |
| Metasilicate | : Sodium metasilicate ($SiO_2$:$Na_2O$ ratio = 1.0) |
| Zeolite A | : Hydrated aluminosilicate of formula $Na_{12}(AlO_2SiO_2)_{12}$. $27H_2O$ |
| SKS-6 | : Crystalline layered silicate of formula $\delta$-$Na_2Si_2O_5$ |
| Sulfate | : Anhydrous sodium sulphate. |
| STPP | : Sodium Tripolyphosphate |
| MA/AA | : Random copolymer of 4:1 acrylate/maleate, average molecular weight about 70,000-80,000 |
| AA | : Sodium polyacrylate polymer of average molecular weight 4,500 |
| Polycarboxylate | : Copolymer comprising mixture of carboxylated monomers such as acrylate, maleate and methyacrylate with a MW ranging between 2,000-80,000 such as Sokolan commercially available from BASF, being a copolymer of acrylic acid, MW4,500 |
| BB1 | : 3-(3,4-Dihydroisoquinolinium)propane sulfonate |
| BB2 | 1-(3,4-dihydroisoquinolinium)-decane-2-sulfate |
| PB1 | : Sodium perborate monohydrate |
| PB4 | : Sodium perborate tetrahydrate of nominal formula $NaBO_3.4H_2O$. |
| Percarbonate | : Sodium percarbonate of nominal formula $2Na_2CO_3.3H_2O_2$. |
| TAED | : Tetraacetyl ethylene diamine |
| NOBS | : Nonanoyloxybenzene sulfonate in the form of the sodium salt |
| DTPA | : Diethylene triamine pentaacetic acid |
| HEDP | diphosphonic acid |
| DETPMP | : Diethyltriamine penta (methylene) phosphonate, marketed by Monsanto under the Tradename Dequest 2060 |
| EDDS | : Ethylenediamine-N,N'-disuccinic acid, (S,S) isomer in the form of its sodium salt |
| Diamine | : Dimethyl aminopropyl amine; 1,6-hezane diamine; 1,3-propane diamine; 2-methyl-1,5-pentane diamine; 1,3-pentanediamine; 1-methyl-diaminopropane |
| DETBCHD | 5, 12- diethyl-1,5,8,12-tetraazabicyclo [6,6,2] hexadecane, dichloride, Mn(II) SALT |
| PAAC | : Pentaamine acetate cobalt(III) salt |
| Paraffin | : Paraffin oil sold under the tradename Winog 70 by Wintershall |
| Paraffin Sulfonate | : A Paraffin oil or wax in which some of the hydrogen atoms have been replaced by sulfonate groups |
| Aldose oxidase | : Oxidase enzyme sold under the tradename Aldose Oxidase by Novozymes A/S |
| Galactose oxidase | : Galactose oxidase from Sigma |
| nprE | : The recombinant form of neutral metalloprotease expressed in *Bacillus subtilis* (see e.g., WO 07/044993) |
| PMN | : Purified neutral metalloprotease from *Bacillus amyloliquefacients* |
| Amylase | : A suitable amylolytic enzyme, such as those sold under the tradenames PURAFECT® Ox described in WO 94/18314, WO96/05295 sold by Genencor; NATALASE®, TERMAMYL®, FUNGAMYl® and DURAMYL™, all available from Novozymes A/S |

(continued)

| Abbreviation | Ingredient |
|---|---|
| Lipase | : A suitable lipolytic enzyme such as those sold under the tradenames LIPEX®, LIPOLASE®, LIPOLASE® Ultra by Novozymes A/S and Lipomax™ by Gist-Brocades |
| Cellulase | : A suitable cellulytic enzyme such as those sold under the tradenames CAREZYME®, CELLUZYME®, and/or ENDOLASE® by Novozymes A/S |
| Pectin Lyase | : A suitable pectin lyase, such as those sold under the tradenames PECTAWAY® and PECTAWASH® available from Novozymes A/S |
| PVP | : Polyvinylpyrrolidone with an average molecular weight of 60,000 |
| PVNO | : Polyvinylpyridine-N-Oxide, with an average molecular weight of 50,000 |
| PVPVI | : Copolymer of vinylimidazole and vinylpyrrolidone, with an average molecular weight of 20,000 |
| Brightener 1 | : Disodium 4,4'-bis(2-sulphostyryl)biphenyl |
| Silicone antifoam | : Polydimethylsiloxane foam controller with siloxane-oxyalkylene copolymer as dispersing agent with a ratio of said foam controller to said dispersing agent of 10:1 to 100:1 |
| Suds Suppressor | : 12% Silicone/silica, 18% stearyl alcohol, 70% starch in granular form |
| SRP 1 | : Anionically end capped polyesters |
| PEG X | : Polyethylene glycol, of a molecular weight of X |
| PVP K60® | : Vinylpyrrolidone homopolymer (average MW 160,000) |
| Jeffamine® ED-2001 | : Capped polyethylene glycol from Huntsman |
| Isachem® AS | : A branched alcohol alkyl sulphate from Enichem |
| MME PEG (2000) | : Monomethyl ether polyethylene glycol (MW 2000) from Fluka Chemie AG |
| DC3225C | : Silicone suds suppresser, mixture of Silicone oil and Silica from Dow Corning |
| TEPAE | : Tetreaethylenepentaamine ethoxylate |
| BTA | : Benzotriazole |
| Betaine | : $(CH_3)_3N+CH_2COO^-$ |
| Sugar | : Industry grade D-glucose or food grade sugar |
| CFAA | : $C_{12}$-$C_{14}$ alkyl N-methyl glucamide |
| TPKFA | : $C_{12}$-$C_{14}$ topped whole cut fatty acids |
| Clay | : A hydrated aluminumu silicate in a general formula $Al_2O_3SiO_2 \cdot xH_2O$. Types: Kaolinite, montmorillonite, atapulgite, illite, bentonite, halloysite |

For North American (NA) and Western European (WE) heavy duty liquid laundry (HDL) detergents, heat inactivation of the enzymes present in commercially-available detergents is performed by placing pre-weighed liquid detergent (in a glass bottle) in a water bath at 95°C for 2 hours. The incubation time for heat inactivation of NA and WE auto dish washing (ADW) detergents is 8 hours. Both un-heated and heated detergents are assayed within 5 minutes of dissolving the detergent to accurately determine percentage deactivated. Enzyme activity is tested by the AAPF assay.

For testing of enzyme activity in heat-inactivated detergents, working solutions of detergents are made from the heat inactivated stocks. Appropriate amounts of water hardness (e.g., 6 grains per gallon (gpg) or 12 gpg) and buffer are added to the detergent solutions to match the desired conditions. The solutions are mixed by vortexing or inverting the bottles. Some commercially-available detergents are described in the following Examples.

**EXAMPLE 1**

**Construction of Variant Proteases**

The variant protease polypeptides of the invention and nucleic acids of the invention that encode such variant proteases can be created by using one or more of a variety of standard methods well known to those of ordinary skill in the art. For example, a nucleic acid encoding a variant protease of the invention can be constructed by performing site-directed mutagenesis of a plasmid DNA encoding a *B. lentus* GG36 protease-encoding nucleotide sequence set forth in SEQ ID NO:18 such that the resultant variant protease encoded therefrom comprises one or more desired amino acid substitutions (mutations) at the desired amino acid positions relative to the amino acid sequence of GG36 set forth in SEQ ID NO:1. For example, one of skill in the art can readily employ standard site-directed mutagenesis procedures using the nucleotide sequence of SEQ ID NO:18 (which encodes the GG36 protease polypeptide) to produce a nucleic acid that encodes a variant protease polypeptide comprising desired amino acid substitutions at one or more amino acid positions of GG36 protease (SEQ ID NO:1). Further, one skilled in the art can readily employ known site-mutagenesis procedures using the nucleotide sequence of SEQ ID NO:18 to produce a nucleic acid that encodes a variant protease polypeptide comprising amino acid substitutions at one or more of amino acid positions of GG36 (SEQ ID NO:1) selected from the group consisting of 76, 87, 118, 128, 129, 130, 188, 244, and 248, wherein each amino acid position is numbered according the to the numbering of corresponding amino acid position in the amino acid sequence of *Bacillus amyloliquefaciens* subtilisin BPN' shown in SEQ ID NO:2 as determined by alignment of the variant protease amino acid sequence of interest with the BPN' amino acid sequence.

Such procedures can also be readily employed, e.g., to produce nucleic acids encoding variant proteases PX3 (SEQ ID NO:6), PX4 (SEQ ID NO:7), and PX5 (SEQ ID NO:8) and variant proteases of each thereof, such as variants of PX3, PX4, and PX5 comprising additional amino acid substitutions, including, e.g., non-conservative and conservative amino acid substitutions, amino acid deletions, and/or amino acid insertions or additions.

A GG36 protease-encoding nucleotide sequence is as follows:

gtgagaagcaaaaaattgtggatcgtcgcgtcgaccgcactactcatttctgttgctttcagttcatcgatcgcatcggct<u>gctgaagaagca</u>

<u>aaagaaaaatatttaattggctttaatgagcaggaagctgtcagtgagtttgtagaacaagtagaggcaaatgacgaggtcgccattctctct</u>

<u>gaggaagaggaagtcgaaattgaattgcttcatgaatttgaaacgattcctgttttatccgttgagttaagcccagaagatgtggacgcgcttg</u>

<u>agctcgatccagcgatttcttatattgaagaggatgcagaagtaacgacaatg</u>GCGCAATCAGTGCCATGGGGAATT

AGCCGTGTGCAAGCCCCAGCTGCCCATAACCGTGGATTGACAGGTTCTGGTGTAAA

AGTTGCTGTCCTCGATACAGGTATTTCCACTCATCCAGACTTAAATATTCGTGGTGG

CGCTAGCTTTGTACCAGGGGAACCATCCACTCAAGATGGGAATGGGCATGGCACGC

ATGTGGCCGGGACGATTGCTGCTTTAAACAATTCGATTGGCGTTCTTGGCGTAGCGC

CGAGCGCGGAACTATACGCTGTTAAAGTATTAGGGGCGAGCGGTTCAGGTTCGGTC

AGCTCGATTGCCCAAGGATTGGAATGGGCAGGGAACAATGGCATGCACGTTGCTAA

TTTGAGTTTAGGAAGCCCTTCGCCAAGTGCCACACTTGAGCAAGCTGTTAATAGCGC

GACTTCTAGAGGCGTTCTTGTTGTAGCGGCATCTGGAAATTCAGGTGCAGGCTCAAT

CAGCTATCCGGCCCGTTATGCGAACGCAATGGCAGTCGGAGCTACTGACCAAAACA

ACAACCGCGCCAGCTTTTCACAGTATGGCGCAGGGCTTGACATTGTCGCACCAGGT

GTAAACGTGCAGAGCACATACCCAGGTTCAACGTATGCCAGCTTAAACGGTACATC

GATGGCTACTCCTCATGTTGCAGGTGCAGCAGCCCTTGTTAAACAAAAGAACCCAT

CTTGGTCCAATGTACAAATCCGCAATCATCTAAAGAATACGGCAACGAGCTTAGGA

AGCACGAACTTGTATGGAAGCGGACTTGTCAATGCAGAAGCTGCAACTCGTTAA

(SEQ ID NO:18)

As shown above, the DNA sequence of SEQ ID NO:18 comprises a nucleotide sequence encoding a signal peptide (shown above in non-underlined, lowercase letters), a nucleotide sequence encoding a propeptide (shown above in underlined, lower-case letters), and a nucleotide sequence encoding mature GG36 polypeptide (shown above in uppercase letters).

In the protein sequence of GG36 provided below, the signal peptide sequence is shown in lower-case letters, the propeptide sequence is shown in lower-case, underlined letters, and the GG36 mature protease sequence is shown in uppercase letters:

vrskklwivastallisvafsssiasaaeeeakekyligfneqeavsefveqveandevailseeeeveiellhefetipvlsvelspedvdaleldpaisy
ieedaevttmAQSVPWGISRVQAPAAHNRGLTGSGVKVAVLDTGISTHPDLNIRGGASFVPGEPSTQD
GNGHGTHVAGTIAALNNSIGVLGVAPSAELYAVKVLGASGSGSVSSIAQGLEWAGNNGMHVA
NLSLGSPSPSATLEQAVNSATSRGVLVVAASGNSGAGSISYPARYANAMAVGATDQNNNRASFS
QYGAGLDIVAPGVNVQSTYPGSTYASLNGTSMATPHVAGAAALVKQKNPSWSNVQIRNHLKN
TATSLGSTNLYGSGLVNAEAATR (SEQ ID NO:19)

The amino acid sequence of the mature variant protease referred to herein as the PX3 polypeptide and having amino acid substitutions N76D+S87R+G118R+S128L+P129Q+S130A+S188D+N248R relative to SEQ ID NO:1 (using BPN' numbering determined by alignment of the PX3 polypeptide sequence with the BPN' polypeptide sequence shown in SEQ ID NO:2) is:

AQSVPWGISRVQAPAAHNRGLTGSGVKVAVLDTGISTHPDLNIRGGASFVPGEPSTQDGNGHGT
HVAGTIAAL**D**NSIGVLGVAP**R**AELYAVKVLGASGSGSVSSIAQGLEWAGNN**R**MHVANLSLG**LQ**
**A**PSATLEQAVNSATSRGVLVVAASGNSGAGSISYPARYANAMAVGATDQNNNRA**D**FSQYGAG
LDIVAPGVNVQSTYPGSTYASLNGTSMATPHVAGAAALVKQKNPSWSNVQIR**R**HLKNTATSLG
STNLYGSGLVNAEAATR (SEQ ID NO:6).

The amino acid sequence of the mature variant protease (variant subtilisin) referred to herein as the PX4 polypeptide and comprising amino acid substitutions N76D/S87R/G118R/S128L/P129Q/S130A relative to SEQ ID NO:1 (using BPN' numbering determined by alignment of the PX4 polypeptide sequence with the BPN' polypeptide sequence shown in SEQ ID NO:2) is:

AQSVPWGISRVQAPAAHNRGLTGSGVKVAVLDTGISTHPDLNIRGGASFVPGEPSTQDGNGHGT
HVAGTIAAL**D**NSIGVLGVAP**R**AELYAVKVLGASGSGSVSSIAQGLEWAGNN**R**MHVANLSLG**LQ**
**A**PSATLEQAVNSATSRGVLVVAASGNSGAGSISYPARYANAMAVGATDQNNNRASFSQYGAGL
DIVAPGVNVQSTYPGSTYASLNGTSMATPHVAGAAALVKQKNPSWSNVQIR**N**HLKNTATSLGS
TNLYGSGLVNAEAATR (SEQ ID NO:7).

The amino acid sequence of the mature variant protease (variant subtilisin) referred to herein as the PX5 polypeptide and comprising amino acid substitutions N76D/S87R/G118R/S128L/P129Q/S130A/S188D/V244R relative to SEQ ID NO:1 (using BPN' numbering determined by alignment of the PX3 polypeptide sequence with the BPN' polypeptide sequence shown in SEQ ID NO:2) is:

AQSVPWGISRVQAPAAHNRGLTGSGVKVAVLDTGISTHPDLNIRGGASFVPGEPSTQDG
NGHGTHVAGTIAAL**D**NSIGVLGVAP**R**AELYAVKVLGASGSGSVSSIAQGLEWAGNN**R**MHVAN
LSLG**LQA**PSATLEQAVNSATSRGVLVVAASGNSGAGSISYPARYANAMAVGATDQNNNRA**D**FS
QYGAGLDIVAPGVNVQSTYPGSTYASLNGTSMATPHVAGAAALVKQKNPSWSN**R**QIRNHLKN
TATSLGSTNLYGSGLVNAEAATR (SEQ ID NO:8).

A nucleic acid sequence encoding the mature variant protease (variant subtilisin) PX3 is:

GCGCAATCAGTGCCATGGGGAATTAGCCGTGTGCAAGCCCCAGCTGCCCATAACCGTGGAT
TGACAGGTTCTGGTGTAAAAGTTGCTGTCCTCGATACAGGTATTTCCACTCATCCAGACTTA
AATATTCGTGGTGGCGCTAGCTTTGTACCAGGGGAACCATCCACTCAAGATGGGAATGGGC
ATGGCACGCATGTGGCCGGGACGATTGCTGCTTTAGACAATTCGATTGGCGTTCTTGGCGTA
GCGCCGAGAGCGGAACTATACGCTGTTAAAGTATTAGGGGCGAGCGGTTCAGGTTCGGTCA
GCTCGATTGCCCAAGGATTGGAATGGGCAGGGAACAATCGTATGCACGTTGCTAATTTGAG
TTTAGGACTGCAGGCACCAAGTGCCACACTTGAGCAAGCTGTTAATAGCGCGACTTCTAGA
GGCGTTCTTGTTGTAGCGGCATCTGGAAATTCAGGTGCAGGCTCAATCAGCTATCCGGCCCG
TTATGCGAACGCAATGGCAGTCGGAGCTACTGACCAAAACAACAACCGCGCCGATTTTTCA
CAGTATGGCGCAGGGCTTGACATTGTCGCACCAGGTGTAAACGTGCAGAGCACATACCCAG
GTTCAACGTATGCCAGCTTAAACGGTACATCGATGGCTACTCCTCATGTTGCAGGTGCAGCA
GCCCTTGTTAAACAAAAGAACCCATCTTGGTCCAATGTACAAATCCGCAGACATCTAAAGA
ATACGGCAACGAGCTTAGGAAGCACGAACTTGTATGGAAGCGGACTTGTCAATGCAGAAGC
TGCAACTCGTTAA (SEQ ID NO:9)

A nucleic acid sequence encoding the mature variant protease (variant subtilisin) PX4 is:

GCGCAATCAGTGCCATGGGGAATTAGCCGTGTGCAAGCCCCAGCTGCCCATAACCGTGGAT
TGACAGGTTCTGGTGTAAAAGTTGCTGTCCTCGATACAGGTATTTCCACTCATCCAGACTTA
AATATTCGTGGTGGCGCTAGCTTTGTACCAGGGGAACCATCCACTCAAGATGGGAATGGGC
ATGGCACGCATGTGGCCGGGACGATTGCTGCTTTAGACAATTCGATTGGCGTTCTTGGCGTA
GCGCCGAGAGCGGAACTATACGCTGTTAAAGTATTAGGGGCGAGCGGTTCAGGTTCGGTCA
GCTCGATTGCCCAAGGATTGGAATGGGCAGGGAACAATCGTATGCACGTTGCTAATTTGAG
TTTAGGACTGCAGGCACCAAGTGCCACACTTGAGCAAGCTGTTAATAGCGCGACTTCTAGA
GGCGTTCTTGTTGTAGCGGCATCTGGAAATTCAGGTGCAGGCTCAATCAGCTATCCGGCCCG
TTATGCGAACGCAATGGCAGTCGGAGCTACTGACCAAAACAACAACCGCGCCAGCTTTTCA
CAGTATGGCGCAGGGCTTGACATTGTCGCACCAGGTGTAAACGTGCAGAGCACATACCCAG
GTTCAACGTATGCCAGCTTAAACGGTACATCGATGGCTACTCCTCATGTTGCAGGTGCAGCA
GCCCTTGTTAAACAAAAGAACCCATCTTGGTCCAATGTACAAATCCGCAATCATCTAAAGAA
TACGGCAACGAGCTTAGGAAGCACGAACTTGTATGGAAGCGGACTTGTCAATGCAGAAGCT
GCAACTCGTTAA (SEQ ID NO:10)

A nucleic acid sequence encoding the mature variant protease (variant subtilisin) PX5 is:

GCGCAATCAGTGCCATGGGGAATTAGCCGTGTGCAAGCCCCAGCTGCCCATAACCGTGGAT
TGACAGGTTCTGGTGTAAAAGTTGCTGTCCTCGATACAGGTATTTCCACTCATCCAGACTTA
AATATTCGTGGTGGCGCTAGCTTTGTACCAGGGGAACCATCCACTCAAGATGGGAATGGGC
ATGGCACGCATGTGGCCGGGACGATTGCTGCTTTAGACAATTCGATTGGCGTTCTTGGCGTA

GCGCCGAGAGCGGAACTATACGCTGTTAAAGTATTAGGGGCGAGCGGTTCAGGTTCGGTCA

GCTCGATTGCCCAAGGATTGGAATGGGCAGGGAACAATCGTATGCACGTTGCTAATTTGAG

TTTAGGACTGCAGGCACCAAGTGCCACACTTGAGCAAGCTGTTAATAGCGCGACTTCTAGA

GGCGTTCTTGTTGTAGCGGCATCTGGAAATTCAGGTGCAGGCTCAATCAGCTATCCGGCCCG

TTATGCGAACGCAATGGCAGTCGGAGCTACTGACCAAAACAACAACCGCGCCGATTTTTCA

CAGTATGGCGCAGGGCTTGACATTGTCGCACCAGGTGTAAACGTGCAGAGCACATACCCAG

GTTCAACGTATGCCAGCTTAAACGGTACATCGATGGCTACTCCTCATGTTGCAGGTGCAGCA

GCCCTTGTTAAACAAAAGAACCCATCTTGGTCCAATCGTCAAATCCGCAATCATCTAAAGAA

TACGGCAACGAGCTTAGGAAGCACGAACTTGTATGGAAGCGGACTTGTCAATGCAGAAGCT

GCAACTCGTTAA (SEQ ID NO:11)

Exemplary site-directed mutagenesis procedures well known in the art include, but are not limited to, e.g., the QuikChange® Multi Site-Directed Mutagenesis method embodied in the QuikChange® Multi Site-Directed Mutagenesis Kit (QCMS; Agilent Technologies - Stratagene, La Jolla, CA), which allows for site-directed mutagenesis of plasmid DNA at up to five different sites simultaneously. Additional exemplary known mutagenesis procedures are described elsewhere herein. Nucleic acids of the invention encoding variant proteases of the invention as described herein, including e.g., the PX3, PX4, and PX5 variant protease polypeptides, can also be readily made from, e.g., the GG36 protease-encoding nucleotide sequence set forth in SEQ ID NO:18 by one skilled in the art using well-known gene synthesis methods and/or fusion PCR methods (see, e.g., U.S. Pat. Application Pub. No. 2006/0252155).

Nucleic acids encoding the variant proteases can also be made by chemical synthesis using, e.g., the classical phosphoramidite method (see, e.g., Beaucage et al., Tetrahedron Letters 22:1859-69 (1981)) or the method described by Matthes et al., EMBO J. 3:801-05 (1984), e.g., as is typically practiced in automated synthesis methods. Alternatively, nucleic acids encoding the variant proteases can be ordered from a variety of commercial sources, such as from The Midland Certified Reagent Company (Midland, Texas) (worldwide website address at oligos.com), the Great American Gene Company (worldwide website address genco.com), Operon Technologies, Inc. (Alameda, Calif.) (now Qiagen, see worldwide website at qiagen.com), or DNA2.0 (Menlo Park, CA). Other techniques for synthesizing nucleic acids and related principles are described in, e.g., Itakura et al., Annu. Rev. Biochem. 53:323 (1984) and Itakura et al., Science 198:1056 (1984).

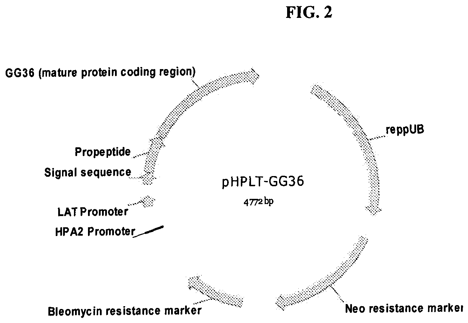

In one aspect, for example, if gene synthesis is used to create the variant protease-encoding nucleic acids, such nucleic acids can be designed with flanking restriction sites such as, e.g., BglII, which can be used to clone the variant-encoding nucleic acids into an expression plasmid (e.g., *B. subtilis* expression plasmid) also digested with BglII, such as the pHPLT-GG36 *B. subtilis* expression plasmid described herein. This exemplary pHPLT *B. subtilis* expression vector contains the *B. licheniformis* LAT promoter (Plat), HPA2 promoter, and additional elements from pUB110 (see, e.g., McKenzie et al., Plasmid, 15:93-103 (1986)), including a replicase gene (reppUB), a neomycin resistance gene (neo), and a bleomycin resistance marker (bleo) (see also Figure 4 of U.S. Patent No. 6,566,112). The pHPLT-GG36 plasmid map is provided at Figure 2, and the GG36 expression cassette sequence is provided below. For the QuikChange® Multi Site-Directed Mutagenesis Kit (QCMS Kit) or fusion PCR methods described herein, the pHPLT-GG36 plasmid comprising the *B. lentus* GG36-encoding nucleic acid can be used as the DNA template for making the variant proteases of the invention. In an exemplary format, nucleotide primers containing the desired mutations are annealed to the GG36-encoding nucleic acid in the pHPLT-GG36 plasmid and extended with a DNA polymerase as described in the Stratagene QCMS product manual and in U.S. Patent Application Pub. No. 2006/0252155 for fusion PCR. Table 1-1 provides exemplary nucleotide sequences of the primers that can be used for site-directed mutagenesis.

| Table 1-1. Exemplary Primers Used for QuikChange® Multi Site-Directed Mutagenesis Method | |
|---|---|
| Primer Sequence | Primer Name |
| CGGGACGATTGCTGCTTTA**GAC**AATTCGATTGGCGTTC (SEQ ID NO:12) | N76D |
| GGCGTTCTTGGCGTAGCGCCG**AAC**GCGGAACTATACG (SEQ ID NO:13) | S87N |
| CCAAGGATTGGAATGGGCAGGGAACAAT**CGT**ATGCACGTTG (SEQ ID NO:14) | G118R |
| TAATTTGAGTTTAGGA**CTGCAGGCA**CCAAGTGCCACACTTGAGC (SEQ ID NO:15) | S128L,P129Q,S130A |

48

(continued)

| Table 1-1. Exemplary Primers Used for QuikChange® Multi Site-Directed Mutagenesis Method | |
|---|---|
| Primer Sequence | Primer Name |
| CCAAAACAACAACCGCGCC**GAT**TTTTCACAGTATGGCGC (SEQ ID NO:16) | S188D |
| ATCTTGGTCCAAT**CGT**CAAATCCGCAATCATCTAAAGAATACGGC (SEQ ID NO:17) | V244R |

The incorporation of mutations in each variant protease can be carried out in multiple rounds till the final variant protease is obtained. Rolling circle amplification (GE Healthcare, Piscataway, NJ) can be used as described by the manufacturer to amplify the mutant plasmids contained in the QCMS or fusion PCR ligation reactions before transformation in *B. subtilis* (GE Healthcare, Piscataway, NJ) cells.

Competent *B. subtilis* cells (phenotype: Δ*aprE*, Δ*nprE*, *oppA*, Δ*spoIIE*, *degUHy32*, Δ*amyE*::(*xylR,pxylA-comK*)) can be transformed with the variant plasmids or 1 μL of the rolling circle amplification reaction to obtain protease positive transformants using procedures known in the art (see, e.g., WO 02/14490). The bacteria can be made competent by the induction of the *comK* gene under control of a xylose inducible promoter (see, e.g., Hahn et al., Mol. Microbiol. 21:763-775 (1996). Variant protease positive clones can be selected on skim milk/agar plates, isolated, sequenced and variant protease protein produced in shaker flask cultures to generate significant quantities of enzyme samples for characterization.

## EXAMPLE 2

### Production of Variant Proteases in *Bacillus subtilis*

The variant proteases (e.g., variant subtilisins) were produced by growing the *B. subtilis* transformants overnight at 37°C in 10ml TSB (tryptone and soy-based broth) medium. A 250 μl aliquot of the overnight culture was transferred into 25ml of a MOPS based defined medium in a 100ml shake flask and grown at 37°C for 68 hours. The defined medium was made essentially as known in the art (see Neidhardt et al., J Bacteriol. 119: 736-747, 1974), except that $NH_4Cl$, $FeSO_4$, and $CaCl_2$ were left out of the base medium, 3 mM $K_2HPO_4$ was used, and the base medium was supplemented with 60 mM urea, 75 g/L glucose, and 1% soytone. Also the micronutrients were made up as a 100X stock containing in one liter, 400 mg $FeSO_4.7H_2O$, 100 mg $MnSO_4 .H_2O$, 100 mg $ZnSO_4.7H_2O$, 50 mg $CuCl_2.2H_2O$, 100 mg $CoCl_2.6H_2O$, 100 mg $NaMoO_4.2H_2O$, 100 mg $Na_2B_4O_7.10H_2O$, 10 ml of 1M $CaCl_2$, and 10 ml of 0.5 M sodium citrate. The proteases of interest were isolated from the culture medium.

## EXAMPLE 3

### Analytical Methods to Determine the Purity of Variant Protease Samples

In this Example, methods used to determine the purity of the recombinant variant proteases (e.g., variant subtilisins) obtained from *B. subtilis* cultures are described. Variant proteases were considered pure when a single band or peak was found by gel electrophoresis and high performance liquid chromatography (HPLC), respectively.

Polyacrylamide gel electrophoresis (PAGE) in the presence of sodium dodecyl sulphate (SDS) was conducted as known in the art (Laemmli, Nature, 227:680-685, 1970). However, prior to denaturation of the protein samples (e.g., 10 min in SDS-containing sample buffer at 100°C), inactivation of the protease activity was required in order to prevent auto-degradation. Protease inactivation was accomplished by incubating the protein sample with 1 mM PMSF for 30 min at room temperature or by precipitation of the protein with 8% trichloroacetic acid (TCA) for 30 min on ice. Protein samples were subjected to native PAGE carried out at pH 7.45. The gel buffer consisted of 20 mM histidine and 50 mM 3-(N-morpholino)propanesulfonic acid (MOPS), and the 5% polyacrylamide gels had a acrylamide:bisacrylamide ratio of 20:1. Protein samples were loaded on top of slab gels and electrophoresed towards the cathode. The same histidine/MOPS buffer was used as electrophoresis (tank) buffer, but adjusted to pH 6.3. Following electrophoresis (∼1-2 hr at 350 V), the gel was soaked in 8% acetic acid to fix the proteins in the gel and subsequently stained with Coomassie Brilliant Blue R250 and destained as known in the art, to locate protein bands on the gel.

The protease sample purity was also confirmed by HPLC analysis using a MonoS cation exchange column followed by a TSK 2000 gel filtration column. The former was run in a 10 mM sodium phosphate buffer pH 5.5 with elution of the bound protease using a linear gradient of 10-300 mM sodium phosphate, pH 5.5. The gel filtration column was run in 0.25M sodium acetate pH 5.5. Protein elution profiles were monitored at 280 nm to locate the protease of interest and to determine the percent purity of the sample.

## EXAMPLE 4

### Determination of the Variant Protease Concentration

In this Example, methods used to determine the concentrations of the variant proteases are described. In some experiments, extinction (absorbance) measurements were made at 280 nm using the calculated extinction coefficient ($\varepsilon$), and active site titrations were used to determine the protein concentration in a purified protease solution, as described below.

The extinction coefficient at 280 nm was calculated from the number of tryptophans (Trp, $\varepsilon$ = 5,600 M$^{-1}$.cm$^{-1}$) and tyrosines (Tyr, $\varepsilon$ = 1,330 M$^{-1}$.cm$^{-1}$) per enzyme molecule. For the PB92 protease, the molar extinction coefficient was 26,100 M$^{-1}$.cm$^{-1}$ (3 Trp + 7 Tyr residues) equivalent to $\varepsilon$ 1%, measured at 280 nm = 9.7 (M$_r$ = 26,729 Da). In the case of mutant proteases with an altered number of tryptophan and/or tyrosine residues, corrections were made accordingly.

An estimation of the concentration of active enzyme molecules was obtained by active site titration. Since the widely used method of acylation by N-transcinnamoylimidazole (Bender et al., J. Amer. Chem. Soc., 88:5890-5931, 1966) proved not to work satisfactorily for the PB92 protease, a method using the irreversible inhibitor PMSF was developed instead. In this method, a protease solution with an estimated enzyme concentration (from the 280 nm absorption) was mixed with 0.25, 0.50, 0.75, 1.00 and 1.25 equivalents of PMSF, respectively, and allowed to react for one hour at room temperature in 10 mM sodium phosphate pH 6.5. Residual protease activity was measured spectrophotometrically using succinyl-L-alanyl-L-alanyl-L-prolyl-L-phenyl-alanyl-para-nitroanilide (suc-AAPF-pNA) as a substrate. For these studies, the purity (and hence concentration) of PMSF was determined by NMR-spectroscopy and stock solutions of PMSF were prepared in isopropanol. The active site titration results were found to be in agreement with the protein concentration results from the purity check using the HPLC method.

## EXAMPLE 5

### Assays

In this Example, methods to assess the stability and cleaning performance of a variant protease of the invention are described.

### AAPF Hydrolysis Method

The thermostability of the serine variant protease was determined by assaying protease activity using the AAPF assay after incubation of variant proteases at 68°C for 1 hour. Under the conditions of the assay, the residual activity of the reference protease (e.g., wild-type GG36 = GCI-P036) was about 50%. The equipment used was: F-bottom MTPs (Costar No. 9017), Biomek FX and/or Biomek FXp Robot (Beckman Coulter), Spectramax Plus 384 MTP Reader (Molecular Devices), iEMS Incubator/Shaker (1 mm amplitude) (Thermo/Labsystems), Sealing tape (Nunc No. 236366), and ice bath. Glycine buffer was prepared by dissolving 3.75g glycine (Merck No. 1.04201.1000) in 960 mL water. 1 ml of 5% Tween®-80 (Sigma No. P-8074) and 10 ml of a stock solution of 1000 mM CaCl$_2$ (MerckNo. 1.02382.1000) (29.4 g dissolved to 200 ml) was added to this solution. The pH was adjusted to 10.5 with 4N NaOH and the volume brought up to 1000 ml. Final concentrations of glycine, CaCl$_2$ and TWEEN®-80 were: 50 mM, 10 mM and 0.005% respectively. The incubators were set at 68°C (for incubation) and at 25°C (for the AAPF assay). 90 $\mu$l and 190 $\mu$l glycine buffer was added to the empty dilution and incubation plates respectively. 10 $\mu$l of supernatant was then added to the dilution plate, followed by addition of 10$\mu$l from the dilution plate to the incubation plate. Then 10 $\mu$l of mixture from the incubation plate was added to a pre-warmed plate containing suc-AAPF-pNA substrate. The suc-AAPF-pNA plate was read in MTP Reader at 410 nm (t = 0 measurement). The incubation plate was covered with tape and incubated for 1 hour at 68°C and 400 rpm. At the end of the incubation, the plate was removed from the incubator and cooled down on ice for at least 5 minutes. 10 $\mu$l of mixture from the incubation plate was transferred to the plate containing suc-AAPF-pNA substrate and the plate read at 410 nm (t=60 measurement). Percent residual activity was calculated as:

$$\text{\% residual activity: (mOD.min-1 at t=60) / (mOD.min-1 at t=0) x 100}$$

### Surfactant and Chelant Stability Assays

LAS and LAS/EDTA stability was measured after incubation of the test protease in the presence of LAS and LAS/EDTA respectively, as a function of residual activity determined using the AAPF assay.

**LAS Stability Method**

**Reagents:**

Dodecylbenzenesulfonate, Sodium salt (=LAS): Sigma D-2525
TWEEN®-80: Sigma P-8074
TRIS buffer (free acid): Sigma T-1378); 6.35 g is dissolved in about 960 ml water; pH is adjusted to 8.2 with 4N HCl. Final concentration of TRIS is 52.5 mM.
LAS stock solution: Prepare a 10.5 % LAS solution in MQ water (=10.5 g per 100 ml MQ)
TRIS buffer-100 mM / pH 8.6 (100mM Tris/0.005% Tween®-80)
TRIS-Ca buffer, pH 8.6 (100mM TRIS/10mM $CaCl_2$/0.005% Tween®-80)

**Hardware:**

Flat bottom MTPs (Costar No. 9017)
Biomek FX
ASYS Multipipettor
Spectramax MTP Reader
iEMS Incubator/Shaker
Innova 4330 Incubator/Shaker
Biohit multichannel pipette
BMG Thermostar Shaker

A 0.063% LAS solution was prepared in 52.5 mM TRIS buffer pH 8.2. The suc-AAPF-pNA working solution was prepared by adding 1 ml of 100 mg/ml suc-AAPF-pNA stock solution (in DMSO) to 100 ml (100 mM) TRIS buffer, pH 8.6. To dilute the supernatants, flat-bottomed plates were filled with dilution buffer and an aliquot of the supernatant was added and mixed well. The dilution ratio depended on the concentration of the protease controls in the growth plates (AAPF activity). The desired protein concentration was 80 ppm.

Ten $\mu$l of the diluted supernatant were added to 190 $\mu$l 0.063% LAS buffer/well. The MTP was covered with tape, shaken for a few seconds and placed in an incubator (Innova 4230) at 25°C or 35°C for ASP or 45°C for BPN' or GG36, for 60 minutes at 200 rpm agitation. The initial activity ($t$=10 minutes) was determined after 10 minutes of incubation by transferring 10 $\mu$l of the mixture in each well to a fresh MTP containing 190$\mu$l suc-AAPF-pNA work solution. These solutions were mixed well and the AAPF activity was measured using a MTP Reader (20 readings in 5 minutes and 25°C).

The final activity ($t$=60 minutes) was determined by removing another 10 $\mu$l of solution from the incubating plate after 60 minutes of incubation. The AAPF activity was then determined as described above. The stability of the samples was determined by calculating the ration of the residual and initial AAPF activity as follows:

$$\text{Residual Activity (\%)} = (t\text{-}60 \text{ value})*100 \, / \, (t\text{-}10 \text{ value}).$$

**LAS/EDTA Stability Method**

The stability of a variant protease in the presence of a representative anionic surfactant (LAS=linear alkylbenzene sulfonate, sodium dodecylbenzenesulfonate-DOBS) and di-sodium EDTA was measured after incubation under defined conditions and the residual activity was determined using the AAPF assay. The reagents used were dodecyllbenzene sulfonate, sodium salt (DOBS, Sigma No. D-2525), TWEEN®-80 (Sigma No. P-8074), di-sodium EDTA (Siegfried Handel No. 164599-02), HEPES (Sigma No. H-7523), unstress buffer: 50 mM HEPES (11.9 g/l) + 0.005% TWEEN®-80, pH 8.0, Stress buffer: 50 mM HEPES (11.9 g/l), 0.1% (w/v) DOBS (1 g/l), 10 mM EDTA (3.36 g/l), pH 8.0, reference protease and variant protease culture supernatants, containing 200 - 400 $\mu$g/ml protein. The equipment used was V- or U-bottom MTP as dilution plates (Greiner 651101 and 650161 respectively), F-bottom MTP (Corning 9017) for unstress and LAS/EDTA buffer as well as for suc-AAPF-pNA plates, Biomek FX (Beckman Coulter), Spectramax Plus 384 MTP Reader (Molecular Devices), iEMS Incubator/Shaker (1 mm amplitude) from Thermo Electron Corporation, sealing tape: Nunc (236366).

The iEMS incubator/shaker (Thermo/Labsystems) was set at 29°C. Culture supernatants were diluted into plates containing unstress buffer to a concentration of ∼ 25 ppm (master dilution plate). 20 μl of sample from the master dilution plate was added to plates containing 180 μl unstress buffer to give a final incubation concentration of 2.5 ppm. The contents were mixed and kept at room temperature and an AAPF assay was performed on this plate. 20 μl of sample from the master dilution plate was also added to plates containing 180 μl stress buffer (50 mM HEPES (11.9 g/l), 0.1% (w/v) DOBS (1 g/l), 10 mM EDTA (3.36 g/l), pH 8.0). The solutions were mixed and immediately placed in 29°C iEMS shaker for 30 min at 400 rpm. Following 30 minutes of incubation, a AAPF assay was performed on the stress plate. (OD = optical density absorbance). The stability of the samples was determined by calculating the ration of the residual and initial AAPF activity as follows: Residual Activity (%) = (mOD.min-1 stressed)*100 / (mOD. min-1 unstressed).

### EXAMPLE 6

### Wash Performance Test for Variant PX3

In this Example, methods suitable for evaluation of dishwashing and fabric cleaning performance of the variant protease PX3 (e.g., variant subtilisin) and the GCI-P038 reference subtilisin in commercially available dish and laundry detergents are described.

### Dishwashing Performance

The methods used to measure the dishwashing performance of the variant proteases of the invention (e.g., subtilisin variants) and the GCI-P038 reference subtilisin in commercially available dish detergents are described herein.

The performance of the variant proteases was tested under various automatic dishwashing conditions. The compositions of the dish detergents are shown in Tables 6-1 and 6-2. These detergents are commercially available from wfk Testmaterials (www.testgewebe.de/en/products/detergents/) and are referred to by their wfk Testmaterials designations. These detergents were obtained from the source without the presence of enzymes, to permit analysis of the variant proteases.

| Table 6-1. Phosphate-Free Detergent IEC-60436 WFK Type B (pH=10.4 in 3g/l) | |
|---|---|
| **Component** | **Wt %** |
| Sodium citrate dehydrate | 30.0 |
| Maleic acid/ Acrylic acid copolymer sodium Salt | 12.0 |
| Sodium perborate monohydrate | 5.0 |
| TAED | 2.0 |
| Sodium disilicate: Protil A (Cognis) | 25.0 |
| Linear fatty alcohol ethoxylate | 2.0 |
| Sodium carbonate anhydrous | add to 100 |

| Table 6-2. Phosphate-Containing Detergent: IEC-60436 WFK Type C (pH=10.5 in 3 g/l) | |
|---|---|
| **Component** | **Wt %** |
| Sodium tripolyphosphate | 23.0 |
| Sodium citrate dehydrate | 22.3 |
| Maleic acid/ Acrylic acid copolymer sodium salt | 4.0 |
| Sodium perborate monohydrate | 6.0 |
| TAED | 2.0 |

(continued)

| Table 6-2. Phosphate-Containing Detergent: IEC-60436 WFK Type C (pH=10.5 in 3 g/l) | |
|---|---|
| **Component** | **Wt %** |
| Sodium disilicate: Protil A (Cognis) | 5.0 |
| Linear fatty alcohol ethoxylate | 2.0 |
| Sodium carbonate anhydrous | add to 100 |

The protocols for preparation of each of the stain types (egg yolk, minced meat and egg, and egg with milk) are provided below. Before the individual soil types were applied to the test dishes, the dishes were thoroughly washed. This was particularly necessary, as residues of certain persistent stains may still be present on the dishes from previous tests. New dishes were also subjected to three thorough washes before being used for the first time in a test.

**Preparation of Egg Yolk Stains on Stainless Steel**

The stainless steel sheets (10 x 15 cm; brushed on one side) used in these experiments were thoroughly washed at 95°C in a laboratory dishwasher with a high-alkalinity commercial detergent (e.g., ECOLAB® detergent; Henkel) to provide sheets that were clean and grease-free. These sheets were deburred prior to their first use. The sheets were dried for 30 minutes at 80°C in a thermal cabinet before being soiled with egg yolk. The surfaces to be brushed were not touched prior to soiling. Also, no water stains or fluff on the surfaces were permitted. The cooled sheets were weighed before soiling.

The egg yolks were prepared by separating the yolks of approximately 10-11 eggs (200 g of egg yolk) from the whites. The yolks were stirred with a fork in a glass beaker to homogenize the yolk suspension. The yolks were then strained (approximately 0.5 mm mesh) to remove coarse particles and any egg shell fragments.

A flat brush (2.5") was used to apply $2.0 \pm 0.1$ g egg yolk suspension as uniformly as possible over an area of 140 $cm^2$ on the brushed sides of each of the stainless steel sheets, leaving an approximately 1 cm wide unsoiled rim (adhesive tape was used if needed). The soiled sheets were dried horizontally (to prevent formation of droplets on the edges of the sheets), at room temperature for 4 hours (max. 24 hr).

To denature the egg yolk proteins, the sheets were immersed for 30 seconds in boiling, demineralized water (using a holding device if necessary). Then the sheets were dried again for 30 min at 80°C. After drying and cooling, the sheets were weighed. After weighing, the sheets were left for at least 24 hrs (20°C, 40-60% relatively humidity) before submitting them to the wash test. In order to meet the testing requirements, only sheets with $1000 \pm 100$ mg/140 $cm^2$ (egg yolk after denaturation) were used in the testing. After the wash tests were conducted, the sheets were dried for 30 min at 80°C in the thermal cabinet and weighed again after cooling. The percent cleaning performance was determined by dividing the mg of egg yolk released upon washing by the mg of denatured egg yolk applied and multiplying by 100.

**Preparation of Minced Meat and Egg Stains on Porcelain Plates**

For these experiments, dessert plates (Arzberg, 19 cm diameter, white, glazed porcelain) conforming to EN 50242, form 1495, No. 0219, were used. A total of 225 g lean pork and beef (50:50 ratio) was finely chopped and maintained cool. The mixture was twice run through a mincer. Temperatures above 35°C were avoided. The 225 g of the minced meat was then mixed with 75 g of egg (white and yolk mixed together). The preparation was then frozen for up to three months at -18°C, prior to use. If pork was not available, 100% beef was used, as these are interchangeable.

The minced meat and egg mixture (300 g) was brought to room temperature and mixed with 80 ml demineralized water. The mixture was then homogenized for 2 min using a kitchen hand blender. A fork was used to spread 3 g of the minced meat/egg/water mixture on each white porcelain plate, leaving an approximately 2 cm wide unsoiled margin around the rim. The amount applied was $11.8 \pm 0.5$ mg/$cm^2$. The plates were dried for 2 hours at 120°C in a preheated thermal cabinet. As soon as the plates were cooled, they were ready for use.

After conducting the dishwashing tests, the plates were sprayed with ninhydrin solution (prepared to 1% in ethanol) for better identification of the minced meat protein residues. To promote the color reaction, the plates were heated for 10 min at 80°C in the thermal cabinet. Evaluation of the washing performance was done by visually inspecting the color reactions of the minced meat residue with reference to the IKW photographic catalogue (IKW - The German Cosmetic, Toiletry, Perfumery and Detergent Association).

**Preparation of Egg/Milk Stains on Stainless Steel**

The stainless steel sheets (10 x 15 cm; brushed on one side) used in these experiments were thoroughly washed at 95°C in a laboratory dishwasher with a high-alkalinity commercial detergent to remove grease and clean the sheets. The sheets were polished dry with a cellulose cloth. The surfaces to be brushed were not touched prior to soiling. Also, no water stains or fluff on the surfaces were permitted. Before soiling, the sheets were placed in a thermal cabinet at 80°C, for 30 min. The cooled sheets were weighed before soiling.

The egg yolks and whites of whole raw eggs (3-4 eggs; approximately160 g/egg) were placed in a bowl and beaten with an egg whisk. Then 50 ml semi-skimmed milk (1.5% fat, ultra-high-temperature, homogenized) were added to the mixture. The milk and egg were mixed without generating froth. A flat brush was used to uniformly distribute 1.0 ± 0.1 g of the egg/milk mixture on the brushed side of the stainless steel sheets, using a balance to check the distribution. A margin of approximately 1.0 cm was left around the short sides of the sheets. The soiled sheets were dried horizontally (to prevent formation of droplets on the edges of the sheets), at room temperature for 4 hours (max. 24 hr).

The sheets were then immersed for 30 seconds in boiling, demineralized water (using a holding device if necessary). Then the sheets were dried again for 30 min at 80°C. After drying and cooling the sheets were weighed. After weighing the sheets were left to sit for at least 24 hours (20°C, 40-60% relatively humidity) before submitting them to the wash test. In order to meet the testing requirements, only sheets with 190 ± 10 mg egg yolk/milk were used.

After the wash tests were conducted, the sheets were dried for 30 min at 80°C, in the thermal cabinet, and weighed again after cooling. The percentage cleaning performance was determined by dividing the mg of egg/milk released upon washing by the mg of egg/milk applied and multiplying by 100.

**Washing Equipment and Conditions**

The washing tests were performed in an automatic dishwasher (Miele model G690SC), equipped with soiled dishes and stainless steel sheets, prepared as described above. A defined amount of the detergent was used. The temperature tested was 50°C. The water hardness was 21° GH (German hardness).

As described above, after washing the plates soiled with minced meat were visually assessed using a photo rating scale of 0 to 10, wherein "0" designated a completely dirty plate and "10" designated a clean plate. These values correspond to the stain or soil removal (SR) capability of the enzyme-containing detergent.

The washed stainless steel plates soiled with egg yolk or egg yolk/milk were analyzed gravimetrically to determine the amount of residual stain after washing. The subtilisin variant PX3 and the GCI-P038 reference subtilisin were tested at a level of between 0 and 30 mg/active protein per wash.

The results for various dishwashing tests are provided below in Tables 6-3 to 6-6. In each of these experiments, different concentrations of active protease per wash were used. The wash performance of the GCI-P038 reference subtilisin was assigned a value of "100," while the wash performance of the variants was compared to this value. For example, if the GCI-P038 reference subtilisin had a result of 45% stain removal and a variant had a result of 52% stain removal, the result for the subtilisin variant shown as a performance index (PI) would be 52/45 x 100 = 116. Thus, in both detergents tested, the subtilisin variant PX3 was more effective than or as effective as the GCI-P038 reference subtilisin in removing proteinaceous stains in dishwashing applications.

| Table 6-3. Phosphate-Containing Detergent, 50°C, 21°GH, dosed at 0.05% active protein | | | |
|---|---|---|---|
| Enzyme | PI Egg Yolk | PI Minced Meat | PI Egg Yolk/Milk |
| Reference GCI-P038 | 100 | 100 | 100 |
| Variant PX3 | 111 | 157 | 147 |

| Table 6-4. Phosphate-Containing Detergent 50°C, 21°GH, dosed at 0.15% active protein | | | |
|---|---|---|---|
| Enzyme | PI Egg Yolk | PI Minced Meat | PI Egg Yolk/Milk |
| Reference GCI-P038 | 100 | 100 | 100 |

(continued)

| Table 6-4. Phosphate-Containing Detergent 50°C, 21°GH, dosed at 0.15% active protein | | | |
|---|---|---|---|
| Enzyme | PI Egg Yolk | PI Minced Meat | PI Egg Yolk/Milk |
| Variant PX3 | 135 | 100* | 113 |
| * Under these specified conditions soil removal by GCI-P038was 100%. | | | |

| Table 6-5. Phosphate-Free Detergent 50°C, 21°GH, dosed at 0.05% active protein | | | |
|---|---|---|---|
| Enzyme | PI Egg Yolk | PI Minced Meat | PI Egg Yolk/Milk |
| Reference GCI-P038 | 100 | 100 | 100 |
| Variant PX3 | 124 | 150 | 117 |

| Table 6-6. Phosphate-Free Detergent 50°C, 21°GH, dosed at 0.15% active protein | | | |
|---|---|---|---|
| Enzyme | PI Egg Yolk | PI Minced Meat | PI Egg Yolk/Milk |
| Reference GCI-P038 | 100 | 100 | 100 |
| Variant PX3 | 115 | 118 | 103 |

## EXAMPLE 7

### Wash Performance Tests

In this Example, methods suitable for evaluation of fabric cleaning performance of the variant proteases of the invention in commercially available laundry detergents.

| Table 7-1. Laundry Washing Conditions | | | | | | | |
|---|---|---|---|---|---|---|---|
| Region | Form | Dose | Detergent* | Buffer | Gpg | pH | T (°C) |
| Laundry (heavy duty liquid and granular) | | | | | | | |
| NA | HDL | 0.78 g/l | P&G TIDE® 2X | 5 mM HEPES | 6 | 8.0 | 20 |
| WE | HDL | 5.0 g/L | Henkel PERSIL™ | 5 mM HEPES | 12 | 8.2 | 40 |
| WE | HDG | 8.0 g/L | P&G ARIELI™ | 2 mM $Na_2 CO_3$ | 12 | 10.5 | 40 |
| JPN | HDG | 0.7 g/L | P&G TIDE® | 2 mM $Na_2 CO_3$ | 6 | 10.0 | 20 |
| NA | HDG | 1.0 g/L | P&G TIDE® | 2 mM $Na_2 CO_3$ | 6 | 10.0 | 20 |
| * Abbreviations: Procter & Gamble (P&G); Reckitt Benckiser (RB); heavy duty liquid detergent (HDL); heavy duty granular detergent (HDG). | | | | | | | |

### Blood Milk Ink (BMI) Microswatch Assay

The stain removal performance of the variant proteases can be determined on a microtiter plate (MTP) scale in commercially available detergents. Samples of a reference protease enzyme (e.g., reference subtilisin) and the variant proteases are obtained from filtered culture broth of cultures grown in MTP plates for 3 days at 37 °C/ 300 rpm/ 90% relative humidity. The equipment to be used includes: 96 well polystyrene plates (Costar No. 9017 medium binding flat bottom), Biomek FX and/or Biomek FXp (Beckman Coulter), Spectramax Plus 384 (Molecular Devices), iEMS Incubator/Shaker with 1 mm amplitude (Thermo Electron Corporation) and sealing tape (Nunc No. 236366). The reagents to be used include: 5 mM HEPES, pH 8.0 or 5 mM MOPS, pH 7 buffer, 3:1 Ca: Mg for medium water hardness (CaCl$_2$: MgCl$_2$•6H2O); 15000 grains per gallon (gpg) stock diluted to 6 gpg, two BMI (blood/milk/ink) swatches per plate: EMPA-116 BMI cotton swatches processed by CFT: pre-rinsed and punched two swatches per well, and heat inactivated TIDE® 2X off-the-shelf detergent in which lack of protease activity can be confirmed. In this assay, the proteases hydrolyze the substrate and liberate pigment and insoluble particles from the substrate.

| Table 7-2. Working Detergent Solutions | | | | | |
|---|---|---|---|---|---|
| Detergent | Temp (°C) | Detergent g/L | pH | Buffer | Grains per gallon (gpg) |
| TIDE® 2X | 16 | 0.98 | 8 | 5mM HEPES | 6 |
| TIDE® 2X | 32 | 0.98 | 8 | 5mM HEPES | 6 |
| TIDE® 2X | 16 | 0.98 | 7 | 5mM MOPS | 6 |

The incubator is set at the desired temperature (16°C or 32°C). First, 10 μL samples from the master dilution plate of ~10 ppm enzyme are added to BMI 2-swatch plates with 190 μL working detergent solutions listed above. The volume is adjusted to give final concentration of 0.5 ppm for variant proteases in the assay plates. The plates are then immediately transferred to iEMS incubators and incubated for 30 minutes with 1400 rpm shaking at given temperature. Following incubation, 100 μL of supernatant is transferred into a new 96-well plate and the absorbance is measured in MTP Reader at 405nm and/or 600nm. Control wells containing one or two microswatches and detergent without the addition of protease samples are also included in the test. The absorbance measurement at 405 nm provides a higher value and tracks pigment removal, while the absorbance measurement at 600 nm tracks turbidity and cleaning.

**Calculation of the Stain Removal Activity:**

The absorbance value obtained is corrected for the blank value (substrate without enzyme), providing a measure of hydrolytic activity. For each sample (e.g., variant protease enzyme or reference protease enzyme), the performance index (PI) is calculated. The performance index compares the performance of the variant protease (actual value) and the standard reference protease enzyme (theoretical value) at the same protein concentration. In addition, the theoretical values can be calculated, using the parameters of the Langmuir equation of the standard enzyme. A performance index (PI) that is greater than 1 (PI>1) identifies a better variant protease as compared to the reference protease enzyme (e.g., wild-type subtilisin); that is, a performance index greater than 1 identifies a variant protease having an enhanced or improved ability to lessen, eliminate, or remove a stain of interest compared to the ability of the reference protease enzyme to lessen or remove the same or a similar stain. A PI of 1 (PI=1) identifies a variant protease that performs the same or approximately the same as the reference protease enzyme (i.e., the variant protease has the same or about the same ability to lessen, eliminate, or remove a stain of interest compared to the ability of the reference protease enzyme to lessen or remove the same or a similar stain). A PI that is less than 1 (PI<1) identifies a variant protease that performs less well than the reference protease enzyme (i.e., the variant protease has a decreased or lower ability to lessen, eliminate, or remove a stain of interest compared to the ability of the reference protease enzyme to lessen or remove the same or a similar stain). Thus, the PI value identifies variant proteases having an improved or enhanced ability to lessen, eliminate, or remove a stain of interest compared to the ability of the reference protease to lessen, eliminate, or remove the same or a similar stain) under certain circumstances or conditions, as well as variant proteases that are less desirable for use under certain circumstances, such as, e.g., those variant proteases having a decreased ability to lessen, eliminate or remove a stain on interest compared to the ability of a reference protease to remove the same or a similar stain).

**EXAMPLE 8**

**Cleaning Performance of PX3, PX4, and PX5 Variant Proteases**

**BMI Microswatch Assay in Laundry Applications**

Results of experiments conducted to determine cleaning performance in laundry applications of variants PX4 and PX5 are shown in Table 8-1. The test detergents used were heat inactivated commercially obtained TIDE® 2X Free (P&G; "NA HDL") TIDE Free (P&G; "NA HDD"). Cleaning performance of BMI stained microswatches was tested using 0.2 ppm of the variants at 25°C for 30 minutes with 1400 rpm shaking in a volume of 200 μL. Functionality of GCI-P036 variants were quantified as a performance index (Pi), which is the ratio of performance of a variant to a parent GCI-P036 protein. Subtilisin FNA (BPN'-Y217L) and GCI-P036-S87N-G118V-S128L-P129Q-S130A proteins were also tested.

| Table 8-1. P$_i$ Values of GCI-P036 Variants Tested for Stain Removal Performance on BMI Microswatches in NA HDL and NA HDD Detergents | | | |
|---|---|---|---|
| UV # | Variants Based on GCI-P036 | NA HDL pH 8 | NA HDD pH 10 |
| GCI-P036 | - | 1.00 | 1.00 |
| PX4 | N76D/S87R/G118R/S128L/ P129Q/S130A | 0.97 | 1.07 |
| PX5 | S87R/ N76D/G118R/ S128L/ P129Q/ S130A/ S188D/V244R | 0.87 | 0.98 |
| GCI-P036-variant | S87N-G118V-S128L-P129Q-S130A | 1.22 | 1.14 |
| FNA | BPN' Y217L | 1.29 | 0.66 |

## EXAMPLE 9

## Cleaning Performance of PX3, PX4, and PX5 Variant Proteases

## Baked Egg Yolk Microswatch Assay in Dishwashing Applications

The stain removal performance of the variant proteases (e.g., variant subtilisins) was determined on a microtiter plate (MTP) scale in commercially available detergents (CALGONIT® (Reckitt-Benckiser) and CASCADE® (P&G)). Samples for testing the variant subtilisins were obtained from filtered culture broth of cultures grown in MTP plates for 3 days at 37 °C/ 300 rpm/ 90% relative humidity. The equipment used included: a Biomek FX Robot (Beckman Coulter), a SpectraMAX microtiter plate (MTP) Reader (type 340; Molecular Devices), an iEMS incubator/shaker (Thermo/Labsystems); F-bottom MTPs (Costar type 9017) for reading of reaction plates after incubation and V-bottom MTPs (Greiner 651101) for pre-dilution of supernatant. CS-38 microswatches (egg-yolk with pigment, aged by heating), obtained from CFT Vlaardingen were used as substrate. Two swatches were used per well. Automatic dishwashing (ADW) tablets from Calgonit 5in1 were used to prepare the detergent solution. To inactivate the protease activity present in the tablets, a 21g tablet was dissolved in Milli-Q water heated in a water bath to a temperature of 60°C. The solution was cooled to room temperature and the volume of water adjusted to 700 mL. The solution was further diluted with water to achieve a final concentration of 3 g/l. Water hardness was adjusted to 21°GH by adding 1.46 ml of the Ca/Mg-mixture (Ca/Mg mixture ((3:1), 1.92 M CaCl$_2$ =282.3 g/L CaCl$_2$.2H$_2$0; 0.64 M MgCl$_2$ = 130.1 g/L MgCl$_2$.6H$_2$O), 15000gpg). The enzyme samples were prediluted in 10 mM NaCl, 0.1 mM CaCl$_2$, 0.005% TWEEN®-80 solution and tested at appropriate concentrations.

The incubator was set at the desired temperature of 40°C or 50°C, and 72 μl of dilution buffer was added to the empty V-bottom plate (= dilution plate) followed by 8 μl supernatant. Then, 9 μl from the dilution plate was added to plates containing the microswatches incubated in 171 μl detergent solution. The microswatch plate (with detergent and enzyme) was covered with tape and placed in the incubator/shaker for 30 minutes at 1400 rpm. Following incubation, 75 μl of the reaction mixture was transferred to an empty F-bottom plate and the absorbance was read in a MTP Reader at 405 nm after debubbling with a hair dryer. Blank controls, containing one or two microswatches and detergent without the addition of the reference subtilisin containing samples were also included in the test.

| Table 9-1. Automatic Dishwashing Conditions | | | | | | | |
|---|---|---|---|---|---|---|---|
| Region | Form | Dose | Detergent* | Buffer | Gpg | pH | T (°C) |
| WE | ADW | 3.0 g/L | RB CALGONIT™ | 2 mM Na$_2$CO$_3$ | 21 | 10.0 | 40 |
| NA | ADW | 3.0 g/L | P&G CASCADE™ | 2 mM Na$_2$CO$_3$ | 9 | 10.0 | 40 |
| * Abbreviations: Procter & Gamble (P&G); and Reckitt Benckiser (RB). | | | | | | | |

**Calculation of the Stain Removal Activity:**

The absorbance value obtained was corrected for the blank value (substrate without enzyme), providing a measure of hydrolytic activity. For each sample (e.g., variant protease enzyme or reference protease enzyme), the performance index (PI) was calculated. The performance index compares the performance of the variant protease (actual value) and the reference protease enzyme (theoretical value) at the same protein concentration. In addition, the theoretical values can be calculated, using the parameters of the Langmuir equation of the standard enzyme. A performance index (PI) that is greater than 1 (PI>1) identifies a better variant protease as compared to the reference protease enzyme (e.g., wild-type subtilisin); that is, a performance index greater than 1 identifies a variant protease having an enhanced or improved ability to lessen, eliminate, or remove a stain of interest (e.g., egg yolk with pigment, aged by heating) compared to the ability of the reference protease enzyme to lessen or remove the same or a similar stain. A PI of 1 (PI=1) identifies a variant protease that performs the same or approximately the same as the reference protease enzyme (i.e., the variant protease has the same or about the same ability to lessen, eliminate, or remove a stain of interest compared to the ability of the reference protease enzyme to lessen or remove the same or a similar stain). A PI that is less than 1 (PI<1) identifies a variant protease that performs less well than the reference protease enzyme (i.e., the variant protease has a decreased or lower ability to lessen, eliminate, or remove a stain of interest compared to the ability of the reference protease enzyme to lessen or remove the same or a similar stain). Thus, the PI value identifies variant proteases having an improved or enhanced ability to lessen, eliminate, or remove a stain of interest compared to the ability of the reference protease to lessen, eliminate, or remove the same or a similar stain) under certain circumstances or conditions, as well as variant proteases that are less desirable for use under certain circumstances, such as, e.g., those variant proteases having a decreased ability to lessen, eliminate or remove a stain on interest compared to the ability of a reference protease to remove the same or a similar stain).

The cleaning performance of variant proteases (e.g., variant subtilisins) was determined using a microswatch assay (CS-38 swatches, e.g., stained with egg yolk with pigment, aged by heating). The LAS/EDTA stability, thermostability and protein determination by TCA precipitation for the variant(s) was also determined using methods described above. Results are shown in Table 9-2.

**Table 9-2. P$_I$ Values of GCI-P036 Variants Compared to GCI-P038 Tested for Stain Removal Performance on CS38 Microswatches, LAS/EDTA Stability, Protein Determination and Thermostability ("ND" indicates not determined)**

| Varian t | Variants based on GCI-P036 | CALGONIT ® 5 in 1 40°C | CALGONIT ® 5 in 1 50°C | CASCADE ® Complete 50°C | LAS/EDT A Stability | TC A | Thermo - stability |
|---|---|---|---|---|---|---|---|
| PX3 | S87R/G118R/S128L/P129Q / S130A/N76D/S188D/N248 R | 0.93 | 1.20 | 1.24 | 1.54 | 1.39 | 0.7 |
| PX4 | N76D/S87R/G118R/S128L/ P129Q/S130A | 1.20 | 1.44 | 1.35 | ND | ND | ND |
| PX5 | N76D/S87R/G118R/S128L/ P129Q/S130A/S188D/V244 R | 0.97 | 1.16 | 1.17 | ND | ND | ND |

As shown in Table 9-2, the variant proteases exhibited enhanced or improved cleaning performance compared to the reference protease GG36 at 50°C in various detergent compositions. Specifically, the PX3 variant exhibited cleaning performance (PI values)) greater than the GG36 protease in CALGONIT® 5 in 1 and CASCADE® Complete at 50°C and nearly the same cleaning performance as CALGONIT® 5 in 1 at 40°C. The PX4 variant exhibited cleaning performance (PI values) significantly greater than the GG36 protease in CALGONIT® 5 in 1 and CASCADE® Complete at 50°C and in CALGONIT® 5 in 1 at 40°C. Thus, it is expected that a lower amount of PX4 protease would be needed to achieve the same cleaning performance as the GG36 protease. Further, it is noteworthy the PX4 variant demonstrated substantially better cleaning performance than the GG36 protease even at a lower temperature. The PX5 variant protease exhibited cleaning performance (PI values) greater than the GG36 protease in CALGONIT® 5 in 1 and CASCADE® Complete at 50°C and nearly the same cleaning performance as CALGONIT® 5 in 1 at 40°C.

## EXAMPLE 10

**Liquid Laundry Detergent Compositions**

This Example provides various formulations for liquid laundry detergent compositions of the invention. Exemplary liquid laundry detergent formulations to which at least one variant protease of the invention may be added are provided in Tables 10-1 and 10-2 below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 10-1.**

| Compound | Formulations | | | | |
|---|---|---|---|---|---|
| | I | II | III | IV | V |
| LAS | 24.0 | 32.0 | 6.0 | 3.0 | 6.0 |
| $NaC_{16}$-$C_{17}HSAS$ | - | - | - | 5.0 | - |
| $C_{12}$-$C_{15}AE_{1.8}S$ | - | - | 8.0 | 7.0 | 5.0 |
| $C_8$-$C_{10}$ propyl dimethyl amine | 2.0 | 2.0 | 2.0 | 2.0 | 1.0 |
| $C_{12}$-$C_{14}$ alkyl dimethyl amine oxide | - | - | - | - | 2.0 |
| $C_{12}$-$C_{15}$ AS | - | - | 17.0 | - | 8.0 |
| CFAA | - | 5.0 | 4.0 | 4.0 | 3.0 |
| $C_{12}$-$C_{14}$ Fatty alcohol ethoxylate | 12.0 | 6.0 | 1.0 | 1.0 | 1.0 |
| $C_{12}$-$C_{18}$ Fatty acid | 3.0 | - | 4.0 | 2.0 | 3.0 |
| Citric acid (anhydrous) | 4.5 | 5.0 | 3.0 | 2.0 | 1.0 |
| DETPMP | - | - | 1.0 | 1.0 | 0.5 |
| Monoethanolamine | 5.0 | 5.0 | 5.0 | 5.0 | 2.0 |
| Sodium hydroxide | - | - | 2.5 | 1.0 | 1.5 |
| 1 N HCl aqueous solution | #1 | #1 | - | - | - |
| Propanediol | 12.7 | 14.5 | 13.1 | 10. | 8.0 |
| Ethanol | 1.8 | 2.4 | 4.7 | 5.4 | 1.0 |
| DTPA | 0.5 | 0.4 | 0.3 | 0.4 | 0.5 |
| Pectin Lyase | - | - | - | 0.005 | - |
| Amylase | 0.001 | 0.002 | - | - | - |
| Cellulase | - | - | 0.0002 | - | 0.0001 |
| Lipase | 0.1 | - | 0.1 | - | 0.1 |
| NprE (optional) | 0.05 | 0.3 | - | 0.5 | 0.2 |

(continued)

| Compound | Formulations | | | | |
|---|---|---|---|---|---|
| | I | II | III | IV | V |
| PMN | - | - | 0.08 | - | - |
| Protease A (optional) | - | - | - | - | 0.1 |
| Aldose Oxidase | - | - | 0.3 | - | 0.003 |
| ZnCl2 | 0.1 | 0.05 | 0.05 | 0.05 | 0.02 |
| Ca formate | 0.05 | 0.07 | 0.05 | 0.06 | 0.07 |
| DETBCHD | - | - | 0.02 | 0.01 | - |
| SRP1 | 0.5 | 0.5 | - | 0.3 | 0.3 |
| Boric acid | - | - | - | - | 2.4 |
| Sodium xylene sulfonate | - | - | 3.0 | - | - |
| Sodium cumene sulfonate | - | - | - | 0.3 | 0.5 |
| DC 3225C | 1.0 | 1.0 | 1.0 | 1.0 | 1.0 |
| 2-butyl-octanol | 0.03 | 0.04 | 0.04 | 0.03 | 0.03 |
| Brightener 1 | 0.12 | 0.10 | 0.18 | 0.08 | 0.10 |
| Balance to 100% perfume / dye and/or water | | | | | |
| #1: Add 1N HCl aq. solution to adjust the neat pH of the formula in the range from about 3 to about 5. | | | | | |

The pH of formulations (I)-(II) is about 5 to about 7, and the pH of formulations (III)-(V) is about 7.5 to about 8.5.

**Table 10-2.**

| Compound | Formulations | | | | | |
|---|---|---|---|---|---|---|
| | I | II | III | IV | V | VI |
| LAS | 11.5 | 11.5 | 9.0 | - | 4.0 | - |
| $C_{12}$-$C_{15}AE_{2.85}S$ | - | - | 3.0 | 18.0 | - | 16.0 |
| $C_{14}$-$C_{15}E_{2.5}S$ | 11.5 | 11.5 | 3.0 | - | 16.0 | - |
| $C_{12}$-$C_{13}E_9$ | - | - | 3.0 | 2.0 | 2.0 | 1.0 |
| $C_{12}$-$C_{13}E_7$ | 3.2 | 3.2 | - | | - | - |
| CFAA | - | - | - | 5.0 | - | 3.0 |
| TPKFA | 2.0 | 2.0 | - | 2.0 | 0.5 | 2.0 |
| Citric Acid (Anhydrous) | 3.2 | 3.2 | 0.5 | 1.2 | 2.0 | 1.2 |
| Ca formate | 0.1 | 0.1 | 0.06 | 0.1 | - | - |
| Na formate | 0.5 | 0.5 | 0.06 | 0.1 | 0.05 | 0.05 |
| ZnCl2 | 0.1 | 0.05 | 0.06 | 0.03 | 0.05 | 0.05 |
| Na Culmene Sulfonate | 4.0 | 4.0 | 1.0 | 3.0 | 1.2 | - |
| Borate | 0.6 | 0.6 | 1.5 | - | - | - |
| Na Hydroxide | 6.0 | 6.0 | 2.0 | 3.5 | 4.0 | 3.0 |
| Ethanol | 2.0 | 2.0 | 1.0 | 4.0 | 4.0 | 3.0 |
| 1,2 Propanediol | 3.0 | 3.0 | 2.0 | 8.0 | 8.0 | 5.0 |
| Monoethanolamine | 3.0 | 3.0 | 1.5 | 1.0 | 2.5 | 1.0 |

(continued)

| Compound | Formulations | | | | | |
|---|---|---|---|---|---|---|
| | I | II | III | IV | V | VI |
| TEPAE | 2.0 | 2.0 | - | 1.0 | 1.0 | 1.0 |
| nprE (optional) | 0.03 | 0.05 | - | 0.03 | - | 0.02 |
| PMN | - | - | 0.01 | - | 0.08 | - |
| Protease A (optional) | - | - | 0.01 | - | - | - |
| Lipase | - | - | - | 0.002 | - | - |
| Amylase | - | - | - | - | 0.002 | - |
| Cellulase | - | - | | - | - | 0.0001 |
| Pectin Lyase | 0.005 | 0.005 | - | | - | - |
| Aldose Oxidase | 0.05 | - | - | 0.05 | - | 0.02 |
| Galactose oxidase | - | 0.04 | | | | |
| PAAC | 0.03 | 0.03 | 0.02 | - | - | - |
| DETBCHD | - | - | - | 0.02 | 0.01 | - |
| SRP 1 | 0.2 | 0.2 | - | 0.1 | - | - |
| DTPA | - | - | - | 0.3 | - | - |
| PVNO | - | - | - | 0.3 | - | 0.2 |
| Brightener 1 | 0.2 | 0.2 | 0.07 | 0.1 | - | - |
| Silicone antifoam | 0.04 | 0.04 | 0.02 | 0.1 | 0.1 | 0.1 |
| Balance to 100% perfume/dye and/or water | | | | | | |

## EXAMPLE 11

### Liquid Hand Dishwashing Detergent Compositions

This Example provides various formulations for liquid hand dishwashing detergent compositions of the invention. Exemplary liquid hand dishwashing detergent formulations to which at least one variant protease of the invention may be added are provided below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 11-1.**

| Compound | Formulations | | | | | |
|---|---|---|---|---|---|---|
| | I | II | III | IV | V | VI |
| $C_{12}$-$C_{15}AE_{1.8}S$ | 30.0 | 28.0 | 25.0 | - | 15.0 | 10.0 |
| LAS | - | - | - | 5.0 | 15.0 | 12.0 |
| Paraffin Sulfonate | - | - | - | 20.0 | - | - |
| $C_{10}$-$C_{18}$ Alkyl Dimethyl Amine Oxide | 5.0 | 3.0 | 7.0 | - | - | - |
| Betaine | 3.0 | - | 1.0 | 3.0 | 1.0 | - |
| $C_{12}$ poly-OH fatty acid amide | - | - | - | 3.0 | - | 1.0 |
| $C_{14}$ poly-OH fatty acid amide | - | 1.5 | - | - | - | - |

(continued)

| Compound | Formulations | | | | | |
|---|---|---|---|---|---|---|
| | I | II | III | IV | V | VI |
| $C_{11}E_9$ | 2.0 | - | 4.0 | - | - | 20.0 |
| DTPA | - | - | - | - | 0.2 | - |
| Tri-sodium Citrate dihydrate | 0.25 | - | - | 0.7 | - | - |
| Diamine | 1.0 | 5.0 | 7.0 | 1.0 | 5.0 | 7.0 |
| $MgCl_2$ | 0.25 | - | - | 1.0 | - | - |
| nprE (optional) | 0.02 | 0.01 | - | 0.01 | - | 0.05 |
| PMN | - | - | 0.03 | - | 0.02 | - |
| Protease A (optional) | - | 0.01 | - | - | - | - |
| Amylase | 0.001 | - | - | 0.002 | - | 0.001 |
| Aldose Oxidase | 0.03 | - | 0.02 | - | 0.05 | - |
| Sodium Cumene Sulphonate | - | - | - | 2.0 | 1.5 | 3.0 |
| PAAC | 0.01 | 0.01 | 0.02 | - | - | - |
| DETBCHD | - | - | - | 0.01 | 0.02 | 0.01 |
| Balance to 100% perfume / dye and/or water | | | | | | |

The pH of formulations (I)-(VI) is about 8 to about 11.

## EXAMPLE 12

### Liquid Automatic Dishwashing Detergent Compositions

This Example provides various formulations for liquid automatic dishwashing detergent compositions of the invention. Exemplary liquid automatic dishwashing detergent formulations to which at least one variant protease of the invention may be added are provided below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

Table 12-1.

| Compound | Formulations | | | | |
|---|---|---|---|---|---|
| | I | II | III | IV | V |
| STPP | 16 | 16 | 18 | 16 | 16 |
| Potassium Sulfate | - | 10 | 8 | - | 10 |
| 1,2 propanediol | 6.0 | 0.5 | 2.0 | 6.0 | 0.5 |
| Boric Acid | | - | - | 4.0 | 3.0 |
| $CaCl_2$ dihydrate | 0.04 | 0.04 | 0.04 | 0.04 | 0.04 |
| Nonionic | 0.5 | 0.5 | 0.5 | 0.5 | 0.5 |
| nprE (optional) | 0.1 | 0.03 | - | 0.03 | - |
| PMN | - | - | 0.05 | - | 0.06 |
| Protease B (optional) | - | - | - | 0.01 | - |
| Amylase | 0.02 | - | 0.02 | 0.02 | - |

(continued)

| Compound | Formulations | | | | |
|---|---|---|---|---|---|
| | I | II | III | IV | V |
| Aldose Oxidase | - | 0.15 | 0.02 | - | 0.01 |
| Galactose Oxidase | - | - | 0.01 | - | 0.01 |
| PAAC | 0.01 | - | - | 0.01 | - |
| DETBCHD | - | 0.01 | - | - | 0.01 |
| Balance to 100% perfume / dye and/or water | | | | | |

## EXAMPLE 13

### Granular and/or Tablet Laundry Compositions

This Example provides various formulations for granular and/or tablet laundry detergent compositions of the invention. Exemplary laundry compositions of present invention, which may be in the form of granules or tablet, to which at least one variant protease of the invention may be added are provided below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 13-1.**

| Compound | Formulations | | | | |
|---|---|---|---|---|---|
| | I | II | III | IV | V |
| $C_{14}$-$C_{15}$AS or TAS | 8.0 | 5.0 | 3.0 | 3.0 | 3.0 |
| LAS | 8.0 | - | 8.0 | - | 7.0 |
| $C_{12}$-$C_{15}$AE$_3$S | 0.5 | 2.0 | 1.0 | - | - |
| $C_{12}$-$C_{15}$E$_5$ or E$_3$ | 2.0 | - | 5.0 | 2.0 | 2.0 |
| QAS | - | - | - | 1.0 | 1.0 |
| Zeolite A | 20.0 | 18.0 | 11.0 | - | 10.0 |
| SKS-6 (dry add) | - | - | 9.0 | - | - |
| MA/AA | 2.0 | 2.0 | 2.0 | - | - |
| AA | - | - | - | - | 4.0 |
| 3Na Citrate 2H$_2$O | - | 2.0 | - | - | - |
| Citric Acid (Anhydrous) | 2.0 | - | 1.5 | 2.0 | - |
| DTPA | 0.2 | 0.2 | - | - | - |
| EDDS | - | - | 0.5 | 0.1 | - |
| HEDP | - | - | 0.2 | 0.1 | - |
| PB1 | 3.0 | 4.8 | - | - | 4.0 |
| Percarbonate | - | - | 3.8 | 5.2 | - |
| NOBS | 1.9 | - | - | - | - |
| NACA OBS | - | - | 2.0 | - | - |
| TAED | 0.5 | 2.0 | 2.0 | 5.0 | 1.00 |
| BB1 | 0.06 | - | 0.34 | - | 0.14 |

(continued)

| Compound | Formulations | | | | |
|---|---|---|---|---|---|
| | I | II | III | IV | V |
| BB2 | - | 0.14 | - | 0.20 | - |
| Anhydrous Na Carbonate | 15.0 | 18.0 | - | 15.0 | 15.0 |
| Sulfate | 5.0 | 12.0 | 5.0 | 17.0 | 3.0 |
| Silicate | - | 1.0 | - | - | 8.0 |
| nprE (optional) | 0.03 | - | 0.1 | 0.06 | - |
| PMN | - | 0.05 | - | - | 0.1 |
| Protease B (optional) | - | 0.01 | | - | - |
| Protease C (optional) | - | - | - | 0.01 | - |
| Lipase | - | 0.008 | - | - | - |
| Amylase | 0.001 | | - | - | 0.001 |
| Cellulase | - | 0.0014 | - | - | - |
| Pectin Lyase | 0.001 | 0.001 | 0.001 | 0.001 | 0.001 |
| Aldose Oxidase | 0.03 | - | 0.05 | - | - |
| PAAC | - | 0.01 | - | - | 0.05 |
| Balance to 100% Moisture and/or Minors* | | | | | |
| * Perfume, dye, brightener / SRP1 / Na carboxymethylcellulose / photobleach / MgSO$_4$ / PVPVI / suds suppressor / high molecular polyethylene glycol (PEG) / clay. | | | | | |

## EXAMPLE 14

### High-Density Automatic Dishwashing Detergent Compositions

This Example provides various formulations for high-density automatic dishwashing detergent compositions of the invention. Exemplary high-density automatic dishwashing detergent compositions to which at least one variant protease of the invention may be added are provided below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 14-1.**

| Compound | Formulations | | | | | |
|---|---|---|---|---|---|---|
| | I | II | III | IV | V | VI |
| STPP | - | 45.0 | 45.0 | - | - | 40.0 |
| 3Na Citrate 2H$_2$O | 17.0 | - | - | 50.0 | 40.2 | - |
| Na Carbonate | 17.5 | 14.0 | 20.0 | - | 8.0 | 33.6 |
| Bicarbonate | | - | - | 26.0 | - | - |
| Silicate | 15.0 | 15.0 | 8.0 | - | 25.0 | 3.6 |
| Metasilicate | 2.5 | 4.5 | 4.5 | - | - | - |
| PB1 | - | - | 4.5 | - | - | - |
| PB4 | - | - | - | 5.0 | - | - |
| Percarbonate | - | - | - | - | - | 4.8 |

(continued)

| Compound | Formulations | | | | | |
|---|---|---|---|---|---|---|
| | I | II | III | IV | V | VI |
| BB1 | - | 0.1 | 0.1 | - | 0.5 | - |
| BB2 | 0.2 | 0.05 | - | 0.1 | - | 0.6 |
| Nonionic | 2.0 | 1.5 | 1.5 | 3.0 | 1.9 | 5.9 |
| HEDP | 1.0 | - | - | - | - | - |
| DETPMP | 0.6 | - | - | - | - | - |
| PAAC | 0.03 | 0.05 | 0.02 | - | - | - |
| Paraffin | 0.5 | 0.4 | 0.4 | 0.6 | - | - |
| nprE (optional) | 0.072 | 0.053 | - | 0.026 | - | 0.01 |
| PMN | - | - | 0.053 | - | 0.059 | - |
| Protease B (optional) | - | - | - | - | - | 0.01 |
| Amylase | 0.012 | - | 0.012 | - | 0.021 | 0.006 |
| Lipase | - | 0.001 | - | 0.005 | - | - |
| Pectin Lyase | 0.001 | 0.001 | 0.001 | | - | - |
| Aldose Oxidase | 0.05 | 0.05 | 0.03 | 0.01 | 0.02 | 0.01 |
| BTA | 0.3 | 0.2 | 0.2 | 0.3 | 0.3 | 0.3 |
| Polycarboxylate | 6.0 | | - | - | 4.0 | 0.9 |
| Perfume | 0.2 | 0.1 | 0.1 | 0.2 | 0.2 | 0.2 |
| Balance to 100% Moisture and/or Minors* | | | | | | |
| *Brightener / dye / SRP1 / Na carboxymethylcellulose/ photobleach / $MgSO_4$ / PVPVI/ suds suppressor /high molecular PEG/clay. | | | | | | |

The pH of formulations (I)-(VI) is from about 9.6 to about 11.3.

## EXAMPLE 15

### Tablet Detergent Compositions

This Example provides various tablet detergent formulations. The following exemplary tablet detergent compositions of the present invention to which at least one variant protease of the invention may be added are prepared by compression of a granular dishwashing detergent composition at a pressure of 13KN/cm$^2$ using a standard 12 head rotary press. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 15-1.**

| Compound | Formulations | | | | | | | |
|---|---|---|---|---|---|---|---|---|
| | I | II | III | IV | V | VI | VII | VIII |
| STPP | - | 48.8 | 44.7 | 38.2 | - | 42.4 | 46.1 | 46.0 |
| 3Na Citrate 2H$_2$O | 20.0 | - | - | - | 35.9 | - | - | - |
| Na Carbonate | 20.0 | 5.0 | 14.0 | 15.4 | 8.0 | 23.0 | 20.0 | - |
| Silicate | 15.0 | 14.8 | 15.0 | 12.6 | 23.4 | 2.9 | 4.3 | 4.2 |

(continued)

| Compound | Formulations | | | | | | | |
|---|---|---|---|---|---|---|---|---|
| | I | II | III | IV | V | VI | VII | VIII |
| Lipase | 0.001 | - | 0.01 | - | 0.02 | - | - | - |
| Protease B (optional) | 0.01 | - | - | - | - | - | - | - |
| Protease C (optional) | - | - | - | - | - | 0.01 | - | - |
| nprE (optional) | 0.01 | 0.08 | - | 0.04 | - | 0.023 | - | 0.05 |
| PMN | - | - | 0.05 | - | 0.052 | - | 0.023 | - |
| Amylase | 0.012 | 0.012 | 0.012 | - | 0.015 | - | 0.017 | 0.002 |
| Pectin Lyase | 0.005 | - | - | 0.002 | - | - | - | - |
| Aldose Oxidase | - | 0.03 | - | 0.02 | 0.02 | - | 0.03 | - |
| PB1 | - | - | 3.8 | - | 7.8 | - | - | 4.5 |
| Percarbonate | 6.0 | - | - | 6.0 | - | 5.0 | - | - |
| BB1 | 0.2 | - | 0.5 | - | 0.3 | 0.2 | - | - |
| BB2 | - | 0.2 | - | 0.5 | - | - | 0.1 | 0.2 |
| Nonionic | 1.5 | 2.0 | 2.0 | 2.2 | 1.0 | 4.2 | 4.0 | 6.5 |
| PAAC | 0.01 | 0.01 | 0.02 | - | - | - | - | - |
| DETBCHD | - | - | - | 0.02 | 0.02 | - | - | - |
| TAED | - | - | - | - | - | 2.1 | - | 1.6 |
| HEDP | 1.0 | - | - | 0.9 | - | 0.4 | 0.2 | - |
| DETPMP | 0.7 | - | - | - | - | - | - | - |
| Paraffin | 0.4 | 0.5 | 0.5 | 0.5 | - | - | 0.5 | - |
| BTA | 0.2 | 0.3 | 0.3 | 0.3 | 0.3 | 0.3 | 0.3 | - |
| Polycarboxylate | 4.0 | - | - | - | 4.9 | 0.6 | 0.8 | - |
| PEG 400-30,000 | - | - | - | - | - | 2.0 | - | 2.0 |
| Glycerol | - | - | - | - | - | 0.4 | - | 0.5 |
| Perfume | - | - | - | 0.05 | 0.2 | 0.2 | 0.2 | 0.2 |
| Balance to 100% Moisture and/or Minors* | | | | | | | | |
| *Brightener / SRP1 / Na carboxymethylcellulose/ photobleach / $MgSO_4$ / PVPVI/ suds suppressor /high molecular PEG/clay. | | | | | | | | |

The pH of formulations (I)-(VII) is from about 10 to about 11.5; pH of formulation (VIII) is from about 8 to about 10. The tablet weight of formulations (I)-(VIII) is from about 20 grams to about 30 grams.

## EXAMPLE 16

### Liquid Hard Surface Cleaning Detergents

This Example provides various formulations for liquid hard surface cleaning detergent compositions of the invention. Exemplary liquid hard surface cleaning detergent compositions to which at least one variant protease of the invention may be added are provided below. At least one variant protease of the invention may be included in each such formulation at a concentration of from about 0.0001 to about 10 percent by weight of the resultant formulation (composition). The formulation may comprise an alternative concentration of the variant protease(s), as determined by the formulator, based on the formulator's needs.

**Table 16-1.**

| Compound | Formulations | | | | | | |
|---|---|---|---|---|---|---|---|
| | I | II | III | IV | V | VI | VII |
| $C_9$-$C_{11}E_5$ | 2.4 | 1.9 | 2.5 | 2.5 | 2.5 | 2.4 | 2.5 |
| $C_{12}$-$C_{14}E_5$ | 3.6 | 2.9 | 2.5 | 2.5 | 2.5 | 3.6 | 2.5 |
| $C_7$-$C_9E_6$ | - | - | - | - | 8.0 | - | - |
| $C_{12}$-$C_{14}E_{21}$ | 1.0 | 0.8 | 4.0 | 2.0 | 2.0 | 1.0 | 2.0 |
| LAS | - | - | - | 0.8 | 0.8 | - | 0.8 |
| Sodium culmene sulfonate | 1.5 | 2.6 | - | 1.5 | 1.5 | 1.5 | 1.5 |
| Isachem ® AS | 0.6 | 0.6 | - | - | - | 0.6 | - |
| $Na_2CO_3$ | 0.6 | 0.13 | 0.6 | 0.1 | 0.2 | 0.6 | 0.2 |
| 3Na Citrate $2H_2O$ | 0.5 | 0.56 | 0.5 | 0.6 | 0.75 | 0.5 | 0.75 |
| NaOH | 0.3 | 0.33 | 0.3 | 0.3 | 0.5 | 0.3 | 0.5 |
| Fatty Acid | 0.6 | 0.13 | 0.6 | 0.1 | 0.4 | 0.6 | 0.4 |
| 2-butyl octanol | 0.3 | 0.3 | - | 0.3 | 0.3 | 0.3 | 0.3 |
| PEG DME-2000® | 0.4 | - | 0.3 | 0.35 | 0.5 | - | - |
| PVP | 0.3 | 0.4 | 0.6 | 0.3 | 0.5 | - | - |
| MME PEG (2000) ® | - | - | - | - | - | 0.5 | 0.5 |
| Jeffamine ® ED-2001 | - | 0.4 | - | - | 0.5 | - | - |
| PAAC | - | - | - | 0.03 | 0.03 | 0.03 | - |
| DETBCHD | 0.03 | 0.05 | 0.05 | - | - | - | - |
| nprE (optional) | 0.07 | - | 0.08 | 0.03 | - | 0.01 | 0.04 |
| PMN | - | 0.05 | - | - | 0.06 | - | - |
| Protease B (optional) | - | - | - | - | - | 0.01 | - |
| Amylase | 0.12 | 0.01 | 0.01 | - | 0.02 | - | 0.01 |
| Lipase | - | 0.001 | - | 0.005 | - | 0.005 | - |
| Pectin Lyase | 0.001 | - | 0.001 | | - | - | 0.002 |
| ZnCl2 | 0.02 | 0.01 | 0.03 | 0.05 | 0.1 | 0.05 | 0.02 |
| Calcium Formate | 0.03 | 0.03 | 0.01 | - | - | - | - |
| PB1 | - | 4.6 | - | 3.8 | - | - | - |
| Aldose Oxidase | 0.05 | - | 0.03 | - | 0.02 | 0.02 | 0.05 |
| Balance to 100% perfume / dye and/or water | | | | | | | |

The pH of formulations (I)-(VII) is from about 7.4 to about 9.5.

## Claims

1. An isolated variant protease comprising an amino acid sequence having at least 90% sequence identity to the amino acid sequence of SEQ ID NO:1 said variant protease amino acid sequence comprising a substitution of each of the amino acid residues in positions 76, 87, 118, 128, 129, 130, 188, and 244 of SEQ ID NO:1 with a different amino acid residue to yield a variant protease having an overall charge of -2, -1, 0, +1, +2, +3, or +4, wherein:

(i) the amino acid residue at each of positions 76 and 188 is substituted with a negatively charged amino acid residue selected from the group consisting of aspartic acid and glutamic acid;

(ii) the amino acid residue at each of positions 87, 118, and 244 is substituted with a positively charged amino acid residue selected from the group consisting of arginine, histidine, and lysine; and

(iii) the amino acid residue at each of positions 128, 129, and 130 is substituted with a neutral amino acid residue selected from the group consisting of serine, threonine, asparagine, glutamine, alanine, isoleucine, leucine, methionine, phenylalanine, tryptophan, tyrosine, and valine, and wherein each amino acid position is numbered according to the numbering of corresponding amino acid position in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence.

2. The variant protease of claim 1, wherein the amino acid sequence of said variant protease comprises amino acid substitutions
N76D+S87R+G118R+S128L+P129Q+S130A+S188D+V244R,
optionally wherein said variant protease comprises the amino acid sequence set forth in SEQ ID NO:8.

3. An isolated variant protease comprising an amino acid sequence having at least 90% sequence identity to the amino acid sequence of SEQ ID NO:1, said variant protease amino acid sequence comprising a substitution of the amino acid residue in each of amino acid positions 76, 87, 118, 128, 129, and 130 relative to SEQ ID NO:1 with a different amino acid residue to yield a variant protease having an overall charge of -2, -1, 0, +1, +2, +3, or +4, wherein:

(i) the amino acid residue at position 76 is substituted with a negatively charged amino acid residue selected from the group of consisting of aspartic acid and glutamic acid,

(ii) the amino acid residue at each of positions 87 and 118 is substituted with a positively charged amino acid residue selected from the group consisting of arginine, histidine, and lysine, and

(iii) the amino acid residue at each of positions 128, 129, and 130 is substituted with an uncharged amino acid residue selected form the group consisting of serine, threonine, asparagine, glutamine, alanine, isoleucine, leucine, methionine, phenylalanine, tryptophan, tyrosine, and valine, and wherein said variant protease further comprises

(iv) a serine residue at amino acid position 188 or a substitution of the serine residue at position 188 with a different amino acid residue that is or is not an aspartic acid residue, and/or

(v) an asparagine residue at amino acid position 248 or a substitution of the asparagine residue at position 248 with a different amino acid residue that is or is not an arginine residue,

and wherein amino acid positions are numbered according to the numbering of corresponding amino acid positions in the amino acid sequence of *Bacillus amyloliquefaciens* subtilisin BPN' shown in SEQ ID NO:2 as determined by alignment of the variant protease amino acid sequence with the *Bacillus amyloliquefaciens* subtilisin BPN' amino acid sequence.

4. The variant protease of claim 3, wherein said variant protease amino acid sequence comprises a serine residue at amino acid position 188 and/or an arginine residue at amino acid position 248.

5. The variant protease of claim 1 or claim 3, wherein said variant protease has an overall charge of +1.

6. The variant protease of claim 3, wherein the amino acid sequence of said variant protease comprises amino acid substitutions N76D+S87R+G118R+S128L+P129Q+S130A, optionally wherein said variant protease comprises the amino acid sequence set forth in SEQ ID NO:7.

7. An isolated variant protease according to claim 1 or claim 3, said variant protease comprising one of the following sets of amino acid substitutions relative to SEQ ID NO:1

(i) N76D+S87R+G118R+S128L+P129Q+S130A, with the proviso that said variant protease does not comprise N248R+S188D, or

(ii) N76D+S87R+G118R+S128L+P129Q+S130A+S188D+V244R.

8. A composition comprising at least one variant protease of any of claims 1-7, optionally wherein said composition is a detergent and/or cleaning composition.

**9.** The composition of claim 8, wherein:

(i) the cleaning composition is a laundry cleaning composition or a laundry detergent composition, respectively;
(ii) the cleaning composition is a dishwashing detergent composition, optionally an automatic dishwashing detergent composition or a hand dishwashing detergent composition;
(iii) the cleaning composition is a liquid detergent composition;
(iv) the cleaning composition is a gel, tablet, powder, solid, or granule detergent composition;
(v) the cleaning composition does not contain phosphate;
(vi) the composition is a liquid laundry cleaning composition or a powder laundry cleaning composition;
(vii) the composition is a laundry booster cleaning composition, laundry additive cleaning composition, or laundry pre-spotter cleaning composition;
(viii) the composition is a contact lens cleaning composition; or
(ix) the composition is not an automatic dishwashing composition.

**10.** The composition of claim 8 or claim 9, comprising at least one additional enzyme, optionally wherein the at least one additional enzyme, optionally two or more additional enzymes, is or are selected from the group consisting of hemicellulase, cellulase, amylase, peroxidase, protease, xylanase, lipase, phospholipase, esterase, cutinase, pectinase, pectate lyase, mannanase, keratinase, reductase, oxidase, phenoloxidase, lipoxygenase, ligninase, pullulanase, tannase, pentosanase, malanase, ß-glucanase, arabinosidase, hyaluronidase, chondroitinase, and laccase.

**11.** A method for cleaning an item or surface in need of cleaning, the method comprising contacting the item or surface with a variant protease of any of claims 1-7 or a composition of any of claims 8-10, optionally wherein the method further comprises rinsing the item or surface with water.

**12.** An isolated nucleic acid comprising a polynucleotide sequence that encodes the variant protease of any of claims 1-7.

**13.** An expression vector comprising at least one nucleic acid of claim 12, optionally wherein the at least one nucleic acid is operably linked to a promoter.

**14.** A recombinant host cell comprising the expression vector of claim 13 or the nucleic acid of claim 12 or the variant protease of any one of claims 1-7, optionally wherein:

(i) the host cell is a bacterial cell;
(ii) the host cell is a *Bacillus* cell; or
(iii) the host cell is a *Bacillus* subtilis cell.

**15.** A method of producing a variant protease, the method comprising cultivating the recombinant host cell of claim 14 under conditions conducive to the production of the variant protease, the method optionally further comprising recovering the variant protease from the culture.

**Patentansprüche**

**1.** Isolierte Proteasevariante, umfassend eine Aminosäuresequenz, die eine Sequenzidentität von zumindest 90 % mit der Aminosäuresequenz von SEQ ID NO:1 aufweist, wobei die Proteasevariante-Aminosäuresequenz eine Substitution von jedem der Aminosäurereste in Positionen 76, 87, 118, 128, 129, 130, 188 und 244 der SEQ ID NO:1 mit einem unterschiedlichen Aminosäurerest umfasst, um eine Proteasevariante zu ergeben, die eine Gesamtladung von -2, -1, 0, +1, +2, +3 oder +4 aufweist, wobei:

(i) der Aminosäurerest an jeder der Positionen 76 und 188 mit einem negativ geladenen Aminosäurerest substituiert ist, der aus der Gruppe ausgewählt ist, die aus Asparaginsäure und Glutaminsäure besteht;
(ii) der Aminosäurerest an jeder der Positionen 87, 118 und 244 mit einem positiv geladenen Aminosäurerest substituiert ist, der aus der Gruppe ausgewählt ist, die aus Arginin, Histidin und Lysin besteht; und
(iii) der Aminosäurerest an jeder der Positionen 128, 129 und 130 mit einem neutralen Aminosäurerest substituiert ist, der aus der Gruppe ausgewählt ist, die aus Serin, Threonin, Asparagin, Glutamin, Alanin, Isoleucin, Leucin, Methionin, Phenylalanin, Tryptophan, Tyrosin und Valin besteht, und wobei jede Aminosäureposition nach der Nummerierung der entsprechenden Aminosäureposition in der Aminosäuresequenz von *Bacillus amyloliquefaciens* subtilisin BPN', gezeigt in SEQ ID NO:2 als bestimmt durch Ausrichtung der Proteasevariante-Aminosäuresequenz auf die *Bacillus-amyloliquefaciens*-subtilisin-BPN'-Aminosäuresequenz, nummeriert ist.

2. Proteasevariante nach Anspruch 1, wobei die Aminosäuresequenz der Proteasevariante Aminosäuresubstitutionen N76D+S87R+G118R+S128L+P129Q+S130A+S188D+V244R umfasst, wobei die Proteasevariante optional die in SEQ ID NO:8 dargestellte Aminosäuresequenz umfasst.

3. Isolierte Proteasevariante, umfassend eine Aminosäuresequenz, die eine Sequenzidentität von zumindest 90 % mit der Aminosäuresequenz von SEQ ID NO:1 aufweist, die Proteasevariante-Aminosäuresequenz umfassend eine Substitution von dem Aminosäurerest in jeder der Aminosäurepositionen 76, 87, 118, 128, 129, und 130 relativ zur SEQ ID NO: 1 mit einem unterschiedlichen Aminosäurerest, um eine Proteasevariante zu ergeben, die eine Gesamtladung von -2, -1, 0, +1, +2, +3 oder +4 aufweist, wobei:

(i) der Aminosäurerest an Position 76 mit einem negativ geladenen Aminosäurerest substituiert ist, der aus der Gruppe ausgewählt ist, die aus Asparaginsäure und Glutaminsäure besteht,
(ii) der Aminosäurerest an jeder der Positionen 87 und 118 mit einem positiv geladenen Aminosäurerest substituiert ist, der aus der Gruppe ausgewählt ist, die aus Arginin, Histidin und Lysin besteht, und
(iii) der Aminosäurerest an jeder der Positionen 128, 129 und 130 mit einem ungeladenen Aminosäurerest substituiert ist, der aus der Gruppe ausgewählt ist, die aus Serin, Threonin, Asparagin, Glutamin, Alanin, Isoleucin, Leucin, Methionin, Phenylalanin, Tryptophan, Tyrosin und Valin besteht, und wobei die Proteasevariante ferner Folgendes umfasst:
(iv) einen Serinrest an Aminosäureposition 188 oder eine Substitution des Serinrests an Position 188 mit einem unterschiedlichen Aminosäurerest, der ein Asparaginsäurerest ist oder nicht ist, und/oder
(v) einen Asparaginrest an Aminosäureposition 248 oder eine Substitution des Asparaginrests an Position 248 mit einem unterschiedlichen Aminosäurerest, der ein Argininrest ist oder nicht ist, und wobei Aminosäurepositionen nach der Nummerierung der entsprechenden Aminosäurepositionen in der Aminosäuresequenz von *Bacillus amyloliquefaciens* subtilisin BPN', gezeigt in SEQ ID NO:2 als bestimmt durch Ausrichtung der Proteasevariante-Aminosäuresequenz auf die *Bacillus-amyloliquefaciens*-subtilisin-BPN'-Aminosäuresequenz, nummeriert ist.

4. Proteasevariante nach Anspruch 3, wobei die Proteasevariante-Aminosäuresequenz einen Serinrest an Aminosäureposition 188 und/oder einen Argininrest an Aminosäureposition 248 umfasst.

5. Proteasevariante nach Anspruch 1 oder Anspruch 3, wobei die Proteasevariante eine Gesamtladung von +1 aufweist.

6. Proteasevariante nach Anspruch 3, wobei die Aminosäuresequenz der Proteasevariante Aminosäuresubstitutionen N76D+S87R+G118R+S128L+P129Q+S130A umfasst, wobei die Proteasevariante optional die in SEQ ID NO:7 dargestellte Aminosäuresequenz umfasst.

7. Isolierte Proteasevariante nach Anspruch 1 oder Anspruch 3, die Proteasevariante umfassend einen der folgenden Sätze von Aminosäuresubstitutionen relativ zur SEQ ID NO:1:

(i) N76D+S87R+G118R+S128L+P129Q+S130A, mit der Maßgabe, dass die Proteasevariante nicht N248R+S188D umfasst, oder
(ii) N76D+S87R+G118R+S128L+P129Q+S130A+S188D+V244R.

8. Zusammensetzung, umfassend zumindest eine Proteasevariante nach einem der Ansprüche 1-7, wobei die Zusammensetzung optional eine Waschmittel- und/oder Reinigungszusammensetzung ist.

9. Zusammensetzung nach Anspruch 8, wobei:

(i) die Reinigungszusammensetzung eine Wäschereinigungszusammensetzung bzw. eine Wäschewaschmittelzusammensetzung ist;
(ii) die Reinigungszusammensetzung eine Geschirrwaschmittelzusammensetzung ist, optional eine Maschinengeschirrwaschmittelzusammensetzung oder eine Handgeschirrwaschmittelzusammensetzung;
(iii) die Reinigungszusammensetzung eine flüssige Waschmittelzusammensetzung ist;
(iv) die Reinigungszusammensetzung eine Gel-, Tabletten-, Pulver- Feststoff oder Granulatwaschmittelzusammensetzung ist;
(v) die Reinigungszusammensetzung nicht Phosphat enthält;

(vi) die Zusammensetzung eine flüssige Wäschereinigungszusammensetzung bzw. eine pulverförmige Wäschereinigungszusammensetzung ist;

(vii) die Zusammensetzung eine Wäscheverstärkerreinigungszusammensetzung, Wäschezusatzreinigungszusammensetzung oder Wäschefleckenvorreinigungszusammensetzung ist;

(viii) die Zusammensetzung eine Kontaktlinsenreinigungszusammensetzung ist; oder

(ix) die Zusammensetzung keine Maschinengeschirrspülzusammensetzung ist.

10. Zusammensetzung nach Anspruch 8 oder Anspruch 9, umfassend zumindest ein zusätzliches Enzym, wobei optional das mindestens eine zusätzliche Enzym, optional die zwei oder mehreren zusätzlichen Enzyme aus der Gruppe ausgewählt ist oder sind, die aus Hemicellulase, Cellulase, Amylase, Peroxidase, Protease, Xylanase, Lipase, Phospholipase, Esterase, Cutinase, Pektinase, Pektatlyase, mannanase, Keratinase, Reduktase, Oxidase, Phenoloxidase, Lipoxygenase, Ligninase, Pullulanase, Tannase, Pentosanase, Malanase, β-Glucanase, Arabinosidase, Hyaluronidase, Chondroitinase und Laccase besteht.

11. Verfahren zum Reinigen eines Artikels oder einer Oberfläche, die ein Reinigen benötigen, das Verfahren umfassend das Kontaktieren des Artikels oder der Oberfläche mit einer Proteasevariante nach einem der Ansprüche 1-7 oder einer Zusammensetzung nach einem der Ansprüche 8-10, wobei das Verfahren optional das Spülen des Artikels oder der Oberfläche mit Wasser umfasst.

12. Isolierte Nukleinsäure, umfassend eine Polynukleotidssequenz, die die Proteasevariante nach einem der Ansprüche 1-7 codiert.

13. Expressionsvektor, umfassend zumindest eine Nukleinsäure nach Anspruch 12, wobei zumindest eine Nukleinsäure mit einem Promoter wirkungsverbunden ist.

14. Rekombinante Wirtszelle, umfassend den Expressionsvektor nach Anspruch 13 oder die Nukleinsäure nach Anspruch 12 oder die Proteasevariante nach einem der Ansprüche 1-7, wobei optional:

(i) die Wirtszelle eine bakterielle Zelle ist;

(ii) die Wirtszelle eine *Bacillus*-Zelle ist;

(iii) die Wirtszelle eine *Bacillus-subtilis*-Zelle ist.

15. Verfahren zum Produzieren einer Proteasevariante, das Verfahren umfassend das Kultivieren der rekombinanten Wirtszelle nach Anspruch 14 unter Bedingungen, die für die Produktion der Proteasevariante förderlich sind, das Verfahren optional ferner umfassend das Wiedererlangen der Proteasevariante aus der Kultur.

## Revendications

1. Protéase variante isolée comprenant une séquence d'acides aminés possédant au moins 90% d'identité de séquence avec la séquence d'acides aminés de la SEQ ID NO:1, ladite séquence d'acides aminés de la protéase variante comprenant une substitution de chacun des résidus d'acide aminé dans les positions 76, 87, 118, 128, 129, 130, 188 et 244 de la SEQ ID NO:1 avec un résidu d'acide aminé différent pour donner une protéase variante ayant une charge globale de -2, -1, 0, +1, +2, +3 ou +4, dans laquelle:

(i) le résidu d'acide aminé à chacune des positions 76 et 188 est substitué avec un résidu d'acide aminé chargé négativement choisi dans le groupe constitué d'acide aspartique et d'acide glutamique;

(ii) le résidu d'acide aminé à chacune des positions 87, 118 et 244 est substitué avec un résidu d'acide aminé chargé positivement choisi dans le groupe constitué d'arginine, d'histidine et de lysine; et

(iii) le résidu d'acide aminé à chacune des positions 128, 129 et 130 est substitué avec un résidu d'acide aminé neutre choisi dans le groupe constitué de sérine, thréonine, asparagine, glutamine, alanine, isoleucine, leucine, méthionine, phénylalanine, tryptophane, tyrosine et valine et dans laquelle chaque position d'acide aminé est numérotée selon la numérotation de la position d'acide aminé correspondante dans la séquence d'acides aminés de subtilisine BPN' de *Bacillus amyloliquefaciens* montrée dans la SEQ ID NO:2 comme il est déterminé par un alignement de la séquence d'acides aminés de la protéase variante avec la séquence d'acides aminés de la subtilisine BPN' de *Bacillus amyloliquefaciens.*

2. Protéase variante selon la revendication 1, dans laquelle la séquence d'acides aminés de ladite protéase variante comprend les substitutions d'acides aminés:
N76D+S87R+G118R+S128L+P129Q+S130A+S188D+V244R,
optionnellement où ladite protéase variante comprend la séquence d'acides aminés présentée dans la SEQ ID NO:8.

3. Protéase variante isolée comprenant une séquence d'acides aminés possédant au moins 90% d'identité de séquence avec la séquence d'acides aminés de la SEQ ID NO:1, ladite séquence d'acides aminés de la protéase variante comprenant une substitution du résidu d'acide aminé dans chacune des positions d'acide aminé 76, 87, 118, 128, 129 et 130 relativement à la SEQ ID NO:1 avec un résidu d'acide aminé différent pour donner une protéase variante ayant une charge globale de -2, -1, 0, +1, +2, +3 ou +4, dans laquelle:

(i) le résidu d'acide aminé à la position 76 est substitué avec un résidu d'acide aminé chargé négativement choisi dans le groupe constitué d'acide aspartique et d'acide glutamique;
(ii) le résidu d'acide aminé à chacune des positions 87 et 118 est substitué avec un résidu d'acide aminé chargé positivement choisi dans le groupe constitué d'arginine, d'histidine et de lysine; et
(iii) le résidu d'acide aminé à chacune des positions 128, 129 et 130 est substitué avec un résidu d'acide aminé non chargé choisi dans le groupe constitué de sérine, thréonine, asparagine, glutamine, alanine, isoleucine, leucine, méthionine, phénylalanine, tryptophane, tyrosine et valine et dans laquelle ladite protéase variante comprend en outre:
(iv) un résidu de sérine à la position d'acide aminé 188 ou une substitution du résidu de sérine à la position 188 avec un résidu d'acide aminé différent qui est ou n'est pas un résidu d'acide aspartique, et/ou
(v) un résidu d'asparagine à la position d'acide aminé 248 ou une substitution du résidu d'asparagine à la position 248 avec un résidu d'acide aminé différent qui est ou n'est pas un résidu d'arginine, et dans laquelle les positions d'acide aminé sont numérotées selon la numérotation des positions d'acide aminé correspondantes dans la séquence d'acides aminés de subtilisine BPN' de *Bacillus amyloliquefaciens* montrée dans la SEQ ID NO:2 comme il est déterminé par un alignement de la séquence d'acides aminés de la protéase variante avec la séquence d'acides aminés de la subtilisine BPN' de *Bacillus amyloliquefaciens.*

4. Protéase variante selon la revendication 3, dans laquelle ladite séquence d'acides aminés de la protéase variante comprend un résidu de sérine à la position d'acide aminé 188 et/ou un résidu d'arginine à la position d'acide aminé 248.

5. Protéase variante selon la revendication 1 ou la revendication 3, où ladite protéase variante a une charge globale de +1.

6. Protéase variante selon la revendication 3, dans laquelle la séquence d'acides aminés de ladite protéase variante comprend les substitutions d'acides aminés:
N76D+S87R+G118R+S128L+P129Q+S130A,
optionnellement où ladite protéase variante comprend la séquence d'acides aminés présentée dans la SEQ ID NO:7.

7. Protéase variante isolée selon la revendication 1 ou la revendication 3, ladite protéase variante comprenant un des groupes qui suivent de substitutions d'acides aminés relativement à la SEQ ID NO:1:

(i) N76D+S87R+G118R+S128L+P129Q+S130A, sous réserve que ladite protéase variante ne comprenne pas N248R+S118D, ou
(ii) N76D+S87R+G118R+S128L+P129Q+S130A+S188D+V244R.

8. Composition comprenant au moins une protéase variante selon l'une quelconque des revendications 1-7, optionnellement dans laquelle ladite composition est une composition de détergent et/ou nettoyante.

9. Composition selon la revendication 8, dans laquelle:

(i) la composition nettoyante est une composition nettoyante pour laver le linge ou une composition de détergent pour laver le linge, respectivement;
(ii) la composition nettoyante est une composition de détergent pour laver la vaisselle, optionnellement une composition de détergent pour laver la vaisselle automatiquement ou une composition de détergent pour laver la vaisselle à la main;
(iii) la composition nettoyante est une composition de détergent liquide;
(iv) la composition nettoyante est une composition de détergent en gel, en pastilles, en poudre, solide ou en granulés;

(v) la composition nettoyante ne contient pas de phosphate;

(vi) la composition est une composition nettoyante pour laver le linge liquide ou une composition nettoyante pour laver le linge en poudre;

(vii) la composition est une composition nettoyante de renforçateur pour laver le linge, une composition nettoyante d'additif pour laver le linge ou une composition nettoyante de pré-détachage pour laver le linge;

(viii) la composition est une composition nettoyante pour lentille de contact; ou

(ix) la composition n'est pas une composition pour laver la vaisselle automatiquement.

10. Composition selon la revendication 8 ou la revendication 9, comprenant au moins une enzyme supplémentaire, optionnellement dans laquelle la au moins une enzyme supplémentaire, optionnellement deux ou plus enzymes supplémentaires, est ou sont choisies dans le groupe constitué de: hémicellulase, cellulase, amylase, peroxydase, protéase, xylanase, lipase, phospholipase, estérase, cutinase, pectinase, pectate lyase, mannanase, kératinase, réductase, oxydase, phénoloxydase, lipoxygénase, ligninase, pullulanase, tannase, pentosanase, malanase, β-glucanase, arabinosidase, hyaluronidase, chondroïtinase et laccase.

11. Méthode pour le nettoyage d'un article ou d'une surface ayant besoin d'un nettoyage, la méthode comprenant la mise en contact de l'article ou de la surface avec une protéase variante selon l'une quelconque des revendications 1-7 ou une composition selon l'une quelconque des revendications 8-10, optionnellement où la méthode comprend en outre le rinçage de l'article ou de la surface avec de l'eau.

12. Acide nucléique isolé comprenant une séquence de polynucléotide qui code pour la protéase variante selon l'une quelconque des revendications 1-7.

13. Vecteur d'expression comprenant au moins un acide nucléique selon la revendication 12, optionnellement dans lequel le au moins un acide nucléique est lié de manière fonctionnelle à un promoteur.

14. Cellule hôte recombinante comprenant le vecteur d'expression selon la revendication 13 ou l'acide nucléique selon la revendication 12 ou la protéase variante selon l'une quelconque des revendications 1-7, optionnellement où:

(i) la cellule hôte est une cellule bactérienne;

(ii) la cellule hôte est une cellule de *Bacillus*; ou

(iii) la cellule hôte est une cellule de *Bacillus subtilis.*

15. Méthode pour la production d'une protéase variante, la méthode comprenant la culture de la cellule hôte recombinante selon la revendication 14 dans des conditions favorables à la production de la protéase variante, la méthode comprenant en outre optionnellement la récupération de la protéase variante à partir de la culture.

```
GG36            1 AQSVPWGISR VQAPAAHNRG LTGSGVKVAV LDTGIS-THP DLNIRGGASF VPGEPST-QD
BPN'            1 AQSVPYGVSQ IKAPALHSQG YTGSNVKVAV IDSGIDSSHP DLKVAGGASM VPSETNPFQD
PX3             1 AQSVPWGISR VQAPAAHNRG LTGSGVKVAV LDTGIS-THP DLNIRGGASF VPGEPST-QD
PX4             1 AQSVPWGISR VQAPAAHNRG LTGSGVKVAV LDTGIS-THP DLNIRGGASF VPGEPST-QD
PX5             1 AQSVPWGISR VQAPAAHNRG LTGSGVKVAV LDTGIS-THP DLNIRGGASF VPGEPST-QD
Conservation    1 *****:*:*: ::*** *..* ***.***** .*:**. :** **:: ****: **.*... **

GG36           59 GNGHGTHVAG TIAALNNSIG VLGVAPSAEL YAVKVLGASG SGSVSSIAQG LEWAGNNGMH
BPN'           61 NNSHGTHVAG TVAALNNSIG VLGVAPSASL YAVKVLGADG SGQYSWIING IEWAIANNMD
PX3            59 GNGHGTHVAG TIAALDNSIG VLGVAPRAEL YAVKVLGASG SGSVSSIAQG LEWAGNNRMH
PX4            59 GNGHGTHVAG TIAALDNSIG VLGVAPRAEL YAVKVLGASG SGSVSSIAQG LEWAGNNRMH
PX5            59 GNGHGTHVAG TIAALDNSIG VLGVAPRAEL YAVKVLGASG SGSVSSIAQG LEWAGNNRMH
Conservation   61 .*.******* *:***:**** ****** *.* ********.* **. * * :* :*** * *.

GG36          119 VANLSLGSPS PSATLEQAVN SATSRGVLVV AASGNSGAGS ----ISYPAR YANAMAVGAT
BPN'          121 VINMSLGGPS GSAALKAAVD KAVASGVVVV AAAGNEGTSG SSSTVGYPGK YPSVIAVGAV
PX3           119 VANLSLGLQA PSATLEQAVN SATSRGVLVV AASGNSGAGS ----ISYPAR YANAMAVGAT
PX4           119 VANLSLGLQA PSATLEQAVN SATSRGVLVV AASGNSGAGS ----ISYPAR YANAMAVGAT
PX5           119 VANLSLGLQA PSATLEQAVN SATSRGVLVV AASGNSGAGS ----ISYPAR YANAMAVGAT
Conservation  121 * *:*** : **:*: **: .*.: **:** **:**.*:..      :.**.: *...:****.

GG36          175 DQNNNRASFS QYGAGLDIVA PGVNVQSTYP GSTYASLNGT SMATPHVAGA AALVKQKNPS
BPN'          181 DSSNQRASFS SVGPELDVMA PGVSIQSTLP GNKYGAYNGT SMASPHVAGA AALILSKHPN
PX3           175 DQNNNRADFS QYGAGLDIVA PGVNVQSTYP GSTYASLNGT SMATPHVAGA AALVKQKNPS
PX4           175 DQNNNRASFS QYGAGLDIVA PGVNVQSTYP GSTYASLNGT SMATPHVAGA AALVKQKNPS
PX5           175 DQNNNRADFS QYGAGLDIVA PGVNVQSTYP GSTYASLNGT SMATPHVAGA AALVKQKNPS
Conservation  181 *..*:**.** . *. **::* ***.:*** * *..*.: *** ***:****** ***: .*:*.

GG36          235 WSNVQIRNHL KNTATSLGST NLYGSGLVNA EAATR
BPN'          241 WTNTQVRSSL ENTTTKLGDS FYYGKGLINV QAAAQ
PX3           235 WSNVQIRRHL KNTATSLGST NLYGSGLVNA EAATR
PX4           235 WSNVQIRNHL KNTATSLGST NLYGSGLVNA EAATR
PX5           235 WSNRQIRNHL KNTATSLGST NLYGSGLVNA EAATR
Conservation  241 *:* *:*  * :**:*.**.:   **.**:*. :**::
```

**FIG. 2**

GG36 (mature protein coding region)

Propeptide

Signal sequence

LAT Promoter

HPA2 Promoter

Bleomycin resistance marker

pHPLT-GG36

4772 bp

reppUB

Neo resistance marker

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2008112258 A
- WO 02077187 A
- WO 2010123754 A
- WO 2010056640 A
- WO 9934011 A
- US 6376450 B
- US 4683195 A
- US 4683202 A
- US 4965188 A
- US 6582914 B
- US 6605458 B
- US RE34606 E
- US 5700676 A
- US 5955340 A
- US 6312936 B
- US 6482628 B
- WO 9221760 A
- WO 9523221 A
- WO 9707770 A
- US 5264366 A
- US 4760025 A
- US 4450235 A
- US 4302544 A
- EP 0134048 A
- US 20050202535 A
- US 6610642 B
- US 5705464 A
- US 5710115 A
- US 5698504 A
- US 5695679 A
- US 5686014 A
- US 5646101 A
- WO 9711151 A
- EP 0922499 A
- US 4977252 A
- US 5354559 A
- US 5935826 A
- WO 8906270 A
- US 20080090747 A
- US 5801039 A
- US 5340735 A
- US 5500364 A
- US 5855625 A
- WO 07044993 A
- EP 258068 A
- EP 305216 A
- EP 238023 A
- EP 214761 A
- EP 218272 A
- EP 331376 A
- GB 1372034 A
- JP 64744992 B
- WO 9116422 A
- WO 8809367 A
- WO 9009446 A
- GB 1296839 A
- US 4435307 A
- EP 0495257 A
- US 5874276 A
- US 6566114 B
- US 6602842 B
- US 6440991 B
- WO 9412621 A
- WO 9501426 A
- WO 05056782 A
- US 5879584 A
- US 5691297 A
- US 5574005 A
- US 5569645 A
- US 5565422 A
- US 5516448 A
- US 5489392 A
- US 5486303 A
- US 5576282 A
- US 6306812 B
- US 6326348 B
- EP 2100949 A
- WO 07145964 A
- US 4246612 A
- US 5227084 A
- US 4810410 A
- WO 9906521 A
- US 4430243 A
- US 5597936 A
- US 5595967 A
- WO 200032601 A
- US 6225464 B
- WO 9426860 A
- WO 9426859 A
- US 4515705 A
- US 4537706 A
- US 4515707 A
- US 4550862 A
- US 4561998 A
- US 4597898 A
- US 4968451 A

- US 5565145 A
- US 5929022 A
- US 6294514 B
- US 6376445 B
- WO 02102955 A
- US 4765916 A
- US 4972017 A

- WO 04111178 A
- EP 2100947 A
- WO 9418314 A
- WO 9605295 A
- US 20060252155 A
- US 6566112 B
- WO 0214490 A

**Non-patent literature cited in the description**

- **STROUD.** *Sci. Amer.,* vol. 131, 74-88
- Microbial Proteinases. **KALISZ.** Advances in Biochemical Engineering/Biotechnology. 1988
- **DEL MAR et al.** *Anal. Biochem.,* 1979, vol. 99, 316-320
- **RAWLINGS et al.** MEROPS: the peptidase database. *Nucl. Acids Res.,* 2006, vol. 34
- *Biochem. J.,* 1993, vol. 290, 205-218
- Genetics. **FERRARI et al.** Bacillus. Plenum Publishing Corp, 1989, 57-72
- **SMITH ; WATERMAN.** *Adv. Appl. Math.,* 1981, vol. 2, 482
- **NEEDLEMAN ; WUNSCH.** *J. Mol. Biol.,* 1970, vol. 48, 443
- **PEARSON ; LIPMAN.** *Proc. Natl. Acad. Sci. USA,* 1988, vol. 85, 2444
- **DEVEREUX et al.** *Nucl. Acid Res.,* 1984, vol. 12, 387-395
- **FENG ; DOOLITTLE.** *J. Mol. Evol.,* 1987, vol. 35, 351-360
- **HIGGINS ; SHARP.** *CABIOS,* 1989, vol. 5, 151-153
- **ALTSCHUL et al.** *J. Mol. Biol.,* 1990, vol. 215, 403-410
- **KARLIN et al.** *Proc. Natl. Acad. Sci. USA,* 1993, vol. 90, 5873-5787
- **ALTSCHUL et al.** *Meth. Enzymol.,* 1996, vol. 266, 460-480
- **HENIKOFF ; HENIKOFF.** *Proc. Natl. Acad. Sci. USA,* 1992, vol. 89, 10915
- **KARLIN ; ALTSCHUL.** *Proc. Natl. Acad. Sci. USA,* 1993, vol. 90, 5873-5787
- **HENIKOFF ; HENIKOFF.** *Proc. Natl. Acad. Sci. USA,* 1992, vol. 89 (22), 10915
- **ALTSCHUL et al.** *Nucleic Acids Res.,* 1997, vol. 25 (17), 3389-3402
- **SAMBROOK et al.** Molecular Cloning--A Laboratory Manual. Cold Spring Harbor Laboratory, 1989, vol. 1-3
- CURRENT PROTOCOLS IN MOLECULAR BIOLOGY. Greene Publishing Associates, Inc. and John Wiley & Sons, Inc, 1994
- **CREIGHTON.** Proteins: STRUCTURE AND MOLECULAR PROPERTIES. W.H. Freeman and Company, 1984
- **BEAUCAGE et al.** *Tetrahedron Letters,* 1981, vol. 22, 1859-69

- **MATTHES et al.** *EMBO J.,* 1984, vol. 3, 801-05
- **ITAKURA et al.** *Annu. Rev. Biochem.,* 1984, vol. 53, 323
- **ITAKURA et al.** *Science,* 1984, vol. 198, 1056
- **SAMBROOK et al.** Molecular Cloning: A Laboratory Manual. Cold Spring Harbor Press, 1989
- **AUSUBEL et al.** Current Protocols in Molecular Biology. Wiley Interscience Publishers, 1994
- Guide to Molecular Cloning Techniques. **BERGER ; KIMMEL.** Methods in Enzymol. Acad. Press, Inc, vol. 152
- **OSTERMEIER et al.** *ITCHY,* 1999, vol. 7 (10), 2139-44
- **LUTZ et al.** *SCRACHY,* 2001, vol. 98 (20), 11248-53
- **SIEBER et al.** *SHIPREC,* 2001, vol. 19 (5), 456-60
- **BITTKER et al.** *NRR,* 2001, vol. 20 (10), 1024-9
- *Proc. Natl. Acad. Sci. USA,* 1992, vol. 89 (8), 3581-5
- Molecular Biological Methods for Bacillus. John Wiley & Sons, 1990
- **PEREGO, M.** *Integrational Vectors for Genetic Manipulations in Bacillus subtilis,* 1993, 615-624
- Bacillus subtilis and other Gram-positive bacteria: biochemistry, physiology and molecular genetics. American Society for Microbiology
- **HOCH et al.** *Genetics,* 1973, vol. 73, 215-228
- **PALVA et al.** *Gene,* 1982, vol. 19, 81-87
- **FAHNESTOCK ; FISCHER.** *J. Bacteriol.,* 1986, vol. 165, 796-804
- **WANG et al.** *Gene,* 1988, vol. 69, 39-47
- **MSADEK et al.** *J. Bacteriol.,* 1990, vol. 172, 824-834
- **OLMOS et al.** *Mol. Gen. Genet.,* 1997, vol. 253, 562-567
- **CALDWELL et al.** *J. Bacteriol.,* 2001, vol. 183, 7329-7340
- **ARIGONI et al.** *Mol. Microbiol.,* 1999, vol. 31, 1407-1415
- **PEREGO et al.** *Mol. Microbiol.,* 1991, vol. 5, 173-185
- **SAUNDERS et al.** *J. Bacteriol.,* 1984, vol. 157, 718-726

- **HOCH et al.** *J. Bacteriol.,* 1967, vol. 93, 1925-1937
- **MANN et al.** *Current Microbiol.,* 1986, vol. 13, 131-135
- **HOLUBOVA.** *Folia Microbiol.,* 1985, vol. 30, 97
- **CHANG et al.** *Mol. Gen. Genet.,* 1979, vol. 168, 11-115
- **VOROBJEVA et al.** *FEMS Microbiol. Lett.,* 1980, vol. 7, 261-263
- **SMITH et al.** *Appl. Env. Microbiol.,* 1986, vol. 51, 634
- **FISHER et al.** *Arch. Microbiol.,* 1981, vol. 139, 213-217
- **MCDONALD.** *J. Gen. Microbiol.,* 1984, vol. 130, 203
- **CONTENTE et al.** *Plasmid,* 1979, vol. 2, 555-571
- **HAIMA et al.** *Mol. Gen. Genet.,* 1990, vol. 223, 185-191
- **WEINRAUCH et al.** *J. Bacteriol.,* 1983, vol. 154, 1077-1087
- **WEINRAUCH et al.** *J. Bacteriol.,* 1987, vol. 169, 1205-1211
- **STAHL et al.** *J. Bacteriol.,* 1984, vol. 158, 411-418
- **PALMEROS et al.** *Gene,* 2000, vol. 247, 255-264
- **HOPWOOD.** Practical Streptomyces Genetics. John Innes Foundation, 2000
- **HARDWOOD et al.** Molecular Biological Methods for Bacillus. John Wiley, 1990
- **KROLL, D.J. et al.** *DNA Cell Biol.,* 1993, vol. 12, 441-53
- **PORATH J.** *Protein Expr. Purif.,* 1992, vol. 3, 263-281
- Methods of Enzymatic Analysis. **BERGMEYER et al.** Peptidases, Proteinases and their Inhibitors. Verlag Chemie, 1984, vol. 5
- Proteinases. **WARD.** Microbial Enzymes and Biotechnology. Applied Science, 1983, 251-317
- **WELLS et al.** *Nucleic Acids Res.,* 1983, vol. 11, 7911-7925
- **CHRISTIANSON et al.** *Anal. Biochem.,* 1994, vol. 223, 119-129
- **HSIA et al.** *Anal Biochem.,* 1999, vol. 242, 221-227
- **MADDOX et al.** *J. Exp. Med.,* 1983, vol. 158, 1211
- **DARTOIS et al.** *Biochem. Biophys. Acta,* 1993, vol. 1131, 253-260
- **YAMAGUCHI et al.** *Gene,* 1991, vol. 103, 61-67
- **SCHIMADA et al.** *J. Biochem.,* 1989, vol. 106, 383-388
- **HASS et al.** *Gene,* 1991, vol. 109, 117-113
- **KUGIMIYA et al.** *Biosci. Biotech. Biochem.,* 1992, vol. 56, 716-719
- **MCKENZIE et al.** *Plasmid,* 1986, vol. 15, 93-103
- **HAHN et al.** *Mol. Microbiol.,* 1996, vol. 21, 763-775
- **NEIDHARDT et al.** *J Bacteriol.,* 1974, vol. 119, 736-747
- **LAEMMLI.** *Nature,* 1970, vol. 227, 680-685
- **BENDER et al.** *J. Amer. Chem. Soc.,* 1966, vol. 88, 5890-5931